(19) Europäisches Patentamt
European Patent Office
Office européen des brevets

(11) Publication number : **0 629 633 A2**

(12) **EUROPEAN PATENT APPLICATION**

(21) Application number : **94303883.6**

(22) Date of filing : **27.05.94**

(51) Int. Cl.$^5$ : **C07H 21/00,** C07H 19/04, A61K 31/70

(30) Priority : **05.06.93 GB 9311682**

(43) Date of publication of application : **21.12.94 Bulletin 94/51**

(84) Designated Contracting States : **AT BE CH DE DK ES FR GB IE IT LI LU NL PT SE**

(71) Applicant : **CIBA-GEIGY AG**
**Klybeckstrasse 141**
**CH-4002 Basel (CH)**

(72) Inventor : **Baxter, Anthony David**
**52 Lime Avenue,**
**Leftwhich Green**
**Northwich, Cheshire (GB)**
Inventor : **Baylis, Eric Keith**
**18 Abbey Grove**
**Stockport, Cheshire (GB)**
Inventor : **Collingwood, Stephen Paul**
**39 Rosewood,**
**The Hoskers**
**Westhoughton, Bolton (GB)**
Inventor : **Taylor, Roger John**
**14 Stuart Road**
**Stretford, Manchester (GB)**
Inventor : **Weetman, John**
**58 Snowden Avenue**
**Flixton, Manchester (GB)**

(74) Representative : **Sharman, Thomas et al**
**CIBA-GEIGY PLC.**
**Patent Department,**
**Central Research,**
**Hulley Road**
**Macclesfield, Cheshire SK10 2NX (GB)**

(54) **Dinucleotide analogues, intermediates therefor and oligonucleotides derived therefrom.**

(57) A compound of formula

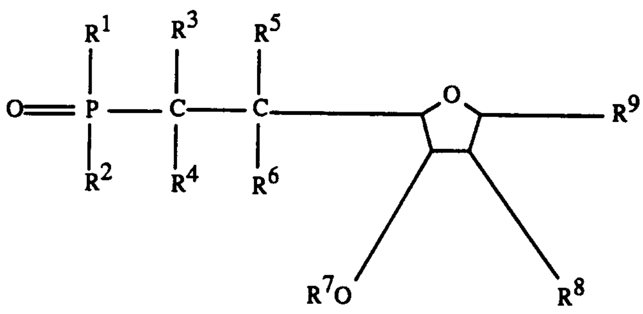

I

where $R^1$ is hydrogen, $R^1_a$ or a group of formula

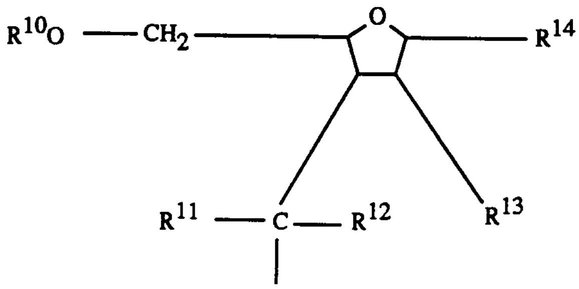

II

$R^1_a$ is $R^1_b$ or a protecting group Q,
$R^1_b$ is $C_1$-$C_{20}$ alkyl, $C_2$-$C_{20}$ alkenyl, $C_3$-$C_{10}$ cycloalkyl, $C_6$-$C_{15}$ aryl, $C_7$-$C_{16}$ aralkyl or a 5- or 6- membered heterocyclic group attached by a carbon atom in the heterocyclic group to the indicated phosphorus atom,
$R^2$ is an unsubstituted or substituted $C_1$-$C_{20}$ alkyl or $C_2$-$C_{20}$ alkenyl group, an unsubstituted or

EP 0 629 633 A2

substituted $C_3$-$C_{10}$ cycloalkyl group, an unsubstituted or substituted $C_6$-$C_{15}$ aryl group, an unsubstituted or substituted $C_7$-$C_{16}$ aralkyl group, or a 5- or 6- membered heterocyclic group attached by a carbon atom in the heterocyclic group to the indicated phosphorus atom,
$R^3$ is hydrogen, halogen, hydroxy, $R^{15}$, $-OR^{15}$ or $-OSO_2R^{15}$
$R^4$ is $R^4_a$ or together with $R^6$ denotes a valence bond,
$R^4_a$ is hydrogen, halogen or $R^{16}$,
$R^5$ is hydrogen, halogen, hydroxy, $R^{17}$, $-OR^{17}$, $-OCOR^{17}$ or $-OSO_2R^{17}$,
$R^6$ is hydrogen, halogen or $R^{18}$, or together with $R^4$ denotes a valence bond,
$R^7$ is hydrogen, alkyl-N, N-dialkylphosphoramidyl or $R^7_a$, or $R^7O$- together with $R^8_a$ denotes a $C_1$-$C_{15}$ hydrocarbylidenedioxy group,
$R^7_a$ is $R^{19}$, $-COR^{19}$, $-SO_2R^{19}$ or tri($C_1$-$C_{15}$ hydrocarbyl)silyl,
$R^8$ is $R^8_a$ or Z,
$R^8_a$ is hydrogen, halogen, hydroxy, $R^{20}$, $-OR^{20}$, $-OCOR^{20}$, $-OSO_2R^{20}$, or tri($C_1$-$C_{15}$ hydrocarbyl)silyloxy, or together with $R^9$ denotes a $C_1$-$C_{15}$ hydrocarbylidenedioxy group, or together with $R^7O$- denotes a $C_1$-$C_{15}$ hydrocarbylidenedioxy group,
$R^9$ is halogen, hydroxy, $-OR^{21}$, $-OCOR^{21}$, $-OSO_2R^{21}$, or $B^2$, or together with $R^8_a$ denotes a $C_1$-$C_{15}$ hydrocarbylidenedioxy group,
$R^{10}$ is hydrogen or $R^{10}_a$,
$R^{10}_a$ is $R^{22}$, $-COR^{22}$, $-SO_2R^{22}$ or tri($C_1$-$C_{15}$ hydrocarbyl)silyl,
$R^{11}$ is hydrogen, halogen, hydroxy, $R^{23}$, $-OR^{23}$, $-OCOR^{23}$, $-OSO_2R^{23}$ or Z,
$R^{12}$ is hydrogen, halogen or $R^{24}$,
$R^{13}$ is $R^{13}_a$ or Z,
$R^{13}_a$ is hydrogen, halogen, hydroxy, $R^{25}$, $-OR^{25}$, $-OCOR^{25}$, $-OSO_2R^{25}$, or tri($C_1$-$C_{15}$ hydrocarbyl)silyloxy, or together with $R^{14}$ denotes a $C_1$-$C_{15}$ hydrocarbylidenedioxy group,
$R^{14}$ is halogen, hydroxy, $-OR^{26}$, $-OCOR^{26}$, $-OSO_2R^{26}$, or $B^1$, or together with $R^{13}_a$ denotes a $C_1$-$C_{15}$ hydrocarbylidenedioxy group,
$R^{15}$, $R^{16}$, $R^{17}$, $R^{18}$, $R^{19}$, $R^{20}$, $R^{21}$, $R^{22}$, $R^{23}$, $R^{24}$, $R^{25}$ and $R^{26}$ are independently a $C_1$ to $C_{10}$ aliphatic group, a $C_3$ to $C_{10}$ cycloaliphatic group, a $C_6$ to $C_{15}$ aromatic group or a $C_7$-$C_{30}$ araliphatic group,
$B^1$ and $B^2$ are independently each a monovalent nucleoside base radical, and
Z is substituted or unsubstituted $C_6$-$C_{10}$ aryloxythiocarbonyloxy; provided that compounds where simultaneously $R^1$, $R^3$, $R^4$ and $R^6$ are hydrogen, $R^2$ is isopropyl, $R^5$ is methoxy, $R^7$ is methyl, $R^8$ and $R^9$ are each hydroxy or each acetoxy or $R^8$ and $R^9$ together denote isopropylidenedioxy, compounds where simultaneously $R^1$, $R^3$, $R^4$ and $R^6$ are hydrogen, $R^2$ is butyl, $R^5$ is methoxy, $R^7$ is benzyl, $R^8$ and $R^9$ are each hydroxy or each acetoxy or $R^8$ and $R^9$ together denote isopropylidenedioxy, and compounds where simultaneously $R^1$, $R^3$, $R^4$, $R^5$, $R^6$ and $R^7$ are hydrogen, $R^2$ is phenyl and $R^8$ and $R^9$ together denote isopropylidenedioxy are excluded.

This invention relates to compounds which are dinucleotide analogues or intermediates therefor, their preparation and to oligonucleotide analogues incorporating units derived from them.

For several years there has been interest in structural analogues of natural oligonucleotides because of their utility as anti-sense probes for inhibiting gene expression in biological systems and as pharmaceuticals in the treatment of viruses such as influenza, herpes and HIV, and in the treatment of cancer. Amongst the analogues of recent interest are those in which the groups linking the sugar moieties of oligonucleotides are modified by the replacement of the 3' and 5' oxy linkages by other linking groups.

WO 92/20822 describes oligonucleotide analogues comprising subunits at least some of which have the structure

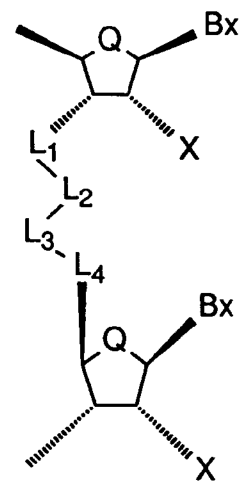

wherein $B_x$ is a variable base moiety; Q is O, $CH_2$, CHF or $CF_2$; X is H, OH, $C_1$ to $C_{10}$ lower alkyl, substituted lower alkyl, alkaryl, or aralkyl, F, Cl, Br, CN, $CF_3$, $OCF_3$, OCN, O-, S-, or N-alkyl, O-, S-, or N-alkenyl, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a group for improving the pharmacokinetic properties of an oligonucleotide or a group for improving the pharmacodynamic properties of an oligonucleotide;

$L_1$ and $L_4$ are, independently, $CH_2$, C=O, C=S, C-$NH_2$, C- $NHR_3$, C-OH, C-SH, C-O-$R_1$ or C-S-$R_1$ and $L_2$ and $L_3$ are, independently, $CR_1R_2$, C=$CR_1R_2$, C=$NR_3$, P(O)$R_4$, P(S)$R_4$, C=O, C=S, O, S, SO, $SO_2$, $NR_3$ or $SiR_5R_5$ or $L_2$ and $L_3$ together form part of an alkene, alkyne, aromatic ring, carbocycle or heterocycle, or $L_1$, $L_2$, $L_3$ and $L_4$ together comprise a -CH=N-NH-$CH_2$- or -$CH_2$-O-N=CH- moiety;

$R_1$ and $R_2$ are independently H, OH, SH, $NH_2$, $C_1$ to $C_{10}$ alkyl, substituted alkyl, alkenyl, alkaryl or aralkyl, alkoxy, thioalkoxy, alkylamino, aralkylamino, substituted alkylamino, heterocycloalkyl, heterocycloalkylamino, aminoalkylamino, polyalkylamino, halo, formyl, keto, benzoxy, carboxamido, thiocarboxamido, ester, thioester, carboxamidine, carbamyl, ureido, guanidino, an RNA cleaving group, a group for improving the pharmacokinetic properties of an oligonucleotide, or a group for improving the pharmacodynamic properties of an oligonucleotide;

$R_3$ is H, OH, $NH_2$, lower alkyl, substitued lower alkyl, alkoxy, lower alkenyl, aralkyl, alkylamino, aralkylamino, substituted alkylamino, heterocyclocalkyl, heterocycloalkylamino, aminoalkylamino, polyalkylamino, a RNA cleaving group, a group for improving the pharmacokinetic properties of an oligonucleotide or a group for improving the pharmacodynamic properties of an oligonucleotide;

$R_4$ is OH, SH, $NH_2$, O-alkyl, S-alkyl, NH-alkyl, O-alkylheterocyclo, S-alkylheterocyclo, N-alkylheterocyclo or a nitrogen-containing heterocycle; and

$R_5$ and $R_6$ are, independently, $C_1$ to $C_6$ alkyl or alkoxy; provided that if $L_1$ is C=O or C=S then $L_2$ is not $NR_3$ or if $L_4$ is C=O or C=S then $L_3$ is not $NR_3$; and that if one of $L_2$ or $L_3$ is C=O or C=S then the other of $L_2$ or $L_3$ is not $NR_3$; $L_2$ is P(O)$R_4$ and $R_4$ is OH and X is OH and $B_x$ is uracil or adenine, then $L_3$ is not O; and that if $L_1$, $L_2$ and $L_4$ are $CH_2$ and X is H or OH and Q is O then $L_3$ is not S, SO or $SO_2$.

The preparation of oligonucleotide analogues in which both the 3' oxy linkage and the 5' oxy linkage are replaced by carbon linkages and the phosphate ester oxy linkage has been replaced by a carbon linkage has remained a significant problem. It has now been found that such oligonucleotide analogues can be prepared from novel dinucleotide analogues, the synthesis of which involves the preparation of novel intermediates. The novel dinucleotide analogues, and oligonucleotides containing units derived therefrom, have good stability towards nuclease hydrolysis and good hybridisation properties, facilitating their use as anti-sense probes and

as pharmaceuticals for the treatment of diseases such as cancer and viruses such as influenza, herpes and HIV. The novel intermediates may themselves be useful as pharmaceuticals in the treatment of such viruses and diseases, as well as diseases which are mediated through inhibition or activation of enzymes/receptors which recognize nucleotide 5' monophosphates as substrates or ligands.

Accordingly, the present invention provides a compound of formula

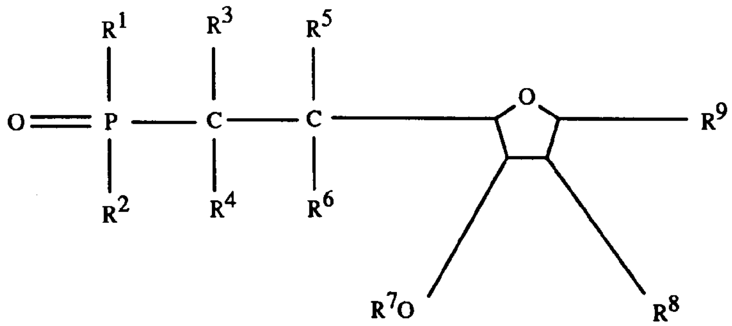

I

where $R^1$ is hydrogen, $R^1{}_a$ or a group of formula

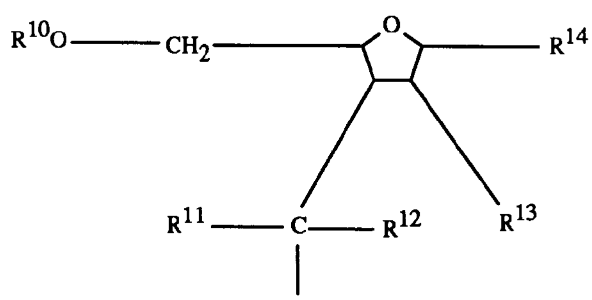

II

$R^1{}_a$ is $R^1{}_b$ or a protecting group Q,
$R^1{}_b$ is $C_1$-$C_{20}$ alkyl, $C_2$-$C_{20}$ alkenyl, $C_3$-$C_{10}$ cycloalkyl, $C_6$-$C_{15}$ aryl, $C_7$-$C_{16}$ aralkyl or a 5- or 6- membered heterocyclic group attached by a carbon atom in the heterocyclic group to the indicated phosphorus atom,
$R^2$ is an unsubstituted or substituted $C_1$- $C_{20}$ alkyl or $C_2$- $C_{20}$ alkenyl group, an unsubstituted or substituted $C_3$- $C_{10}$ cycloalkyl group, an unsubstituted or substituted $C_6$-$C_{15}$ aryl group, an unsubstituted or substituted $C_6$-$C_{15}$ aryl group, an unsubstituted or substituted $C_7$-$C_{16}$ aralkyl group, or a 5- or 6- membered heterocyclic group attached by a carbon atom in the heterocyclic group to the indicated phosphorus atom,
$R^3$ is hydrogen, halogen, hydroxy, $R^{15}$, $-OR^{15}$ or $-OSO_2R^{15}$
$R^4$ is $R^4{}_a$ or together with $R^6$ denotes a valence bond,
$R^4{}_a$ is hydrogen, halogen or $R^{16}$,
$R^5$ is hydrogen, halogen, hydroxy, $R^{17}$, $-OR^{17}$, $-OCOR^{17}$ or $-OSO_2R^{17}$,
$R^6$ is hydrogen, halogen or $R^{18}$, or together with $R^4$ denotes a valence bond
$R^7$ is hydrogen, alkyl-N, N-dialkylphosphoramidyl or $R^7{}_a$, or $R^7O$- together with $R^8{}_a$ denotes a $C_1$-$C_{15}$ hydrocarbylidenedioxy group,
$R^7{}_a$ is $R^{19}$, $-COR^{19}$, $-SO_2R^{19}$ or tri($C_1$-$C_{15}$ hydrocarbyl)silyl,
$R^8$ is $R^8{}_a$ or Z,
$R^8{}_a$ is hydrogen, halogen, hydroxy, $R^{20}$, $-OR^{20}$, $-OCOR^{20}$, $-OSO_2R^{20}$, or tri($C_1$-$C_{15}$ hydrocarbyl)silyloxy, or together with $R^7O$- denotes a $C_1$-$C_{15}$ hydrocarbylidenedioxy group, or together with $R^9$ denotes a $C_1$-$C_{15}$ hydrocarbylidenedioxy group,
$R^9$ is halogen, hydroxy, $-OR^{21}$, $-OCOR^{21}$, $-OSO_2R^{21}$ or $B^2$, or together with $R^8{}_a$ denotes a $C_1$-$C_{15}$ hydrocarbylidenedioxy group,
$R^{10}$ is hydrogen or $R^{10}{}_a$,
$R^{10}{}_a$ is $R^{22}$, $-COR^{22}$, $-SO_2R^{22}$ or tri($C_1$-$C_{15}$ hydrocarbyl)silyl,
$R^{11}$ is hydrogen, halogen, hydroxy, $R^{23}$, $-OR^{23}$, $-OCOR^{23}$, $-OSO_2R^{23}$ or Z,
$R^{12}$ is hydrogen, halogen or $R^{24}$,

$R^{13}$ is $R^{13}{}_a$ or Z,

$R^{13}{}_a$ is hydrogen, halogen, hydroxy, $R^{25}$, $-OR^{25}$, $-OCOR^{25}$, $-OSO_2R^{25}$ or tri($C_1$-$C_{15}$ hydrocarbyl)silyloxy, or together with $R^{14}$ denotes a $C_1$-$C_{15}$ hydrocarbylidenedioxy group,

$R^{14}$ is halogen, hydroxy, $-OR^{26}$, $-OCOR^{26}$, $-OSO_2R^{26}$ or $B^1$ or together with $R^{13}{}_a$ denotes a $C_1$-$C_{15}$ hydrocarbylidenedioxy group,

$R^{15}$, $R^{16}$, $R^{17}$, $R^{18}$, $R^{19}$, $R^{20}$, $R^{21}$, $R^{22}$, $R^{23}$, $R^{24}$, $R^{25}$ and $R^{26}$ are independently a $C_1$ to $C_{10}$ aliphatic group, a $C_3$ to $C_{10}$ cycloaliphatic group, a $C_6$ to $C_{15}$ aromatic group or a $C_7$ to $C_{30}$ araliphatic group,

$B^1$ and $B^2$ are independently each a monovalent nucleoside base radical, and

Z is substituted or unsubstituted $C_6$ to $C_{10}$ aryloxythiocarbonyloxy;

provided that compounds where simultaneously $R^1$, $R^3$, $R^4$ and $R^6$ are hydrogen, $R^2$ is isopropyl, $R^5$ is methoxy, $R^7$ is methyl, $R^8$ and $R^9$ are each hydroxy or each acetoxy or $R^8$ and $R^9$ together denote isopropylidenedioxy, compounds where simultaneously $R^1$, $R^3$, $R^4$ and $R^6$ are hydrogen, $R^2$ is butyl, $R^5$ is methoxy, $R^7$ is benzyl, $R^8$ and $R^9$ are each hydroxy or each acetoxy or $R^8$ and $R^9$ together denote isopropylidenedioxy, and compounds where simultaneously $R^1$, $R^3$, $R^4$, $R^5$, $R^6$ and $R^7$ are hydrogen, $R^2$ is phenyl and $R^8$ and $R^9$ together denote isopropylidenedioxy are excluded.

In compounds of formula I, generally the aliphatic groups are substituted or unsubstituted alkyl or alkenyl groups, the cycloaliphatic groups are substituted or unsubstituted cycloalkyl groups, the aromatic groups are substituted or unsubstituted aryl groups and the araliphatic groups are substituted or unsubstituted aralkyl groups.

The substituted or unsubstituted alkyl groups may be, for example, substituted or unsubstituted methyl, ethyl, n-propyl, isopropyl, n-butyl, isobutyl, tert-butyl, n-pentyl, neopentyl, n-hexyl, n-octyl, 2-ethylhexyl or n-decyl. $R^1$ or $R^2$ as substituted or unsubstituted $C_1$ to $C_{20}$ alkyl may additionally be, for example, substituted or unsubstituted n-dodecyl, n-tetradecyl, n-hexadecyl, n-octadecyl or eicosyl.

The substituted or unsubstituted alkenyl groups may be, for example, substituted or unsubstituted vinyl, allyl, 1-propenyl, isopropenyl, methallyl, 2-butenyl, 1-butenyl, isobutenyl, pentenyl, hexenyl, octenyl or decenyl. $R^1$ or $R^2$ as substituted or unsubstituted alkenyl may additionally be, for example, dodecenyl, hexadecenyl, octadecenyl or eicosenyl.

The substituted or unsubstituted cycloalkyl groups may be, for example, substituted or unsubstituted cyclopropyl, cyclobutyl, cyclopentyl, methylcyclopentyl, cyclohexyl, methylcyclohexyl, dimethylcyclohexyl, tert-butylcyclohexyl, cycloheptyl or cyclooctyl. The substituted or unsubstituted aryl groups may be, for example, substituted or unsubstituted phenyl, o-tolyl, m-tolyl, p-tolyl, o-xylyl, m-xylyl, p-xylyl, alpha-naphthyl, beta-naphthyl, dimethylnaphthyl or anthryl.

$R^1$ or $R^2$ as substituted or unsubstituted $C_7$ to $C_{16}$ aralkyl may be, for example, substituted or unsubstituted benzyl, 4-methylbenzyl, 2-phenylethyl, 2-phenylpropyl, 3-phenylpropyl or diphenylmethyl. The other substituted or unsubstituted $C_7$ to $C_{30}$ aralkyl groups may be, for example, substituted or unsubstituted benzyl, 4-methylbenzyl, 2-phenylethyl, 2-phenylpropyl, 3-phenylpropyl, diphenylmethyl or triphenylmethyl.

Preferably, the alkyl groups are $C_1$ to $C_4$ alkyl, the alkenyl groups are $C_2$ to $C_4$ alkenyl, the cycloalkyl groups are $C_5$ to $C_8$ cycloalkyl, the aryl groups $C_6$ to $C_{10}$ aryl, the $C_7$ to $C_{16}$ aralkyl group is $C_7$ to $C_9$ aralkyl and the $C_7$ to $C_{30}$ aralkyl groups are $C_7$ to $C_{20}$ aralkyl, any of which are substituted or unsubstituted. More preferably, these groups are unsubstituted or substituted by halogen, hydroxy, $C_1$ to $C_4$ alkoxy, cyano, nitro, amino, $C_1$ to $C_4$ alkylamino or di($C_1$-$C_4$ alkyl) amino, the unsubstituted groups being especially preferred.

When $R^1$ in formula I is a protecting group Q, this may be any group which is known to be effective in protecting P-H bonds whilst reactions are carried out which would affect such bonds and be readily removable after such reactions to generate a P-H bond. Such protecting groups may be, for example, those in compounds of formula Ia of EP 0009348, or those in compounds described in Aust. J. Chem. 33, 292 (1980) or US 4 933 478.

Preferred protecting groups Q are $C_1$ to $C_{20}$ hydrocarbyl groups substituted on the carbon atom thereof attached to the indicated phosphorus atom by at least one hydroxy or $C_1$-$C_{10}$ alkoxy group, including those of formula

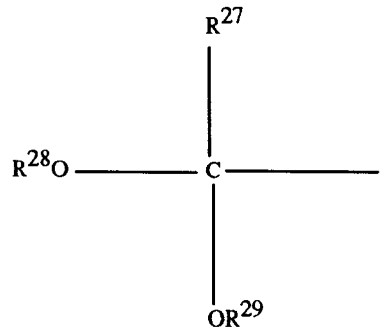

III

where $R^{27}$ is hydrogen, $C_1$-$C_{10}$ alkyl, $C_3$-$C_8$ cycloalkyl, $C_6$-$C_{10}$ aryl or $C_7$-$C_{11}$ aralkyl and $R^{28}$ and $R^{29}$ are independently $C_1$-$C_{10}$ alkyl. Preferred groups of formula III are those where $R^{27}$ is hydrogen or $C_1$-$C_4$ alkyl and $R^{28}$ and $R^{29}$ are each $C_1$-$C_4$ alkyl. In especially preferred compounds, Q is a group of formula III where $R^{27}$ is hydrogen or methyl and $R^{28}$ and $R^{29}$ are each methyl or ethyl.

When a tri($C_1$-$C_{15}$ hydrocarbyl)silyl or tri($C_1$-$C_{15}$ hydrocarbyl)silyloxy group is present in compounds of formula I, the tri($C_1$-$C_{15}$ hydrocarbyl)silyl radical may be, for example, trialkylsilyl such as trimethylsilyl, triethysilyl, tri-n-propylsilyl, tri-isopropysilyl, tri-n-butylsilyl, tri-isobutysilyl, tri-tert-butylsilyl, isopropyldimethylsilyl, tert.butyldimethylsilyl or 1,1,2,2-tetramethylethyldimethylsilyl (thexyldimethylsilyl), aryldialkylsilyl such as phenyldimethylsilyl, phenyldiethylsilyl, phenyldiisopropylsilyl or phenyl di-tert-butylsilyl, or alkyldiarylsilyl such as isopropyldiphenylsilyl or tert-butyldiphenylsilyl, preferably $C_1$-$C_6$ alkyldi($C_6$-$C_8$ aryl)silyl, especially tert-butyldiphenylsilyl, or branched $C_2$-$C_{10}$ alkyl di ($C_1$-$C_4$ alkyl)silyl, especially thexyldimethylsilyl.

When a $C_1$-$C_{15}$ hydrocarbylidenedioxy group is present in compounds of formula I, this may be a group of formula

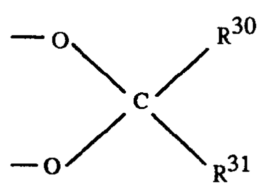

IV

or

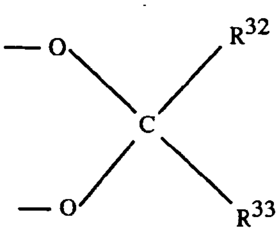

IVA

where $R^{30}$, $R^{31}$, $R^{32}$ and $R^{33}$ are independently hydrogen, $C_1$ to $C_{10}$ alkyl, $C_3$ to $C_8$ cycloalkyl or $C_6$ to $C_{10}$ aryl. Preferred such groups are those where $R^{30}$ and $R^{32}$ are $C_1$ to $C_4$ alkyl, particularly methyl or ethyl. An especially preferred $C_1$-$C_{15}$ hydrocarbylidenedioxy group is an isopropylidenedioxy group.

$R^8$, $R^{11}$ or $R^{13}$ as substituted or unsubstituted $C_6$-$C_{10}$ aryloxythiocarbonyloxy may be, for example, substituted or unsubstituted phenyloxythiocarbonyloxy, preferably $C_1$-$C_4$ alkyl- or halogen- substituted phenyloxythiocarbonyloxy, especially p-tolyloxythiocarbonyloxy or pentafluorophenoxythiocarbonyloxy.

$R^9$ or $R^{14}$ as a monovalent nucleoside base radical $B^1$ or $B^2$ respectively may be the same or different and may be a radical of a naturally occuring nucleoside base, such as adeninyl, cytosinyl, thyminyl, guaninyl or uracilyl, which may be unsubstituted or substituted, for example on an amino nitrogen atom by an acyl group such as acetyl, an aralkyl oxyalkyl group such as benzyloxymethyl or an aracyl group such as benzoyl or nitrobenzoyl, or a synthetic analogue thereof. Preferably, $R^9$ or $R^{14}$ as a monovalent nucleoside base radical is unsubstituted or substituted thyminyl, cytosinyl, guaninyl or adeninyl, especially 1-thyminyl or N-benzyloxymethyl-1-thyminyl.

$R^2$ is preferably $C_1$ to $C_4$ alkyl, more preferably methyl or ethyl; $C_2$ to $C_4$ alkenyl, more preferably vinyl or allyl; $C_5$ to $C_8$ cycloalkyl, more preferably cyclopentyl, cyclohexyl or methylcyclohexyl; $C_6$ to $C_{10}$ aryl, more preferably phenyl, tolyl or naphthyl; or $C_7$ to $C_9$ aralkyl, more preferably benzyl. In certain especially preferred compounds, $R^2$ is methyl, cyclohexyl or phenyl.

Preferably $R^3$ is hydrogen, halogen (usually fluorine or chlorine), hydroxy, $C_1$ to $C_4$ alkyl (more preferably methyl or ethyl), $C_1$ to $C_4$ alkoxy (more preferably methoxy or ethoxy), $C_7$ to $C_9$ aralkyloxy (more preferably benzyloxy), or $-OCOR^{15}$ or $-OSO_2R^{15}$ where $R^{15}$ is $C_1$ to $C_4$ alkyl, particularly methyl or ethyl, or $C_6$ to $C_{10}$ aryl, particularly phenyl or p-tolyl, and $R^4$ is hydrogen, halogen (usually fluorine or chlorine) or $C_1$ to $C_4$ alkyl, particularly methyl or ethyl. In especially preferred compounds, $R^3$ and $R^4$ are each hydrogen.

Preferably $R^5$ is hydrogen, halogen (usually fluorine or chlorine), hydroxy, $C_1$to $C_4$ alkyl (more preferably

methyl or ethyl), $C_1$ to $C_4$ alkoxy (more preferably methoxy or ethoxy), $C_7$ to $C_9$ aralkyloxy (more preferably benzyloxy), or $-OCOR^{17}$ or $-OSO_2R^{17}$ where $R^{17}$ is $C_1$ to $C_4$ alkyl, particularly methyl or ethyl, or $C_6$ to $C_{10}$ aryl, particularly phenyl or p-tolyl, and $R^6$ is hydrogen, halogen (usually fluorine or chlorine) or $C_1$ to $C_4$ alkyl, particularly methyl or ethyl. In especially preferred compounds, $R^5$ and $R^6$ are each hydrogen.

$R^7$ is preferably hydrogen, cyano-$C_1$-$C_4$ alkyl-N, N-di($C_1$-$C_4$ alkyl)phosphoramidyl, $C_1$ to $C_4$ alkyl (more preferably methyl or ethyl), $C_7$ to $C_9$ aralkyl (more preferably benzyl), or $-COR^{19}$ or $-SO_2R^{19}$ where $R^{19}$ is substituted or unsubstituted $C_1$ to $C_4$ alkyl (more preferably methyl or ethyl) or substituted or unsubstituted $C_6$ to $C_{10}$ aryl (more preferably phenyl or tolyl). In some especially preferred compounds, $R^7$ is hydrogen, 2-cyanoethyl-N, N-diisopropylphosphoramidyl, acetyl, benzyl or benzoyl.

$R^8$ is preferably hydrogen, hydroxy or $-OR^{20}$, $-OCOR^{20}$ or $-OSO_2R^{20}$ where $R^{20}$ is substituted or unsubstituted $C_1$ to $C_4$ alkyl (more preferably methyl or ethyl) or substituted or unsubstituted $C_6$ to $C_{10}$ aryl (more preferably phenyl, tolyl or naphthyl), or together with $R^9$ denotes a $C_1$-$C_5$ alkylidenedioxy group (more preferably an isopropylidenedioxy group). In certain especially preferred compounds, $R^8$ is hydrogen, hydroxy, methoxy or acetoxy, or together with $R^9$ denotes an isopropylidenedioxy group.

Preferably, $R^9$ is a monovalent nucleoside base radical $B^2$, hydroxy or $-OR^{21}$, $-OCOR^{21}$ or $-OSO_2R^{21}$ where $R^{21}$ is substituted or unsubstituted $C_1$ to $C_4$ alkyl (more preferably methyl or ethyl) or $C_6$-$C_{10}$ aryl (more preferably phenyl, tolyl or naphthyl), or together with $R^8$ denotes a $C_1$-$C_5$ alkylidenedioxy group. In some especially preferred compounds, $R^9$ is thyminyl, N-benzyloxymethylthyminyl, hydroxy, methoxy or acetoxy, or together with $R^8$ denotes an isopropylidenedioxy group.

$R^{10}$ is preferably hydrogen, substituted or unsubstituted $C_1$ to $C_4$ alkyl (more preferably methyl or ethyl), substituted or unsubstituted $C_7$ to $C_{20}$ aralkyl (more preferably benzyl, diphenylmethyl, triphenylmethyl or dimethoxytriphenylmethyl), $-COR^{22}$ or $-SO_2R^{22}$ where $R^{22}$ is substituted or unsubstituted $C_1$ to $C_4$ alkyl or substituted or unsubstituted $C_6$ to $C_{10}$ aryl (more preferably phenyl, tolyl or naphthyl), or ($C_1$ to $C_6$ alkyl)di($C_6$-$C_8$ aryl)silyl. In certain especially preferred compounds, $R^{10}$ is hydrogen, benzoyl, tert-butyldiphenylsilyl or dimethoxytriphenylmethyl.

Preferably $R^{11}$ is hydrogen, halogen, hydroxy, $-OCOR^{23}$ or $-OSO_2R^{23}$ where $R^{23}$ is substituted or unsubstituted $C_1$ to $C_4$ alkyl or $C_6$ to $C_{10}$ aryl (more preferably methyl, ethyl, phenyl, tolyl or naphthyl), or $C_1$-$C_4$ alkyl- or halogen- substituted phenyloxythiocarbonyloxy, and $R^{12}$ is hydrogen or halogen. In some especially preferred compounds $R^{11}$ is hydrogen, hydroxy or p-tolyloxythiocarbonyloxy and $R^{12}$ is hydrogen.

Preferably $R^{13}$ is hydrogen, hydroxy, or $-OR^{25}$, $-OCOR^{25}$ or$-OSO_2R^{25}$ where $R^{25}$ is substituted or unsubstituted $C_1$ to $C_4$ alkyl or $C_6$ to $C_{10}$ aryl (more preferably methyl, ethyl, phenyl, tolyl or naphthyl), or together with $R^{14}$ denotes a $C_1$-$C_5$ alkylidenedioxy group. In certain especially preferred compounds, $R^{13}$ is hydrogen, hydroxy, methoxy or acetoxy, or together with $R^{14}$ denotes an isopropylidenedioxy group.

Preferably $R^{14}$ is a monovalent nucleotide base radical $B^1$, hydroxy, or $-OR^{26}$, $-OCOR^{26}$ or $-OSO_2R^{26}$ where $R^{26}$ is substituted or unsubstituted $C_1$ to $C_4$ alkyl or $C_6$ to $C_{10}$ aryl (more preferably methyl, ethyl, phenyl, tolyl or naphthyl), or together with $R^{13}$ denotes a $C_1$ to $C_5$ alkylidenedioxy group. In certain especially preferred compounds, $R^{14}$ is thyminyl, N-benzyloxymethylthyminyl, hydroxy, methoxy or acetoxy, or together with $R^{13}$ denotes an isopropylidenedioxy group.

$R^1$ is preferably hydrogen, a protecting group Q or a group of formula II where $R^{10}$ to $R^{14}$ have the preferred meanings hereinbefore defined. In certain especially preferred compounds, $R^1$ is hydrogen, a protecting group of formula III where $R^{27}$ is hydrogen or methyl and $R^{28}$ and $R^{29}$ are each methyl or ethyl, or a group of formula II where $R^{10}$ is hydrogen, benzoyl, tert-butyldiphenylsilyl or dimethoxytriphenylmethyl, $R^{11}$ is hydrogen, hydroxy or p-tolyloxythiocarbonyloxy, $R^{12}$ is hydrogen, $R^{13}$ is hydrogen, hydroxy, methoxy or acetoxy, $R^{14}$ is thyminyl, N-benzyloxymethylthyminyl, hydroxy, methoxy or acetoxy, or $R^{13}$ and $R^{14}$ together denote an isopropylidenedioxy group.

Compounds of the invention may be in the form of one of the possible isomers, for example as a diastereomer, an optical isomer or a racemate, or a mixture thereof.

Preferred isomers of compounds of formula I are those of formula

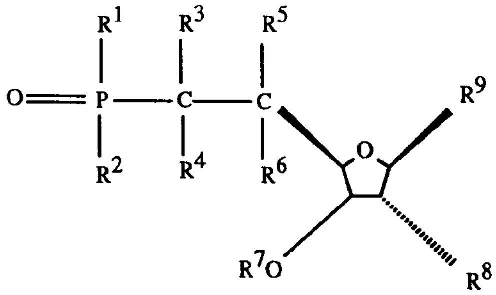

V

where $R^2$ to $R^7$ are as hereinbefore defined, $R^1$ is hydrogen, $R^1_a$ or a group of formula

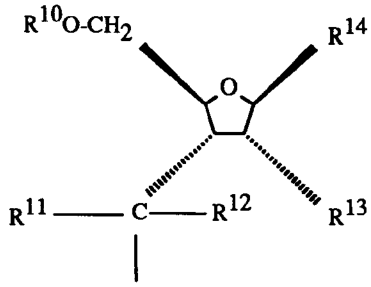

VI

where $R^{10}$ to $R^{12}$ are as hereinbefore defined, $R^8$ is hydrogen, halogen, hydroxy, $R^{20}$, $-OR^{20}$, $-OCOR^{20}$, $-OSO_2R^{20}$, tri($C_1$-$C_{15}$ hydrocarbyl)silyloxy or Z, $R^9$ is $B^2$, halogen, hydroxy, $-OR^{21}$, $-OCOR^{21}$ or $-OSO_2R^{21}$, $R^{13}$ is hydrogen, halogen, hydroxy, $R^{25}$, $-OR^{25}$, $-OCOR^{25}$, $-OSO_2R^{25}$, tri($C_1$-$C_{15}$ hydrocarbyl)silyloxy or Z, $R^{14}$ is $B^1$, halogen, hydroxy, $-OR^{26}$, $-OCOR^{26}$ or $-OSO_2R^{26}$ and Z is as hereinbefore defined, and those of formula

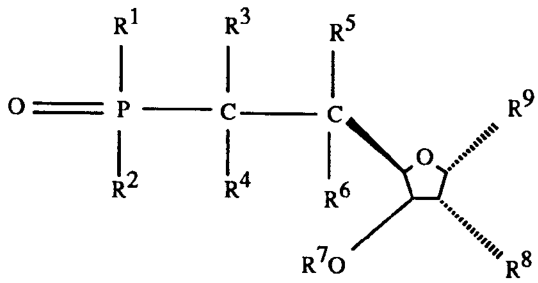

VA

where $R^2$ to $R^8$ are as hereinbefore defined, $R^1$ is hydrogen, $R^1_a$ or a group of formula

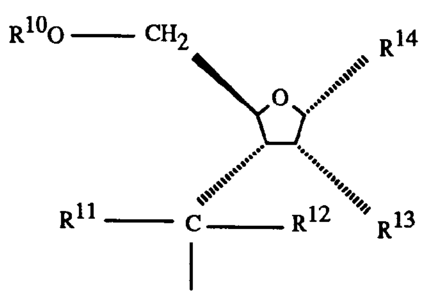

VIA

where $R^{10}$ to $R^{13}$ are as hereinbefore defined, $R^9$ is halogen, hydroxy, $-OR^{21}$, $-OCOR^{21}$ or $-OSO_2R^{21}$ or together with $R^8$ denotes a $C_1$-$C_{15}$ alkylidenedioxy group and $R^{14}$ is halogen, hydroxy, $-OR^{26}$, $-OCOR^{26}$ or $-OSO_2R^{26}$, or together with $R^{13}$ denotes a $C_1$-$C_{15}$ alkylidenedioxy group.

Preferred compounds of the invention include those where $R^1$ is a group of formula II which are dinucleotide analogues of formula

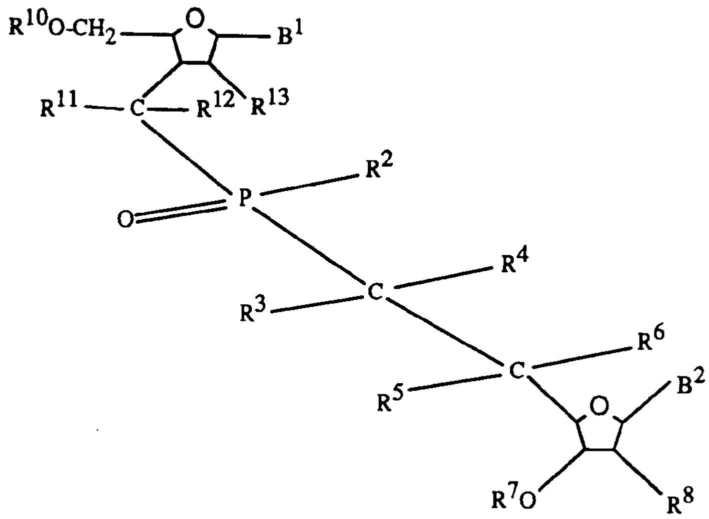

VII

where $B^1$, $B^2$ and $R^2$ to $R^{13}$ are as hereinbefore defined.

Preferred isomers of such compounds are those of formula

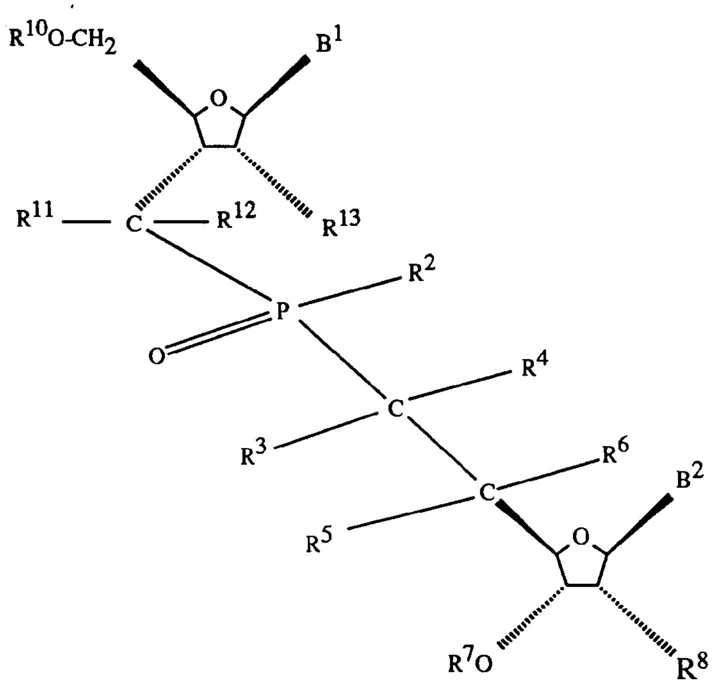

VIII

Most especially preferred compounds of the invention are those hereinafter described in the Examples.

Compounds of formula I where $R^1$ is a group of formula II in which $R^{11}$ is hydroxy and $R^{12}$ is hydrogen may be prepared by reacting a compound of formula I where $R^1$ is hydrogen with an aldehyde of formula

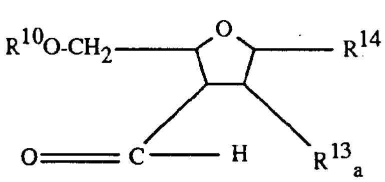

IX

where $R^{10}$, $R^{13}{}_a$ and $R^{14}$ are as hereinbefore-defined, in the presence of a base, preferably a non-nucleophilic

base, for example a hindered amine such as 1, 8-diazabicyclo [5.4.0] undec-7-ene (DBU) or 1,5-diazabicyclo [4.3.0] non-5-ene or an alkali metal alkoxide such as the tert-butoxide of sodium or potassium. The reaction may be carried out at temperatures of -20 to 100°C, preferably 10 to 30°C. It is preferably effected in an organic solvent, for example a hydrocarbon such as benzene, toluene or xylene, a halohydrocarbon such as dichloroethane or methylene chloride or, preferably, an ether such as diethyl ether, dioxan, especially, tetrahydrofuran.

Compounds of formula I where $R^1$ is a group of formula II in which $R^{11}$ is hydroxy and $R^{12}$ is hydrogen may also be prepared by reaction of a compound of formula I where $R^1$ is hydrogen with a silylating agent to give a P(III) silyl compound and reacting the latter with an aldehyde of formula IX as hereinbefore defined. The silylating agent may be, for example, a trialkylhalosilane such as trimethylchlorosilane or triethylchlorosilane which is reacted with the compound of Formula I in the presence of tertiary base such as pyridine or triethylamine. The reaction between the compound of formula I where $R^1$ is hydrogen and the silane may be carried out at temperatures ranging from -20°C to 150°C and can be effected with or without the use of a solvent such as diethylether, tetrahydrofuran, dioxan or toluene. Alternatively, an excess of the silane can be used as diluent. The silylating agent may alternatively be hexamethyldisilazide, which may be reacted with the compound of Formula I in the absence of a solvent at 100-200°C. The reaction of the P(III) silyl compound with the aldehyde of formula IX may be carried out under conditions conventional for substitution reactions on P(III) species. It is preferably carried out by the Arbuzov method, e.g. at temperatures between ambient and elevated temperatures such as 160°C, followed by hydrolysis of the intermediate silyl species.

Aldehydes of formula IX may be prepared by reaction of the corresponding $3^1$-iodo nucleoside with carbon monoxide and tris(trimethylsilyl)silane in the presence of a free radical initiator such as $2,2^1$-azobis(isobutyronitrile), by reduction of the corresponding $3^1$-cyano nucleoside with diisobutylaluminium hydride or otherwise as described in WO 92/20823. Aldehydes of formula IX may also be prepared by treatment of the corresponding $3^1$-amino nucleoside with nitrite as described by S. Shuto et al, Nucleosides & Nucleotides, 1 (3), 263-272(1982), or by hydrolysis of the corresponding $3^1$-C-(4,5-dihydro-5-methyl-1,3,5-dithiazin-2-yl) nucleoside as described by Bamford et al, J. Med. Chem. 1990, 33, 2494.

Compounds of formula I where $R^1$ is a group of formula II may also be prepared by reacting a compound of formula I where $R^1$ is hydrogen with a compound of formula

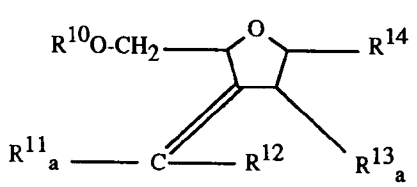

X

where $R^{10}$, $R^{12}$, $R^{13}_a$ and $R^{14}$ are as hereinbefore defined and $R^{11}_a$ is hydrogen, halogen or $R^{23}$ as hereinbefore defined, in the presence of a free radical initiator. Suitable initiators include azo compounds such as azobis(isobutyronitrile), peroxides such as benzoyl peroxide, tert-butyl peroxide or 2,2-bis(tert-butylperoxy)propane, peresters such as tert-butyl perbenzoate or tert-butyl per-2-ethylhexanoate, percarbonates such as diacetyl perdicarbonate or bis(4-tert-butylcyclohexyl)perdicarbonate or persalts such as potassium persulphate. The initiator is generally used in an amount of 0.1 to 100% mol, preferably 5 to 15, mol%, per mol of the compound of formula X. In general, 1 to 5 equivalents, preferably 1 to 2 equivalents, of the compound of formula I where $R^1$ is hydrogen are used per equivalent of the compound of formula X. The reaction may be carried out without a solvent, but is preferably carried out in an organic solvent, usually an aromatic hydrocarbon such as benzene, toluene or xylene. It may be carried out at temperatures of 30 to 150°C, preferably 70 to 100°C.

Compounds of formula I which are dinucleotide analogues of formula VII where $B^1$ and $B^2$ are the same monovalent nucleoside base radical can be prepared by glycosylation of compounds of formula I where $R^1$ is a group of formula II, $R^7$ is $-OR^{19}$, $R^8$ is $-OCOR^{20}$, $R^9$ is $-OCOR^{21}$, $R^{13}$ is $-OCOR^{25}$ and $R^{14}$ is $-OCOR^{26}$, i.e. compounds of formula

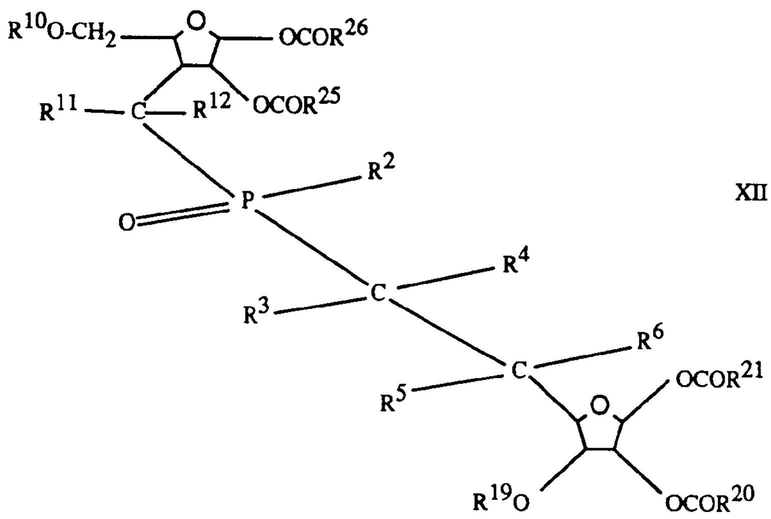

XII

where $R^2$ to $R^6$, $R^{10}$, $R^{11}$, $R^{12}$, $R^{19}$, $R^{20}$, $R^{21}$, $R^{25}$ and $R^{26}$ are as hereinbefore defined, with a base of formula $B^1H$ where $B^1$ is as hereinbefore defined.

Glycosylation is generally effected in the presence of a silylating agent such as trimethylsilyl chloride, bis(trimethylsilyl)acetamide or hexamethyldisilazane and a catalyst such as a fluoroalkanesulphonate salt in an organic solvent such as acetonitrile or 1,2-dichloroethane at a temperature of 40-90°C. Where the compound of formula XII contains a hydroxyl group, the glycosylation product may be reacted with an aqueous acid such as acetic acid to regenerate a hydroxyl group which has been silylated during glycosylation.

The base of formula $B^1H$ is a readily available nucleoside base such as adenine, cytosine, guanine, thymine or uracil or a substituted derivative or analogue thereof prepared by known procedures.

Compounds of formula XII where $R^{20}$, $R^{21}$, $R^{25}$ and $R^{26}$ are the same can be prepared by esterification of a compound of formula I where $R^1$ is a group of formula II, $R^7$ is $-OR^{19}$, $R^8$, $R^9$, $R^{13}$ and $R^{14}$ are each hydroxy and $R^{10}$ is $R^{10}_a$, i.e. a compound of formula

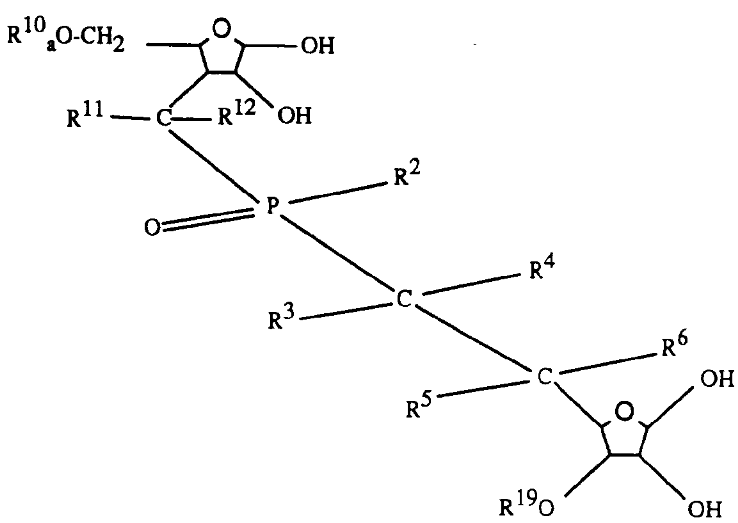

XIII

where $R^2$ to $R^6$, $R^{10}_a$, $R^{11}$, $R^{12}$ and $R^{19}$ are as hereinbefore defined, with an acid of formula $R^{20}$ COOH or an anhydride or acid halide thereof. This esterification reaction may be carried out using conventional procedures, for example by reaction with an anhydride or acid halide in the presence of an organic base such as pyridine, dimethylaminopyridine or triethylamine, optionally in an organic solvent, for example a halohydrocarbon such as methylene chloride, an aromatic hydrocarbon such as toluene, an ether such as tetrahydrofuran or an ester such as ethyl acetate. The esterification can be effected at a temperature from -70°C to 80°C.

Compounds of formula XIII may be prepared by acidic hydrolysis of a compound of formula I where $R^1$ is a group of formula II, $R^7$ is $-OR^{19}$, $R^8$ is hydroxy, $R^9$ is $-OR^{21}$, $R^{10}$ is $R^{10}_a$ and $R^{13}$ and $R^{14}$ together denote a $C_1$ to $C_{15}$ hydrocarbylidenedioxy group, e.g. a compound of formula

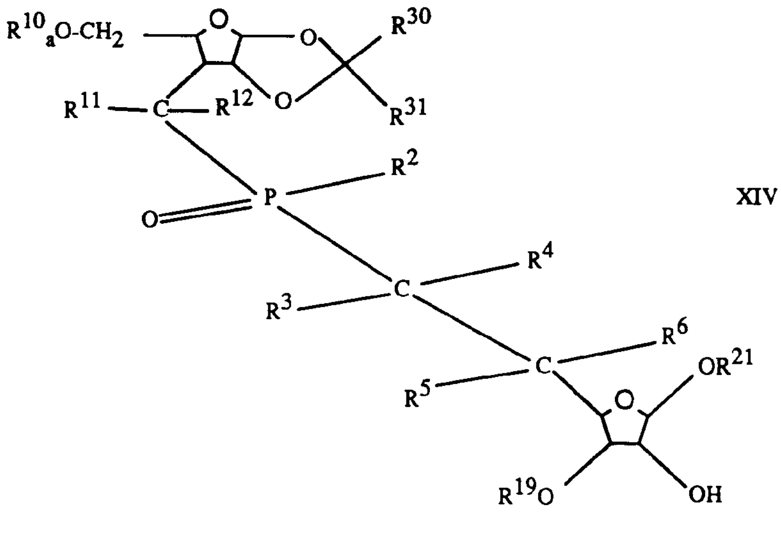

XIV

where $R^2$ to $R^6$, $R^{10}_a$, $R^{11}$, $R^{12}$, $R^{19}$, $R^{21}$, $R^{30}$ and $R^{31}$ are as hereinbefore defined.

The acidic hydrolysis can be carried out by reaction with an organic acid such as formic acid, acetic acid, trifluoroacetic acid or p-toluenesulphonic acid together with water and, optionally, an organic solvent such as dioxan or tetrahydrofuran. It may also be carried out with an aqueous inorganic acid such as hydrochloric acid or sulphuric acid. Hydrolysis with organic or inorganic acids may be effected at temperatures from -30°C to 50°C; usually it is effected at ambient temperature.

The acidic hydrolysis of compounds of formula XIV can also be carried out by heating with an acidic ion exchange resin and a mixture of water and a polar organic solvent such as dimethyl formamide or dichloromethane or an ether such as diethyl ether, tetrahydrofuran, diethoxymethane or dimethoxyethane, generally at a temperature of 40 to 100°C.

Compounds of formula XIV can be prepared by reacting a compound of formula

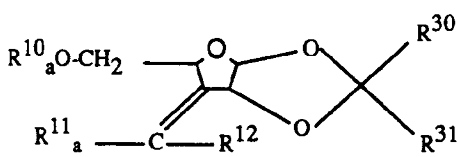

XV

where $R^{10}_a$, $R^{11}_a$, $R^{12}$, $R^{30}$ and $R^{31}$ are as hereinbefore defined, which is a compound of formula X where $R_a^{13}$ and $R^{14}$ together denote a $C_1$ to $C_{15}$ hydrocarbylidenedioxy group, with a compound of formula

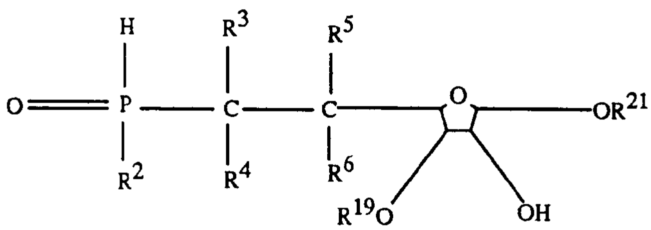

XVI

where $R^2$ to $R^6$, $R^{19}$ and $R^{21}$ are as hereinbefore defined, which is a compound of formula I where $R^1$ is H, $R^7$ is $R^{19}$, $R^8$ is hydroxy and $R^9$ is $-OR^{21}$, in the presence of a free radical initiator. The initiator and reaction conditions may be as hereinbefore described for the reaction of compounds of formula X with compounds of formula I where $R^1$ is hydrogen.

Compounds of formula XIII can also be prepared by acidic hydrolysis of compounds of formula I where $R^1$ is a group of formula II, $R^7$ is $R^{19}$, $R^{10}$ is $R^{10}_a$, $R^8$ and $R^9$ together and $R^{13}$ and $R^{14}$ together each denote a $C_1$ to $C_{15}$ hydrocarbylidenedioxy group, e.g. a compound of formula

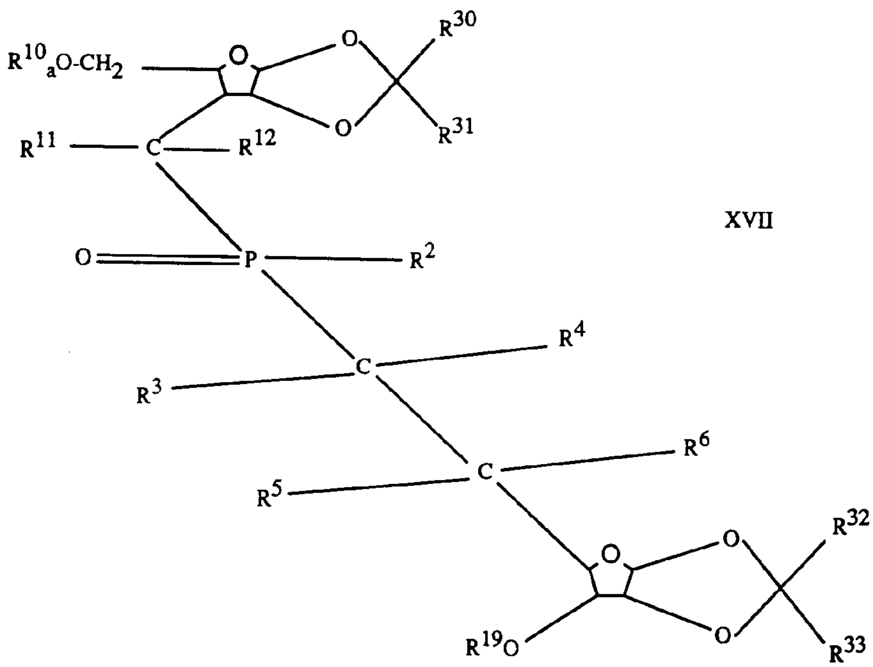

XVII

where $R^2$ to $R^6$, $R^{10}_a$, $R^{11}$, $R^{12}$, $R^{19}$, $R^{30}$, $R^{31}$, $R^{32}$ and $R^{33}$ are as hereinbefore defined.

The acidic hydrolysis can be carried out using an organic or inorganic acid under the same conditions used for the acidic hydrolysis of compounds of formula XIV.

Compounds of formula XVII can be prepared by reacting a compound of formula XV with a compound of formula

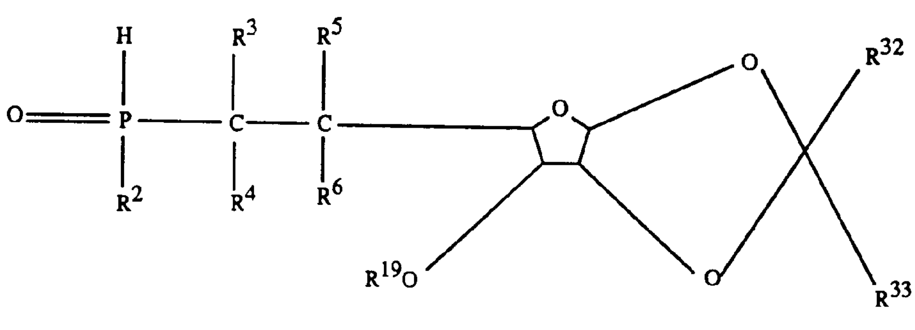

XVIIA

where $R^2$ to $R^6$, $R^{19}$, $R^{32}$ and $R^{33}$ are as hereinbefore defined, which is a compound of formula X where $R^1$ is hydrogen, $R^7$ is $R^{19}$ and $R^8$ and $R^9$ together denote a $C_1$-$C_{15}$ hydrocarbylidenedioxy group, in the presence of a free radical initiator. The initiator and reaction conditions may be as hereinbefore described for the reaction of compounds of formula X with compounds of formula I where $R^1$ is hydrogen.

Compounds of formula I which are dinucleotide analogues of formula VII where $B^1$ and $B^2$ are the same or different monovalent nucleoside base radicals can be prepared by glycosylation with a base of formula $B^1H$, where $B^1$ is as hereinbefore defined, of a compound of formula I where $R^1$ is a group of formula II, $R^7$ is $R^{19}$, $R^8$ is $-OCOR^{20}$, $R^9$ is $B^2$, $R^{10}$ is $R^{10}_a$, $R^{13}$ is $-OCOR^{25}$ and $R^{14}$ is $-OCOR^{26}$, i.e.
a compound of formula

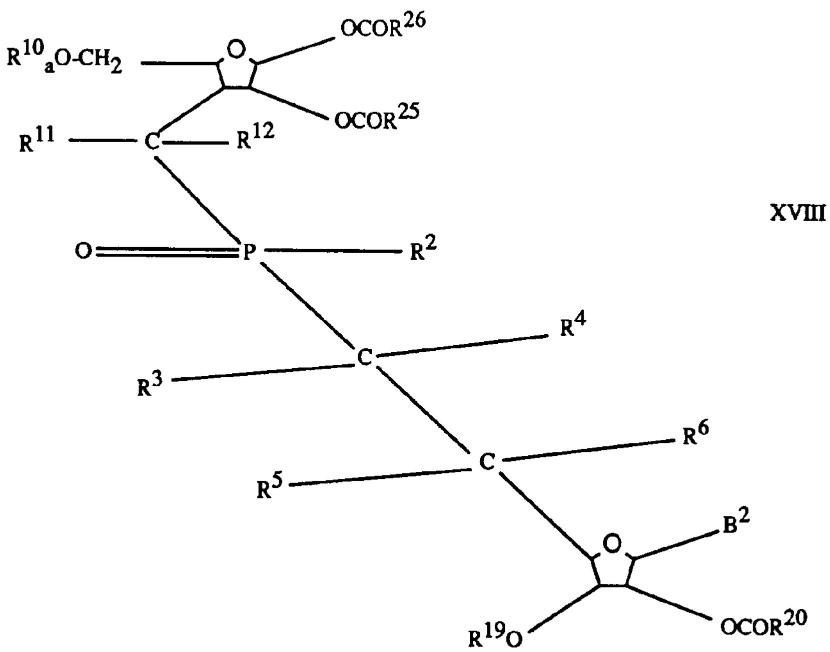

XVIII

where $B^2$, $R^2$ to $R^6$, $R^{10}{}_a$, $R^{11}$, $R^{12}$, $R^{19}$, $R^{20}$, $R^{25}$ and $R^{26}$ are as hereinbefore defined. The glycosylation can be effected using the method described for glycosylation of compounds of formula XII.

Compounds of formula XVIII where $R^{25}$ and $R^{26}$ are the same can be obtained by reaction of corresponding compounds where $R^{13}$ and $R^{14}$ are each hydroxy, i.e. compounds of formula

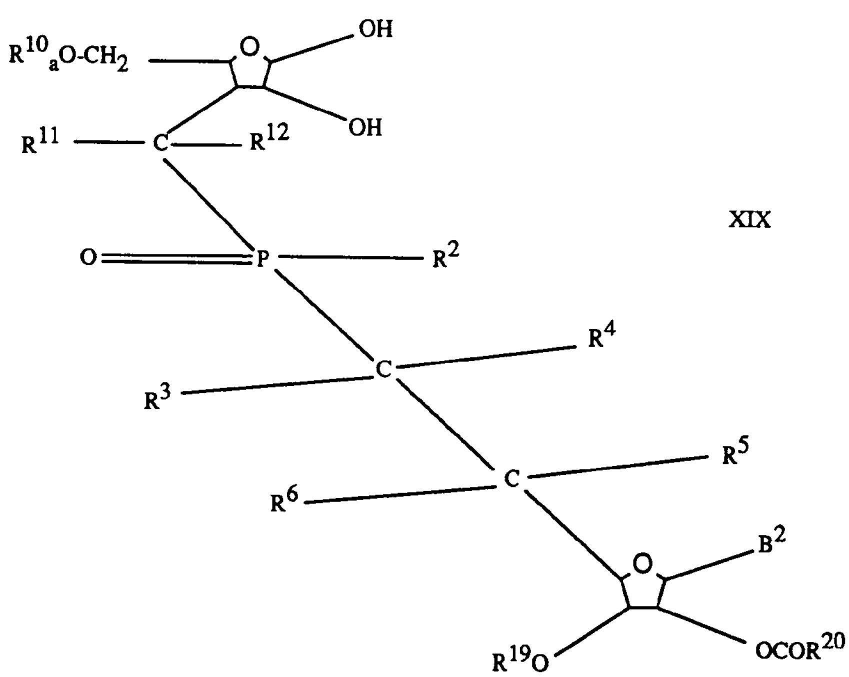

XIX

where $B^2$, $R^2$ to $R^6$, $R^{10}{}_a$, $R^{11}$, $R^{12}$, $R^{19}$ and $R^{20}$ are as hereinbefore defined, with an acid of formula $R^{25}COOH$ or an anhydride or acid halide thereof. This esterification reaction may be carried out using methods hereinbefore described for the esterification of compounds of formula XIII.

Compounds of formula XIX can be prepared by acid hydrolysis of compounds of formula I where $R^1$ is a group of formula II, $R^7$ is $R^{19}$, $R^8$ is $-OCOR^{20}$, $R^9$ is $B^2$, $R^{10}$ is $R^{10}{}_a$ and $R^{13}$ and $R^{14}$ together denote a $C_1$ to $C_{15}$

hydrocarbylidenedioxy group, e.g. compounds of formula

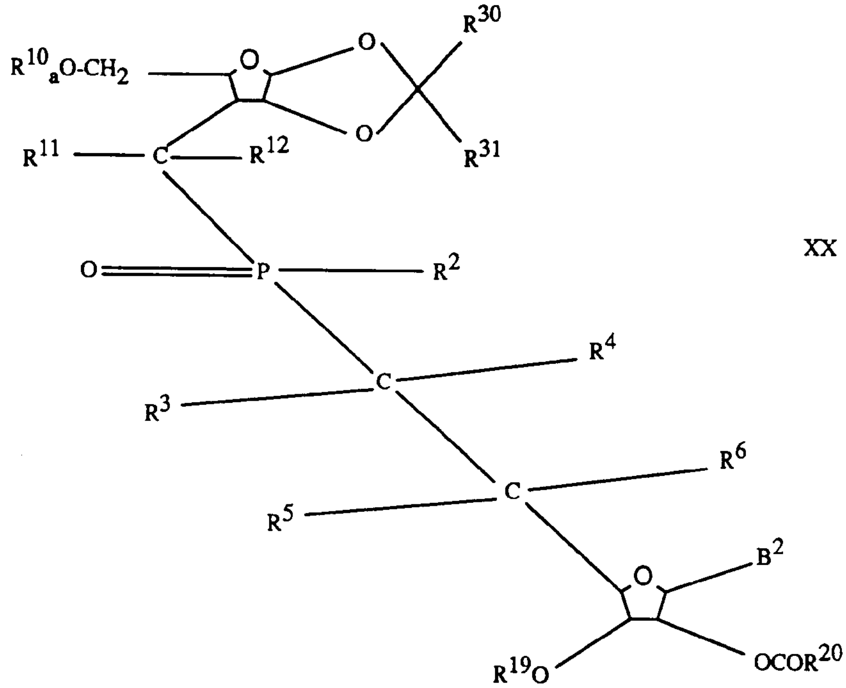

XX

where $B^2$, $R^2$ to $R^6$, $R^{10}{}_a$, $R^{11}$, $R^{12}$, $R^{19}$, $R^{20}$, $R^{30}$ and $R^{31}$ are as hereinbefore defined. The acid hydrolysis may be effected using the methods hereinbefore described for the acid hydrolysis of compounds of formula XIV.

Compounds of formula XX may be obtained by glycosylation, with a base of formula $B^2H$, where $B^2$ is as hereinbefore defined, of compounds of formula I where $R^1$ is a group of formula II, $R^7$ is $R^{19}$, $R^8$ is $-OCOR^{20}$, $R^9$ is $-OR^{21}$, $R^{10}$ is $R^{10}{}_a$ and $R^{13}$ and $R^{14}$ together denote a $C_1$-$C_{15}$ hydrocarbylidenedioxy group, e.g. compounds of formula

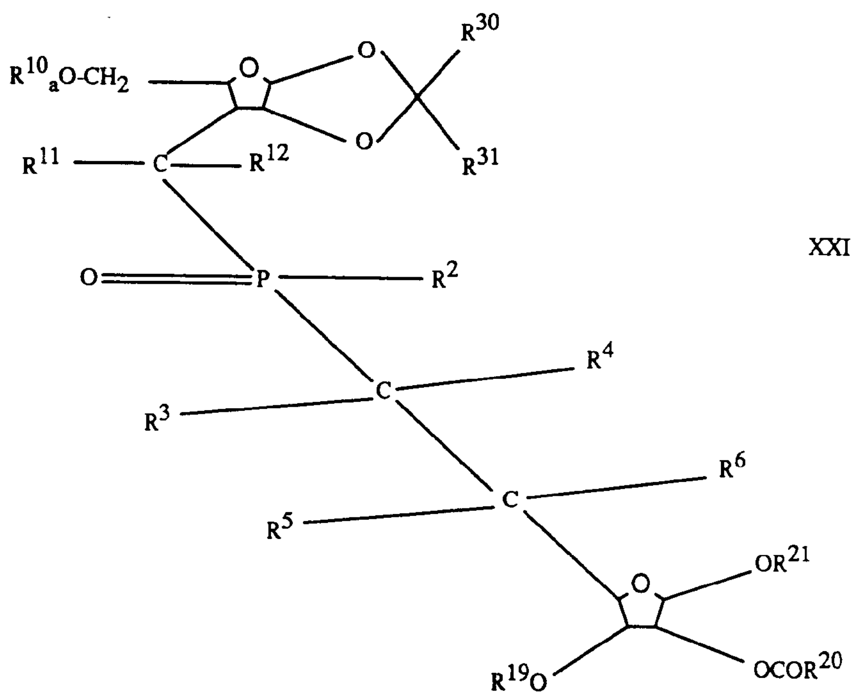

XXI

where $R^2$ to $R^6$, $R^{10}{}_a$, $R^{11}$, $R^{12}$, $R^{19}$, $R^{20}$, $R^{21}$, $R^{30}$ and $R^{31}$ are as hereinbefore defined. The glycosylation may be effected using the method hereinbefore described for the glycosylation of compounds of formula XII.

The base of formula $B^2H$ is a readily available nucleoside base such as adenine, cytosine, guanine, thymine

or uracil or a substituted derivative or analogue thereof prepared by known procedures.

Compounds of formula XXI can be prepared by esterification of compounds of formula XIV with an acid of formula $R^{20}COOH$ or an anhydride or acid halide thereof. This esterification may be effected using the methods hereinbefore described for the esterification of compounds of formula XIII.

Compounds of formula I which are dinucleotide analogues of formula VII where $B^1$ and $B^2$ are the same monovalent nucleoside radical can be prepared by glycosylation, with a base of formula $B^1H$, where $B^1$ is as hereinbefore defined, of compounds of formula I where $R^1$ is a group of formula II, $R^7$ is $-OR^{19}$, $R^8$ is $-OCOR^{20}$, $R^9$ is $-OR^{21}$, $R^{10}$ is $R^{10}_a$, $R^{13}$ is $-OCOR^{25}$ and $R^{14}$ is $-0R^{26}$, i.e. compounds of formula

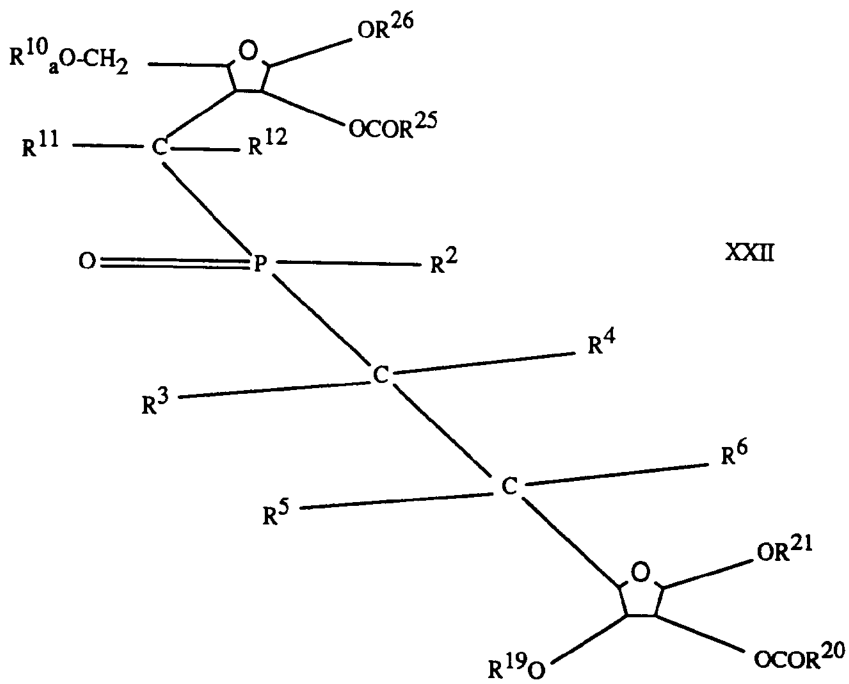

XXII

where $R^2$ to $R^6$, $R^{10}_a$, $R^{11}$, $R^{12}$, $R^{19}$, $R^{20}$, $R^{21}$, $R^{25}$ and $R^{26}$ are as hereinbefore defined. Glycosylation may be effected using the method hereinbefore described for the glycosylation of compounds of formula XII.

Compounds of formula XXII where $R^{20}$ and $R^{25}$ are the same can be prepared by esterification of the corresponding compounds of formula I where $R^8$ and $R^{13}$ are each hydroxyl, i.e. compounds of formula

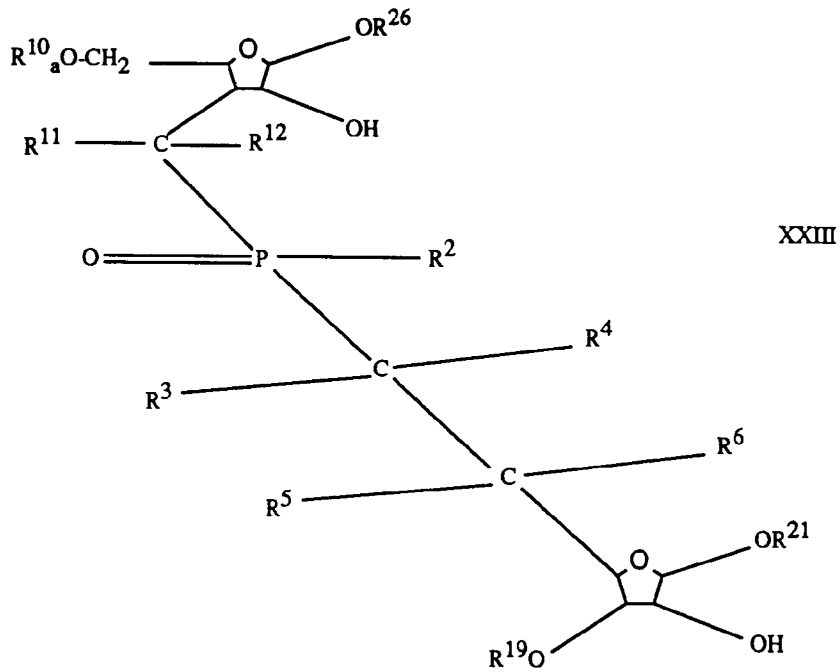

XXIII

where $R^2$ to $R^6$, $R^{10}_a$, $R^{11}$, $R^{12}$, $R^{19}$, $R^{21}$ and $R^{26}$ are as hereinbefore defined, with an acid of formula $R^{20}COOH$ or an anhydride or acid halide thereof, where $R^{20}$ is as hereinbefore defined. The esterification may be effected using the method hereinbefore described for the esterification of compounds of formula XIII.

Compounds of formula XXIII can be obtained by reaction of compounds of formula XIV with an alcohol of formula $R^{26}OH$, where $R^{26}$ is as hereinbefore defined, water and an acid, which may be an organic acid such as acetic, formic or p-toluenesulphonic acid or an inorganic acid such as hydrochloric or sulphuric acid, or an acidic ion exchange resin. The reaction is generally effected at temperatures from 30 to 150°C, preferably 70 to 90°C.

Compounds of formula I where $R^1$ is a group of formula II in which $R^{11}$ and $R^{12}$ are hydrogen can be prepared from corresponding compounds in which $R^{11}$ is hydroxy and $R^{12}$ is hydrogen, prepared as hereinbefore described, by conventional deoxygenation methods, for example by reaction with a suitably substituted reagent to allow free radical mediated cleavage, such as by reaction with a substituted or unsubstituted $C_6$-$C_{10}$ aryloxythiocarbonyl chloride such as p-tolylchlorothionoformate or pentafluorophenylchlorothionoformate to convert the hydroxy group $R^{11}$ into a substituted or unsubstituted $C_6$-$C_{10}$ aryloxythiocarbonyloxy group, and then removing this group by reaction with a trialkylstannane such as tri-n-butylstannane, in the presence of a free radical initiator such as azobis(isobutyronitrile) or the free radical initiators hereinbefore described. Such deoxygenation can be carried out using conventional procedures. Other standard methods for the deoxygenation of alcohols are described by Hartwig, Tetrahedron 39, 2609 (1983).

Similarly, compounds of formula I where $R^8$ and/or $R^{13}$ are hydroxy may be converted, by reaction with an aryloxythiocarbonyl chloride as described above, into compounds of formula I where $R^8$ and/or $R^{13}$ are aryloxythiocarbonyloxy, which can then be reacted with a trialkylstannane in the presence of a free radical initiator to give compounds of formula I where $R^8$ and/or $R^{13}$ are hydrogen.

Compounds of formula I which are dinucleotide analogues of formula VII where $R^7$ is substituted or unsubstituted alkyl-N,N-dialkylphosphoramidyl can be prepared by reacting compounds of formula VII where $R^7$ is hydrogen with a substituted or unsubstituted alkyl-N,N,N$^1$, N$^1$-tetralkylphosphoramidite such as 2-cyanoethyl-N,N,N$^1$,N$^1$-tetraisopropylphosphoramidite. The reaction is conveniently carried out under an inert atmosphere in a solvent such as methylene chloride in the presence of an ammonium tetrazolide such as diisopropylammonium tetrazolide. It can be effected at ambient temperature.

Compounds of formula I where $R^1$ is a group of formula II in which $R^{11}$ is halogen and $R^{12}$ is hydrogen can be prepared from those in which $R^{11}$ is hydroxy and $R^{12}$ is hydrogen by nucleophilic displacement reactions using conventional procedures.

Compounds of formula X where $R^{11}_a$ and $R^{12}$ are each hydrogen and $R^{13}_a$ and $R^{14}$ together denote a $C_1$ to $C_{15}$ hydrocarbylidenedioxy group can be obtained by a Wittig reaction of a ketone of formula

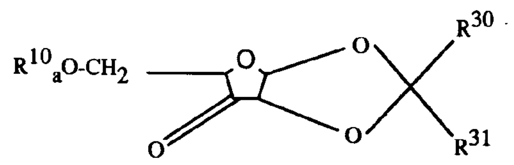

XXIV

where $R^{10}_a$, $R^{30}$ and $R^{31}$ are as hereinbefore defined, with a methyltriphenylphosphonium salt and an alkyllithium, usually at a temperature increasing from within the range -60 to -80°C to ambient temperature, the resulting reaction mixture being acidified, usually with acetic acid. The reaction may be carried out in an organic solvent, e.g. tetrahydrofuran, a hydrocarbon such as hexane or a mixture thereof.

Compounds of formula X where $R^{11}_a$ and $R^{12}$ are each halogen and $R^{13}_a$ and $R^{14}$ together denote a $C_1$-$C_{15}$ hydrocarbylidenedioxy group can be obtained by analogous reactions of compounds of formula XXIV. Reaction of such compounds with carbon tetrachloride and triphenylphosphine in acetonitrile using the procedure described by J.M.J. Tronchet et al, Eur. J. Med. Chem - Chim. Ther. 11(6), 489 (1976) gives compounds of formula X where $R^{11}_a$ and $R^{12}$ are each chlorine. Reaction of compounds of formula XXIV with dibromodifluoromethane, hexamethylphosphorus triamide and zinc in tetrahydrofuran gives compounds of formula X where $R^{11}_a$ and $R^{12}$ are each fluorine.

Compounds of formula XXIV may be prepared from $C_1$-$C_{15}$ hydrocarbylidenexyloses such as 1,2-isopropylidenexylose by reaction of the primary alcohol group with a compound of formula $R^{10}_aX$ or $(R^{22}CO)_2O$ where $R^{10}_a$ and $R^{22}$ are as hereinbefore defined and X is a halogen atom or a hydroxyl group, to convert the methylol group into a group $-CH_2OR^{10}_a$ or $-CH_2OCOR^{22}$, and reacting the product with an oxidising agent such as pyridinium dichromate, usually at 30-40°C in the presence of a dehydrating agent such as acetic anhydride and in an inert solvent such as methylene chloride to convert the $3^1$-hydroxyl group into a keto group. A suitable procedure for preparation of compounds of formula XXIV is described by H.S. Mosher et al, J. Org. Chem 51,2702 (1986).

The compounds of formula X where $R^{13}_a$ and $R^{14}$ together denote a $C_1$ to $C_{15}$ hydrocarbylidenedioxy group, prepared as described above, are of formula

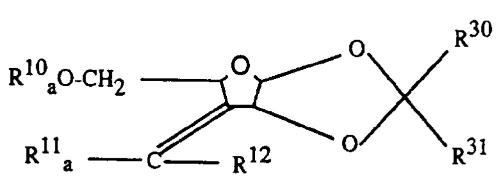

XXV

where $R^{10}_a$, $R^{11}_a$, $R^{12}$, $R^{30}$ and $R^{31}$ are as hereinbefore defined. These compounds can be treated with an acidic ion exchange resin to hydrolyse the hydrocarbylidenedioxy group, usually at 40-100°C, to give corresponding compounds of formula X where $R^{13}_a$ and $R^{14}$ are each hydroxy, which can in turn be esterified by reaction with an acid of formula $R^{25}$ COOH or an anhydride or acid halide thereof, for example by the method hereinbefore described for esterification of compounds of formula XIII, to give compounds of formula X where $R^{13}_a$ is $-OCOR^{25}$ and $R^{14}$ is $-OCOR^{26}$, $R^{25}$ and $R^{26}$ being the same. These compounds can be glycosylated with a base of formula $B^1H$, where $B^1$ is as hereinbefore defined, for example using the method hereinbefore described for glycosylation of compounds of formula XII, to give compounds of formula X where $R^{13}_a$ is $-OCOR^{25}$ and $R^{14}$ is $B^1$.

Compounds of formula X where $R^{13}_a$ is hydroxy and $R^{10}$ is hydrogen can be obtained by hydrolysis of a compound of formula X in which $R^{13}_a$ is a group $-OCOR^{25}$ and $R^{10}$ is $R^{10}_a$ using known procedures. Compounds of formula X where $R^{13}_a$ is halogen may be prepared by nucleophilic displacement reactions of compounds of formula X where $R^{13}_a$ is hydroxy using known procedures. Compounds of formula X where $R^{13}_a$ is $-OR^{25}$ or tri($C_1$-$C_{15}$ hydrocarbyl)silyloxy can be prepared by etherification of compounds of formula X where $R^{13}_a$ is hydroxy using known procedures.

Compounds of formula I where $R^1$ is hydrogen may be prepared by hydrolysis of compounds of formula I where $R^1$ is a protecting group Q to replace Q by a hydrogen atom. This hydrolysis may be carried out using known procedures. For example, where the protecting group Q is of formula III, it may be effected by reaction with a trialkylsilyl halide such as trimethylsilyl chloride, trimethylsilyl bromide or trimethylsilyl iodide. The reaction may be carried out at a temperature of -30°C to 100°C, preferably 0 to 40°C, in the presence of an alcohol such as ethanol, in an organic solvent, for example a halohydrocarbon such as chloroform or trichloroethane, an ether such as tetrahydrofuran or an aromatic hydrocarbon such as benzene, toluene or xylene, or a mixture

of two or more of such solvents.

Hydrolysis of compounds of formula I where $R^1$ is Q, to replace Q by a hydrogen atom, can also be effected by treatment with an acid under hydrolytic conditions. It may be carried out with a mineral acid such as hydrochloric acid.

Compounds of formula I where $R^1$ is $R^1_a$, i.e. $R^1_b$ or a protecting group Q, and $R^7$ is $R^7_a$ may be prepared by reaction of a compound of formula

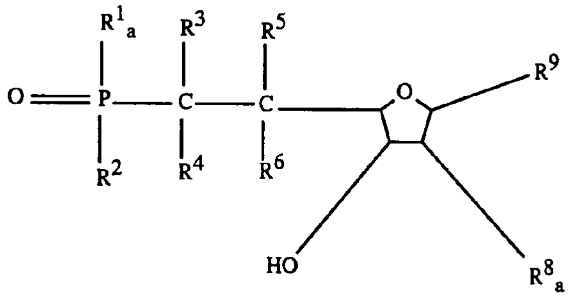

XXVI

where $R^1_a$, $R^2$ to $R^6$, $R^8_a$ and $R^9$ are as hereinbefore defined, with a compound of formula

$R^7_aX$ XXVII

or a carboxylic acid anhydride of formula

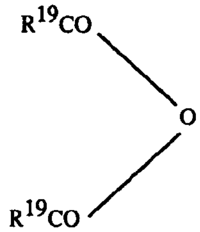

XXVIII

where $R^7_a$ and $R^{19}$ are as hereinbefore defined, and X is a halogen atom or a hydroxyl group. This reaction may be carried out using conventional esterification or etherification procedures. Thus, where $R^7$ is a group $-COR^{19}$, where $R^{19}$ is as hereinbefore defined, esterification may be carried out by reaction of the compound of formula XXVI with an acid of formula $R^{19}$ COOH, or the acid chloride or anhydride thereof, in an inert organic solvent such as an aromatic hydrocarbon, tetrahydrofuran or a mixture thereof, in the presence of an esterification catalyst. When, as in some preferred embodiments of the invention, it is desired to invert the stereochemical orientation of the hydroxy group in the compound of formula I, esterification may be effected using the procedure of Mitsunobu, Synthesis 1981, 1, in the presence of a triarylphosphine and an azodicarboxylate such as diethyl azodicarboxylate or diisopropyl azodicarboxylate.

The compounds of formula XXVII are alkyl halides, alkenyl halides, cycloalkyl halides, aryl halides, aralkyl halides, carboxylic acids, carboxylic acid halides, sulphonic acids, sulphonyl halides or trialkylsilyl halides which are either available commercially or may be prepared by known methods.

Compounds of formula XXVI where $R^5$ and $R^6$ are each hydrogen can be prepared by reaction of oxetane of formula

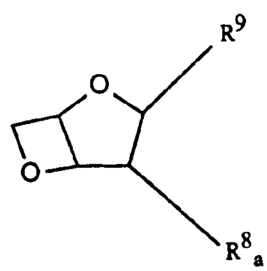

XXIX

where $R^8_a$ and $R^9$ are as hereinbefore defined, with an organometallic compound of formula

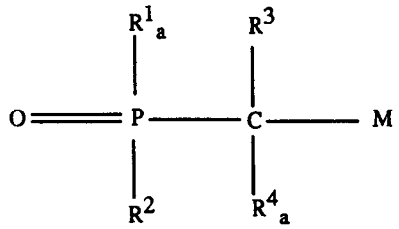

XXX

where $R^1_a$, $R^2$, $R^3$ and $R^4_a$ are as hereinbefore defined and M is lithium or magnesium, in the presence of a Lewis acid, preferably a boron trifluoride complex. The reaction is usually carried out at low temperature, generally -120°C to 40°C, preferably -80 to -60°C, in an organic solvent, e.g. an ether such as tetrahydrofuran, a hydrocarbon such as hexane or a mixture thereof, using 1 to 10 equivalents, preferably 4 to 7 equivalents, of the organometallic compound per equivalent of the oxetane. The organometallic compound of formula XXX is preferably formed in situ by reaction of an organolithium, preferably an alkyllithium, a hindered lithium amide such as lithium diisopropylamide or an organomagnesium halide, preferably an alkylmagnesium halide, with a compound of formula

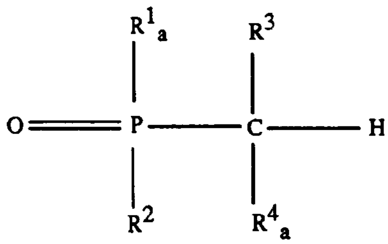

XXXI

where $R^1_a$, $R^2$, $R^3$ and $R^4_a$ are as hereinbefore defined. A suitable procedure for reaction of an organometallic compound with a nucleoside oxetane, such as a compound of formula XXIX where $R^9$ is a nucleoside base radical, is described in H. Tanaka et al, Tetrahedron Lett., 30,2567 (1989).

Compounds of formula XXIX may be prepared by reaction of a compound of formula

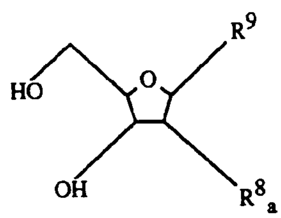

XXXII

where $R^8_a$ and $R^9$ as hereinbefore defined, with methane sulphonyl chloride in pyridine, followed by treatment with aqueous sodium hydroxide, for example using the procedure of J.P. Horwitz et al, J. Org. Chem., 31,205 (1966).

Compounds of formula XXXI can be obtained by reaction of a phosphine oxide of formula

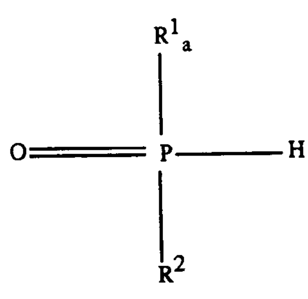

XXXIII

where Q and $R^2$ are as hereinbefore defined, with a compound of formula

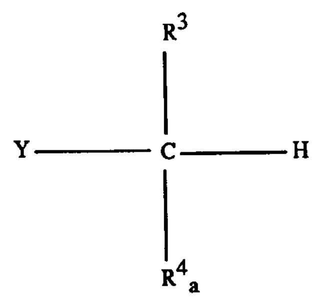

XXXIV

where $R^3$ and $R^4_a$ are as hereinbefore defined and Y denotes a leaving atom or group.

Phosphine oxides of formula XXXIII where $R^1_a$ is $R^1_b$ are either commercially available or may be prepared by conventional methods. Phosphine oxides of formula XXXIII where $R^1_a$ is a protecting group Q can be prepared by reacting a protected phosphinate ester of formula

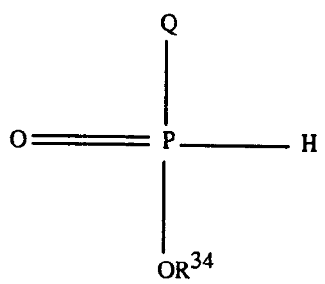

XXXV

where $R^{34}$ is $C_1$-$C_4$ alkyl and Q is as hereinbefore defined, with an organomagnesium halide of formula $R^2MgX$ or an organolithium of formula $R^2Li$, where $R^2$ is as hereinbefore defined, using the process described in EPO 501 702.

The leaving atom or group Y in formula XXXIV may be, for example, a halogen atom or a residue of an organic or inorganic acid after removal of an acidic hydrogen atom therefrom, such as an organic sulphonate group, e.g. a p-toluenesulphonate or trifluoromethanesulphonate group, or a sulphate anion. Preferably Y is a halogen atom or an arylsulphonate group, especially a chlorine, bromine or iodine atom or a p-toluenesulphonate group. Thus compounds of formula XXXIV are known or may be prepared by known methods.

The reaction between the phosphine oxide of formula XXXIII and the compound of formula XXXIV may be carried out under conventional conditions for substitution reactions at a P-H bond, for example using a base such as a tertiary amine, an alkali metal, usually sodium, an organometal of an alkali metal or magnesium, usually an alkyllithium, an alkali metal hydride, usually sodium hydride, or an alkali metal amide such as Li $N-[CH(CH_3)_2]_2$. The reaction may be carried out in an organic solvent, usually an ether such as diethyl ether or tetrahydrofuran, a hydrocarbon such as hexane or toluene or mixtures thereof, and at a temperature from -100°C to 100°C, usually from -80°C to 40°C.

Compounds of formula XXXII where $R^8_a$ is hydrogen or hydroxy and $R^9$ is hydroxy or $B^1$ are readily available saccharides or nucleosides. Compounds of formula XXXII where $R^8_a$ is other than hydrogen or hydroxy can be prepared by halogenation, etherification, esterification or silylation reactions of compounds where $R^8_a$ is hydroxy using known methods. Compounds of formula XXXII where $R^9$ is other than hydroxy or $B^1$ can be prepared by halogenation, etherification or esterification reactions of compounds of formula XXXII where $R^9$ is hydroxy using known procedures.

Compounds of formula I where $R^1$ is $R^1_a$, $R^8$ and $R^9$ together denote a $C_1$-$C_{15}$ hydrocarbylidenedioxy group and $R^3$, $R^4$, $R^5$ and $R^6$ are each hydrogen may be prepared by reacting an olefine of formula

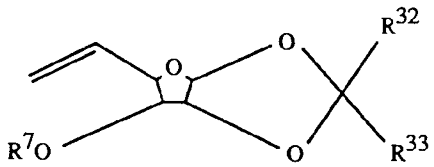

XXXVI

where $R^7$ is hydrogen or $R^7{}_a$, and $R^7{}_a$, $R^{32}$ and $R^{33}$ are as hereinbefore defined, with a phosphine oxide of formula XXXIII, in the presence of a free radical initiator. Suitable initiators include azo compounds such as azobis(isobutyronitrile), peroxides such as benzoyl peroxide, tert-butyl peroxide or 2,2-bis (tert-butylperoxy)propane, peresters such as tert-butyl perbenzoate or tert-butyl per-2-ethylhexanoate, percarbonates such as diacetyl perdicarbonate or bis(4-tert-butylcyclohexyl)perdicarbonate or persalts such as potassium persulphate. The initiator is generally used in an amount of 0.1 to 100 mol%, preferably 5 to 15, mol%, per mol of the olefinic compound of formula XXXVI.

The reaction between the olefine and the phosphine oxide may be carried out without a solvent, but is preferably carried out in an organic solvent, usually an aromatic hydrocarbon such as benzene, toluene or xylene. It may be carried out at temperatures of 30 to 100°C, preferably 70 to 90°C.

The olefine of formula XXXVI may be prepared from a compound of formula

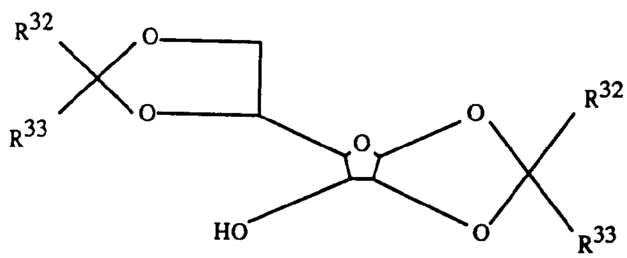

XXXVII

which itself can be prepared using the procedure described (for the preparation of compounds where $R^{32}$ and $R^{33}$ are each methyl) in Carbohyd. Res. 24 (1972) 194-5. The compound of formula XXXVII may be reacted with a compound of formula XXVII or XXVIII to etherify or esterify the hydroxyl group and the product reacted with 80% acetic acid at ambient temperature to give a compound of formula

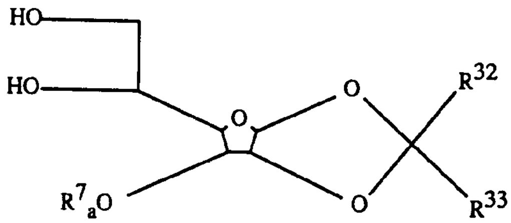

XXXVIII

where $R^7{}_a$, $R^{32}$ and $R^{33}$ are as hereinbefore defined, which is then reacted with methanesulphonyl chloride in the presence of a base to replace both the indicated hydroxyl groups by methane sulphonyloxy groups. The product is reacted with sodium iodide in methyl ethyl ketone at 70-90°C to give an olefine of formula XXXVI where $R^7$ is $R^7{}_a$, which can be hydrolysed by treatment with potassium carbonate in aqueous methanol at ambient temperature to give an olefine of formula XXXVI where $R^7$ is hydrogen.

The product of reaction between the olefine of formula XXXVI and the phosphine oxide of formula XXXIII is a compound of formula

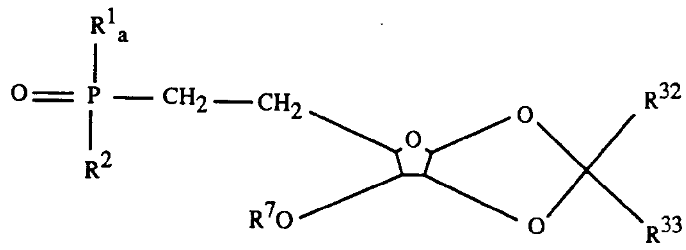

XXXIX

where $R^7$ is hydrogen or $R^7_a$ and $R^1_a$, $R^2$, $R^7_a$, $R^{32}$ and $R^{33}$ are as hereinbefore defined.

Compounds of formula XXVI, for instance compounds of formula XXXIX where $R^7$ is hydrogen, can be converted into compounds where $R^7$ is $R^{19}$ by reaction with a compound of formula $R^{19}X$ where $R^{19}$ is as hereinbefore defined and X is halogen, usually chlorine, bromine or iodine. This reaction can be effected using known etherification procedures, for example in the presence of a base such as sodium, sodium hydride or an alkyllithium at a temperature of -30 to 50°C, usually 5°C to ambient temperature.

Compounds of formula I where $R^1$ is $R^1_a$ and $R^8$ and $R^9$ are each hydroxy may be prepared by reaction of corresponding compounds of formula I where $R^8$ and $R^9$ together denote a hydrocarbylidenedioxy group, for instance compounds of formula XXXIX, with a hydrolysing agent for the hydrocarbylidenedioxy group, for example an acidic ion exchange resin. Where a compound of formula XXXIX is thus hydrolysed, the product is of formula

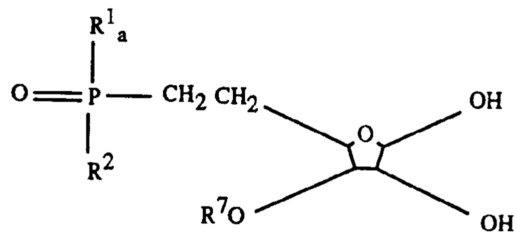

XXXX

where $R^7$ is hydrogen or $R^7_a$ and $R^1_a$, $R^2$ and $R^7_a$ are as hereinbefore defined. The hydrolysis may be effected in a mixture of water and a polar organic solvent such as dimethyl formamide or dichloroethane or an ether such as diethyl ether, tetrahydrofuran, dimethoxymethane or dimethoxyethane, usually using a water: solvent weight ratio of 15:1 to 25:1. Hydrolysis may be carried out at a temperature of 40 to 100°C, usually 60 to 80°C.

Compounds of formula I where $R^1$ is $R^1_a$, $R^8$ is $-OR^{20}$ and $R^9$ is $OR^{21}$, or $R^8$ is $-OCOR^{20}$ and $R^9$ is $-OCOR^{21}$, or $R^8$ is $-OSO^2R_{20}$ and $R^9$ is $-OSO_2R^{21}$, and $R^{20}$ and $R^{21}$ are the same, can be prepared by esterification or etherification of corresponding compounds of formula I where $R^8$ and $R^9$ are hydroxy using known esterifying agents or etherifying agents and known procedures. For example, compounds of formula I where $R^1$ is $R^1_a$, $R^7$ is $-COR^{19}$, $R^8$ is $-OCOR^{20}$ and $R^9$ is $-OCOR^{21}$, and $R^{19}$, $R^{20}$ and $R^{21}$ are the same, may be prepared by esterifying compounds of formula I where $R^1$ is $R^1_a$, $R^7$is hydrogen and $R^8$ and $R^9$ are each hydroxy, for instance compounds of formula XXXX where $R^7$ is hydrogen, with an acid of formula $R^{19}$ COOH or an acid halide or anhydride thereof. The esterification can be carried out using the method hereinbefore described for esterification of compounds of formula XIII.

In another example, compounds of formula I where $R^1$ is $R^1_a$, $R^7$ is $R^{19}$, $R^8$ is $-OCOR^{20}$ and $R^9$ is $-OCOR^{21}$, and $R^{20}$ and $R^{21}$ are the same, can be prepared by esterifying compounds of formula I where $R^1$ is $R^1_a$, $R^7$ is $R^{19}$ and $R^8$ and $R^9$ are each hydroxy, for instance compounds of formula XXXX where $R^7$ is $R^{19}$, with an acid of formula $R^{20}$ COOH or an acid halide or anhydride thereof, for example using the method hereinbefore described for esterification of compounds of formula XIII.

Compounds of formula I where $R^1$ is $R^1_a$, $R^8$ is hydroxy and $R^9$ is $-OR^{21}$ where $R^{21}$ is $C_1$ to $C_4$ alkyl may be prepared by reaction of compounds of formula I where $R^1$ is $R^1_a$, and $R^8$ and $R^9$ together denote a $C_1$-$C_{15}$ hydrocarbylidenedioxy group, for instance compounds of formula XXXIX, with an alcohol of formula $R^{21}OH$, e.g. a $C_1$ to $C_4$ alcohol, water and an acid, e.g. an organic acid such as acetic, formic or p-toluenesulphonic acid or an inorganic acid such as hydrochloric or sulphuric acid. The acid may be used in an amount of 0.001 to 1 mol per mol of the compound containing the hydrocarbylidenedioxy group. The reaction may be carried out at 30 to 150°C, usually at 70 to 90°C. The product may be esterified with an acid of formula $R^{20}COOH$ or an acid halide or anhydride thereof to give a compound where $R^8$ is $-OCOR^{20}$.

Compounds of formula I where $R^1$ is $R^1_a$, $R^7$ is $R^7_a$ and $R^9$ is a monovalent nucleoside base radical $B^1$ may

be prepared by glycosylation of a compound of formula I where $R^1$ is $R^1_a$, $R^7$ is $R^7_a$, $R^8$ is $-OCOR^{20}$ and $R^9$ is $-OR^{21}$ or $-OCOR^{21}$ with a base of formula $B^1H$ where $B^1$ is as hereinbefore defined. The glycosylation can be effected using the method hereinbefore described for the glycosylation of compounds of formula XII.

Compounds of formula I where $R^1$ is $R^1_a$, $R^5$ is hydrogen and $R^6$ is $R^{18}$ as hereinbefore defined, may be prepared by deoxygenation of a compound of formula

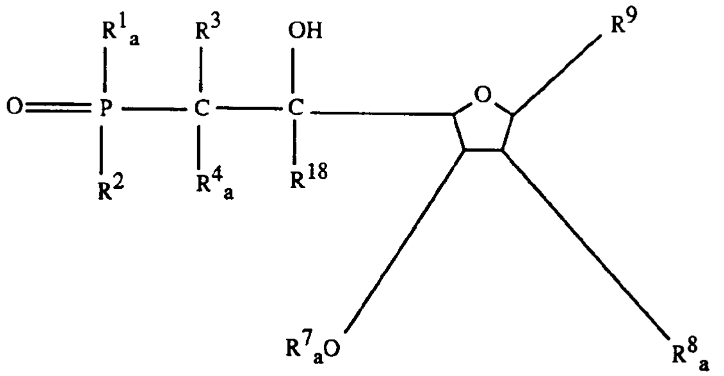

XXXXI

where $R^1_a$, $R^2$, $R^3$, $R^4_a$, $R^7_a$, $R^8_a$, $R^9$ and $R^{18}$ are as hereinbefore defined. The deoxygenation may be effected using deoxygenation procedures hereinbefore described.

Compounds of formula XXXXI can be obtained by reaction of a compound of formula XXX with a compound of formula

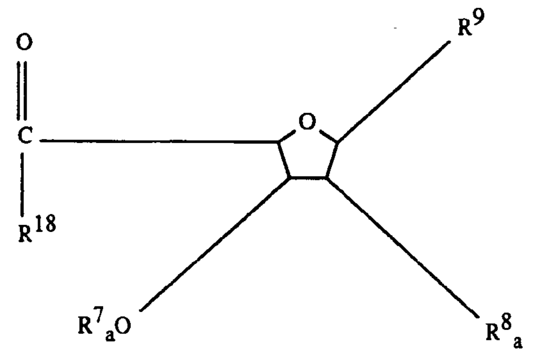

XXXXII

where $R^7_a$, $R^8_a$, $R^9$ and $R^{18}$ are as hereinbefore defined, optionally in the presence of a Lewis acid such as a boron trifluoride complex, for example under the conditions hereinbefore described for the reaction of compounds of formulae XXX and XXXI.

Compounds of formula XXXXII may be prepared by reaction of an aldehyde of formula

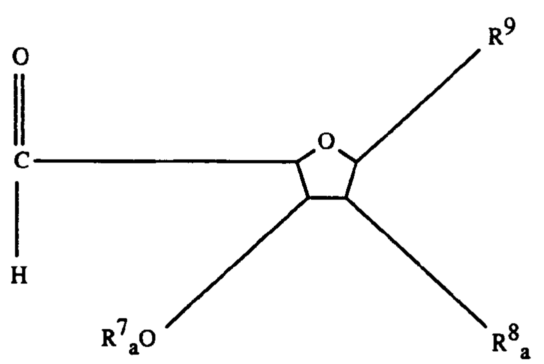

XXXXIII

where $R^7_a$, $R^8_a$ and $R^9$ are as hereinbefore defined, with an organometallic compound of formula $R^{18}Li$ or $R^{18}Mg$ Y where $R^{18}$ is as hereinbefore defined and Y is halogen, usually chlorine or bromine, generally in an organic

solvent, for example an ether such as tetrahydrofuran or diethyl ether, and at a temperature of -120 to 0°C, usually -100 to -60°C, followed by oxidation, for example a Swern oxidation, of the resulting alcohol.

Aldehydes of formula XXXXIII can be obtained by oxidation of the corresponding 5'-hydroxymethyl compounds using known methods, for example by treatment with a haloacetic acid, dimethyl sulphoxide and dicyclohexylcarbodiimide using the procedure of Jones and Moffat, J. Amer. Chem. Soc. 90,5337 (1968) or Ranganatham et al, J. Org. Chem. 39,290 (1974). The 5'-hydroxymethyl compounds are readily available saccharides or, where $R^9$ is $B^1$, nucleosides, or substituted derivatives thereof.

Compounds of formula I where $R^1$ is $R^1_a$, and $R^4$ and $R^6$ together denote a valence bond, i.e. compounds where the carbon atoms linking the phosphorus atom to the furanose ring in formula I are linked by an ethylenic double bond, can be prepared by esterifying compounds of formula XXXXI where $R^4_a$ is hydrogen to convert the indicated hydroxyl group into an aliphatic or aromatic sulphonyl ester group, for example by reaction with methanesulphonyl chloride or p-toluenesulphonyl chloride in the presence of a tertiary base such as triethylamine, and heating the resulting sulphonyl ester in the presence of a strong base, for example an alkali metal alkoxide such as sodium methoxide or a non-nucleophilic base such as 1,8-diazabicyclo [5.4.0] undec-7-ene or 1,5-diazabicyclo [4.3.0] non-5-ene, to effect dehydration to the desired unsaturated compound.

Compounds of formula I where $R^1$ is $R^1_a$, $R^5$ is $-OR^{17}$ and $R^6$ is $R^{18}$ may be prepared by etherification of the hydroxyl group in compounds of formula XXXXI by reaction with a halide of formula $R^{17}Y$ where $R^{17}$ is as hereinbefore defined and Y is halogen, usually bromine or iodine. The reaction is generally carried out in the presence of a base e.g. sodium hydride or a hindered amine such as 1,8-diazobicyclo [5.4.0] undec-7-ene in an organic solvent, usually a hydrocarbon such as benzene or toluene.

A similar etherification procedure may be used to convert compounds of formula

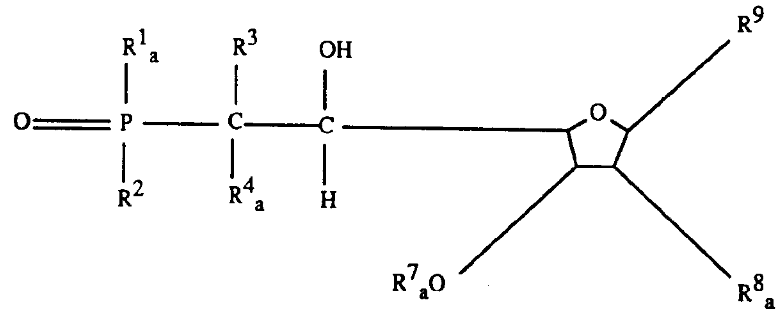

XXXXIV

where $R^1_a$, $R^2$, $R^3$, $R^4_a$, $R^7_a$, $R^8_a$ and $R^9$ are as hereinbefore defined, into compounds of formula I where $R^5$ is $-OR^{17}$ and $R^6$ is hydrogen.

Compounds of formula I where $R^1$ is $R^1_a$, $R^5$ is halogen and $R^6$ is hydrogen or $R^{18}$ can be obtained by a nucleophilic displacement reaction of a compound of formula XXXXIV or XXXXI respectively, for example with diethylamino sulphur trifluoride in an organic solvent such as methylene chloride at a temperature of -100 to -30°C, optionally in the presence of pyridine.

Compounds of formula I where $R^1$ is $R^1_a$, $R^5$ is $-OCOR^{17}$ and $R^6$ is hydrogen or $R^{18}$ may be prepared by reacting a compound of formula XXXXIV or XXXXI respectively with an acid of formula $R^{17}COOH$ or an acid halide or anhydride thereof, where $R^{17}$ is as hereinbefore defined, to esterify the hydroxyl group. The reaction may be carried out using conventional esterification procedures; for example, where an acid chloride or anhydride is used, the esterification may be effected in the presence of an organic base such as pyridine in an organic solvent such as methylene chloride, toluene or ethyl acetate at a temperature from -70 to 30°C, preferably -5 to 20°C.

Compounds of formula XXXXIV can be prepared by reacting an aldehyde of formula XXXXIII with a compound of formula XXX. The reaction is generally carried out at a temperature of -100 to 0°C, preferably -70 to -80°C, in an organic solvent such as tetrahydrofuran, diethyl ether, tert-butyl methyl ether or toluene, optionally in the presence of a Lewis acid such as a boron trifluoride complex.

Compounds of formula XII, formula XIII, formula XIV, formula XVI, formula XVII, formula XVIIA, formula XVIII, formula XIX, formula XX, formula XXI, formula XXII, formula XXIII, formula XXVI, formula XXXIX, formula XXXX, formula XXXXI and formula XXXXIV, as hereinbefore defined, form another group of preferred compounds of the invention.

When mixtures of diastereomers of compounds of formula I or intermediates therefor are obtained, these can be separated by known methods, for example by fractional distillation, crystallisation or chromatography.

The present invention also provides oligonucleotides containing at least one unit derived from a dinucleotide analogue of formula I where $R^1$ is a group of formula II, $R^9$ is $B^1$ and $R^{14}$ is $B^2$, i.e. a compound of formula

VII, and the use of dinucleotide analogues of formula VII in the synthesis of such oligonucleotides. Such oligonucleotides may contain only units derived from the same or different dinucleotide analogues of formula VII, preferably of formula VIII, or may contain at least one unit derived from a dinucleotide of formula VII and at least one other unit derived from another natural or synthetic nucleoside. Generally, the oligonucleotides contain 2 to 200 nucleoside - derived units. Preferred oligonucleotides contain 2 to 100, more preferably 2 to 50, especially 2 to 30 such units. Preferably, the oligonucleotides contain units of the same or different dinucleotide analogues of formula VII together with units of natural or synthetic nucleoside derived from D-ribose or 2-deoxyribose.

The present invention further provides oligonucleotides of formula

$$5'\text{-U-(O-L-O-V)}_n\text{O-L-O-W-3'} \qquad \text{XXXXV}$$

where U, V and W are the same or different residues of natural or synthetic nucleosides and at least one of the residues U, V and W being derived from a dinucleotide analogue of formula VII and having the formula

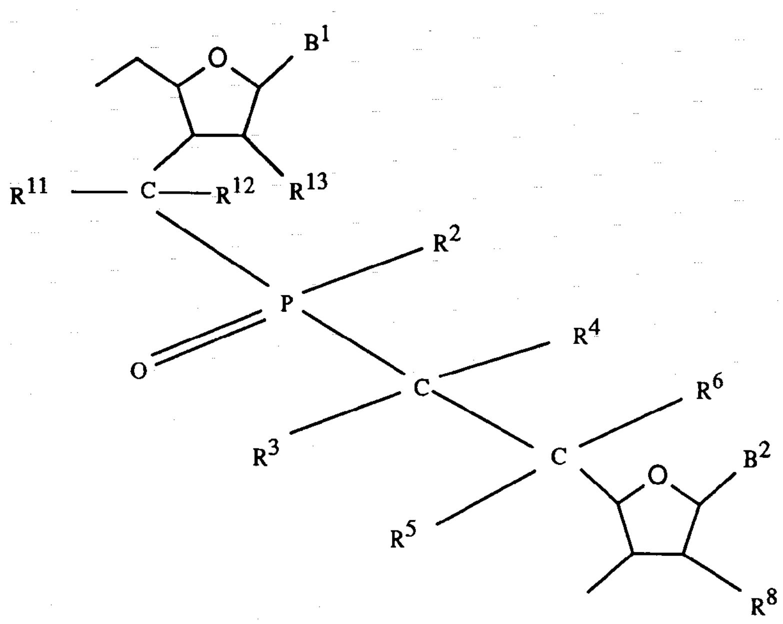

XXXXVI

L is a nucleoside-bridging group and n is a number from 0 to 200, preferably 0 to 100, more preferably 0 to 50, especially 0 to 30. A preferred bridging group L is the natural oligonucleoside-bridging group -P(O)O$^{\ominus}$. Examples of further bridging groups are -P(O)S$^{\ominus}$-, -P(S)S$^{\ominus}$-, -P(O)$R^{35}$-, -P(O)N$R^{36}R^{37}$- or -$CH_2$- where $R^{35}$ is hydrogen or $C_1$-$C_6$ alkyl and $R^{36}$ and $R^{37}$ are independently hydrogen or $C_1$-$C_6$ alkyl.

The residues derived from one or more dinucleotides of formula VII can be bonded terminally or in the nucleotide sequence whereby several, for example 2 to 5 residues derived from one or more dinucleotide analogues of formula VII can be bonded between residues of natural or synthetic nucleosides, or there can be mixed forms of this distribution lying in the nucleotide sequence. Preferably there are 4 to 30 nucleoside units, of which preferably 1 to 12, especially 1 to 6 and particularly 1 to 4 units are residues derived from dinucleotide analogues of formula VII.

Particularly preferred embodiments are oligonucleotides of formula XXXXV where n is 2 to 50, preferably 2 to 30, L is a group -P(O)O$^{\ominus}$, U, V and W are the same or different residues of a natural nucleoside, and at least one of the residues U, V and W is of formula XXXXVI and $B^1$ and $B^2$ are natural nucleoside base radicals. The natural nucleoside may be, for example, adenosine, cytosine, guanosine, uridine, 2-aminoadenosine, 5-methylcytosine, 2'-deoxyadenosine, 2'-deoxycytosine, 2'-deoxyguanosine or thymidine. The residue of formula XXXXV can be bound terminally or in the nucleotide sequence, whereby several, for example 2 to 5 of the same or different residues of formula XXXXV can follow one after another, or the same or different residues of formula XXXXV can be bound between residues of natural nucleosides, or mixed types of these distributions can lie in the nucleotide sequence.

In another especially preferred embodiment of oligonucleotides of formula XXXXV, U, V and W are the same or different residues of formula XXXXVI in which $B^1$ and $B^2$ are natural nucleoside base radicals. In this embodiment, n is preferably 2 to 20, especially 1 to 12, more especially 1 to 6 and most especially 1 to 4.

The preparation of oligonucleotides according to the invention can be carried out using known procedures,

if necessary automatically using commercial nucleic acid synthesizing machines. In the case of the bridging group $P(O)O^{\ominus}$, for example, the phosphotriester process, the phosphite triester process or the H-phosphonate process can be used, all of which are familiar to those skilled in the art. Many suitable procedures are described in Oligonucleotides and Analogues: A Practical Approach, edited by F. Eckstein, Oxford University Press, 1991.

In a typical procedure, a dinucleotide analogue of formula VII where $R^7$ and $R^{10}$ are each hydrogen, i.e. where the 3' and 5' hydroxyls are free, is reacted with $4,4^1$-dimethoxytriphenylmethyl chloride in the presence of a base, for example using the procedure described in Example 3 of WO 92/20823, to give a dinucleotide analogue of formula VII where $R^{10}$ is a dimethoxytrityl group, which is then reacted with 2-cyanoethyl-$N,N,N^1,N^1$-tetraisopropyl phosphordiamidite to replace the 31 hydroxyl by a 2-cyanoethyl-N,N-diisopropyl-phosphoramidyloxy group, thereby activating the dinucleotide analogue for coupling at the $3^1$ position. The functionalised dinucleotide analogues obtained can be inserted into any desired sequence using, for example a CPG-solid support and standard nucleic acid synthesizing machine such as Biosystems 380B and 394 and Milligen/Biosearch 7500 and 8800s.

The dinucleotide analogues of the invention and oligonucleotides incorporating units derived therefrom can be used in therapeutics, for example in the treatment of a human or other animal suffering from a disease which is modulated by a protein, or in the treatment of viruses such as influenza, herpes and HIV. Other compounds of the invention, particularly those where $R^9$ is a monovalent nucleoside base radical $B^2$, can be used in the treatment of viruses. Accordingly, the present invention also provides a pharmaceutical composition comprising as active ingredient a compound of formula I, particularly a dinucleotide analogue of the invention, or an oligonucleotide incorporating at least one unit derived therefrom. Optimum dosages and treatment schedules can readily be determined by those skilled in the art. When administered to mammals of about 70 kg weight, the dose can be, for example, 0.01 to 1000 mg per day. It will generally be preferred to adminster therapeutic agents in accordance with the invention internally, for example orally, by inhalation, intravenously or intramuscularly. Other methods of administration, such as transdermal, topical or intra-lesional methods, and by inclusion in suppositries, can also be useful. Use in conjuction with pharmacologically acceptable carriers is preferred for some therapeutic treatments.

The oligonucleotides according to the invention have a surprisingly high stability to degradation by nucleases. A very good pairing with complementary nucleic acid strands, particularly of the RNA type, is also observed. The oligonucleotides according to the invention are therefore particularly suitable for antisense technology, i.e. for inhibition of the expression of undesired protein products due to the binding to suitable complementary nucleotide sequences in nucleic acids (see EP 0 266 099, WO 87/07300 and WO 89/08146). They can be employed for the treatment of infections and diseases, for example by blocking the expression of bioactive proteins at the nucleic acid stage (for example oncogenes). The oligonucleotides according to the invention are also suitable as diagnostics and can be used as gene probes for the detection of viral infections or of genetically related diseases by selective interaction at the single or double-stranded nucleic acid stage. In particular - due to the increased stability to nucleases - diagnostic use is not only possible *in vitro* but also *in vivo* (for example tissue samples, blood plasma and blood serum). Use possibilities of this type are described, for example, in WO 91/06556.

The pharmacologically active mononucleotides, dinucleotides and oligonucleotides according to the invention can be used in the form of parentally administrable preparations or of infusion solutions. Solutions of this type are preferably isotonic aqueous solutions or suspensions, it being possible to prepare these before use, for example in the case of lyophilised preparations which contain the active substance on its own or together with a carrier, for example mannitol. The pharmaceutical preparations can be sterilised and/or contain excipients, for example preservatives, stabilisers, wetting and/or emulsifying agents, solubilisers, salts for regulating the osmotic pressure and/or buffers. The pharmaceutical preparations, which if desired can contain further pharmacologically active substances such as, for example, antibiotics, are prepared in a manner known per se, for example by means of conventional dissolving or lyophilising processes, and contain about 0.1% to 90%, in particular from about 0.5% to about 30%, for example 1% to 5% of active substance(s).

This invention is illustrated by the following Examples.

Compound X used in the Examples is prepared as follows:

A mixture of 1,2,5,6-di-O-isopropylidene-α-D-allofuranose (a diacetonide of formula XXXVII) prepared according to Carbohyd. Res. 24(1972) 192 (55.72g., 0.214M), acetic anhydride (100ml), and pyridine (50ml) is stirred at room temperature for 3 hours. The solution is evaporated then co-evaporated with methanol three times to remove the excess of acetic anhydride and pyridine. The residue is dissolved in dichloromethane (200ml), washed several times with water and dried ($MgSO_4$). Evaporation gives Compound A as a white solid, m.p.t.75°C, $[\alpha]_D^{25}$ + 109.4° c, 1.03 $CHCl_3$.

Found C 55.5, H 7.30; Calculated for $C_{14}H_{22}O_7$ C 55.6, H, 7.3%

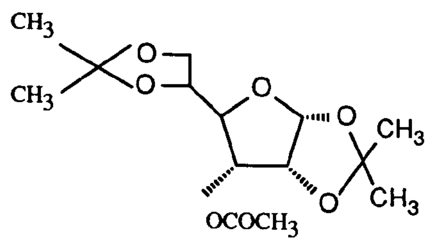

Compound A

Compound A (65g 0.215M) is dissolved in 80% acetic acid (250ml) and allowed to stand for 60 hours. The acetic acid is removed by evaporation and co-evaporation with methanol to give a pale yellow oil. This oil is chromatographed on silica (750g) using ethyl acetate eluant to give Compound B as a colourless oil. $[\alpha]_d^{20}$ + 125.2°c, 1.15 $CHCl_3$
Found C 50.1, H7.0; Calculated for $C_{11}H_{18}O_7$ C50.4, H6.9%

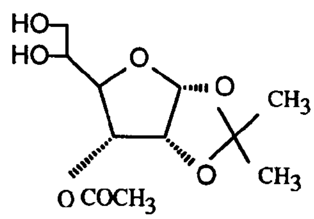

Compound B

Compound B (45.9g, 0.175M) in pyridine (180ml) under argon is cooled to 5°C and methanesulphonyl chloride (60.1g, 0.525M) is added over 1 hour. The temperature is allowed to rise to room temperature and the mixture is stirred for a further 2.5 hours until the reaction, monitored by thin layer chromatography (TLC), appears complete. Chloroform (200ml) and hydrochloric acid (2N, 300ml), are added and the mixture is stirred for 5 minutes. The organic phase is separated, washed with sodium bicarbonate (250ml) then brine (250ml) and dried ($MgSO_4$). Evaporation of solvent gives a yellow oil (70g) which is purified by flash chromatography using ether eluant to give Compound C, m.pt. 84-5°C, $[\alpha]_D^{25}$ + 109.6°c 0.75;
Found C 37.3, H5.3; Calculated for $C_{13}H_{22}O_{11}S_2$, C37.3, H5.3%.

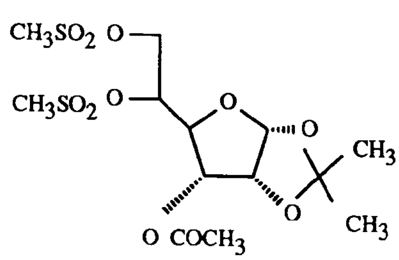

Compound C

A mixture of Compound C (65g, 0.155M) and sodium iodide (94g, 0.62M) in butan-2-one (1.51) is heated to reflux for 5 hours. A further 10% of sodium iodide is added and heating continued for 3 hours. The solvent

is removed and the dark brown residue is partitioned between chloroform and water. Sodium thiosulphate is added portionwise until the solution is colourless. The chloroform extracts are separated, washed with sodium bicarbonate then brine and dried ($MgSO_4$). Evaporation gives a pale yellow oil (35g, 100%) which is purified by chromatography on silica using hexane: ethyl acetate, 2:1, eluant, to give Compound D. An analytical sample is obtained by bulb to bulb distillation (100°C/0.1mm Hg), $[\alpha]_D^{25}$ + 107.5 c, 1.13 $CHCl_3$;
Found C58.0, H 7.0; $C_{11}H_{16}O_5$ requires C57.9, H 7.1%.

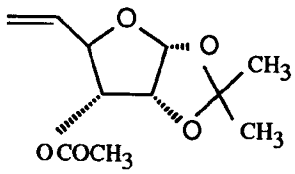

Compound D

Compound D (15g, 0.066M) dissolved in methanol (100ml) is added to a solution of potassium carbonate (22.7g, 0.165M) in water (100ml). After 15 minutes the reaction is complete (TLC). The solvent volume is reduced to 50ml and after further co-evaporation with water the residue is washed with chloroform (3 x 100ml). The chloroform extracts are washed with brine then dried ($MgSO_4$). Evaporation gives Compound X as a white solid, m.pt. 67.5-68°C, $[\alpha]_D^{25}$ + 38.2°c, 0.95 $CHCl_3$. Found C 57.8, H 8.0; Calculated for $C_9H_{14}O_4$,C 58.05, H 7.6%.

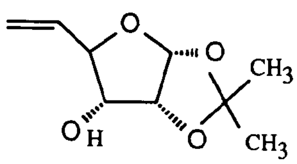

Compound X

Compound Y used in the Examples, which is a compound of formula

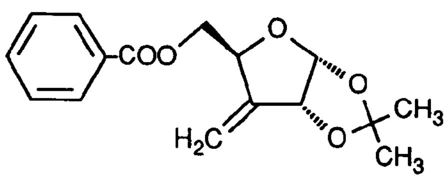

Compound Y

is prepared as follows:

To a suspension of methyltriphenylphosphonium bromide (82.5g, 0.231mol) in dry tetrahydrofuran (500ml) at -70°C under argon is added n-butyl lithium (1.6m in hexane, 144ml, 0.231mol) over about 10 minutes. The mixture is allowed to warm to 15°C during which time an orange solution forms. This mixture is re-cooled to -70°C, more n-butyl lithium (6ml) is added, and after a further 15 minutes, a solution of 5-O-benzoyl-1,2-di-O-acetonide-3-ketoxylose prepared as described by H.S. Mosher. J. Org. Chem. 1986, 51, 2702 (50.0g, 0.171mol) in dry tetrahydrofuran (100ml) is added in 5 batches over 35 minutes, whilst maintaining the reaction temperature at -66 to -70°C. The mixture is kept at -70°C for 2 hours, then allowed to warm to +10°C over 1.5 hours and maintained at this temperature for 3 hours. Acetic acid (3.5ml) is added, followed by hexane (500ml). The resultant precipitate is separated by filtration and the liquors passed through a pad of silica gel (ca 2cm

deep). This pad is washed with ether (1 litre) and the filtrate is evaporated to yield a crude oil. The product is purified by flash column chromatography over silica gel (Merck, Art 15111, 10cm ∅, 5cm L) eluting with hexane then hexane: ether (3:1) mixtures to yield Compound Y as a colourless solid, mp 61-62°C.

δH ($CDCl_3$) 8.10 (2H, d, Ph-H), 7.55 (1H, t, Ph-H), 7.41 (2H, t, Ph-H), 5.95 (1H, d, H-1), 5.50) (1H, s, C=CH), 5.35 (1H, s, C=CH), 5.10 (1H, m, H=4), 4.96 (1H, d, H-2), 4.55 (1H, dd, H-5), 4.40 (1H, dd, H-5), 1.55 (3H, s, $CH_3$), 1.40 (3H, s, $CH_3$) ppm.

δC ($CDCl_3$) 166.2 (COPh), 145.6, 133.1, 129.6, 128.3, 112.7, 112.6, 104.5, 81.6, 77.3, 65.6, 27.3, 27.0 ppm.

Found: C 66.5, H 6.4%; $C_{16}H_{18}O_5$ requires C 66.25, H 6.25%.

Compound Z used in the Examples, which is a compound of formula

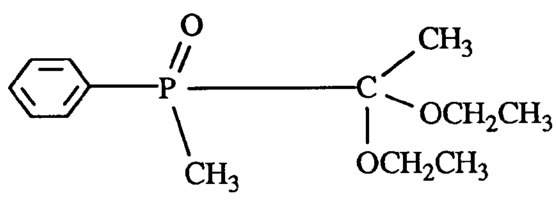

is prepared as follows:

To a stirred solution of methylphenylphosphine oxide (6.8g, 49 mmol) in dichloromethane (10 ml) at -5°C, triethyl orthoacetate (18 ml, 97 mmol) is added, followed dropwise by boron trifluoride etherate (0.30 ml, 2.4 mmol). The reaction mixture is allowed to warm to 20°C and to stand for 6 hours. All volatile material is evaporated and the crude mixture is rapidly purified by dry flash chromatography on silica gel. The product, Compound Z, is obtained as an oil, b.p. 160-170°C (0.15 mm Hg).

$^{31}P$ nmr (162 MHz, $CDCl_3$) δ 39.3 ppm

M/Z C.I. ($NH_3$) 257 ($MH^+$)

The aldehyde of formula IX used in the Examples is prepared as described in WO 92/20823.

Dimethyl (1,1-diethoxyethyl)phosphine oxide used in the Examples is prepared as follows:

To a solution of methyl(1,1-diethoxyethyl)phosphine oxide, prepared as described in EP 0 501 702 (89.3g, 0.5mol) in THF (900ml) at -78°C under an atmosphere of argon is added a solution of potassium bis(trimethylsilyl)amide (688ml, 0.521 mol) in toluene over 15 minutes. After stirring for 1 hour at -78°C, methyl iodide (34ml, 0.55mol) is added over 5 minutes and stirring at -78°C continued for 1 hour. The reaction is then quenched by the addition of a 1% solution of $NaH_2PO_4$ (450ml) and slow warming to room temperature. Concentration gives an aqueous suspension to which dichloromethane (600ml) is added. The organic phase is separated and washed with 1% $NaH_2PO_4$ (500ml), followed by water (500ml). Drying over $MgSO_4$, concentration and purification by flash vacuum silica column chromatography (chloroform-ethanol 40:1) gives a clear oil which is distilled to give dimethyl (1,1-diethoxyethyl)phosphine oxide, b.p. 100°C at 0.05mmHg.

Found C 49.1, H 10.2, P 16.1%; $C_8H_{19}O_3P$ requires C 49.5, H 9.85, P 15.95%.

$^{31}$ Pnmr $^1H$ decoupled ($CDCl_3$, 36 MHz) δ 48.3 ppm.

Example 1

This Example describes the preparation of the compound of formula

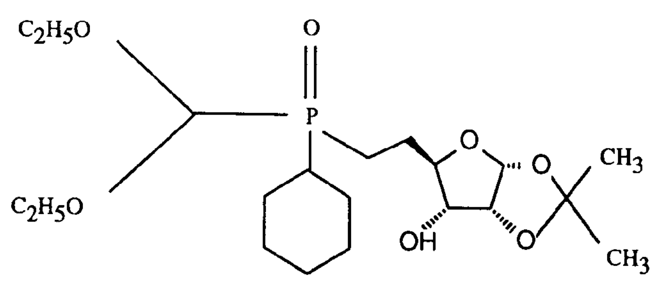

Compound 1

Compound X (0.3g, 0.0016M) is added to a solution of cyclohexyldiethoxymethylphosphine oxide (0.4g, 0.0017M) and tert-butylcyclohexyl perdicarbonate (0.2g, 0.0006M) in toluene (1ml) at 80°C. The mixture is stirred for 6 hours until reaction is complete (monitored by $^{31}P$ nmr), cooled and purified by chromatography on silica using 5% methanol in chloroform as eluant. There is obtained Compound 1 (0.4g, 58%).

$^{31}P$ nmr ($CDCl_3$, 162 MHz) δ = 49 ppm.

Example 2

This Example describes the preparation of the compound of formula

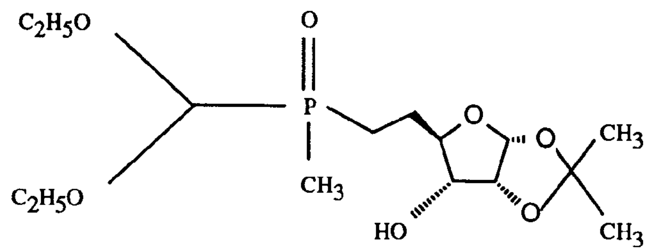

Compound 2

To a solution of diethoxymethyl methyl phosphine oxide (4g, 0.024M) and t-butylcyclohexylperdicarbonate (0.81g, 0.0022M) at 0°C (ice bath) is added dropwise a solution of Compound X (5.35g, 0.029M) in toluene (4ml). The cooling bath is removed and the solution is heated to 80°C and stirred under argon for 4 hours. When the reaction is complete (monitored by $^{31}P$ nmr and thin layer chromatography (TLC), dichloromethane (50ml) and water (50ml) are added. The organic layer is separated, washed with a further quantity of water (50ml) and dried ($MgSO_4$). Evaporation gives a viscous oil which is chromatographed on silica using 6% methanol in dichloromethane as eluant. There is obtained Compound 2 as a viscous oil.

$^{31}P$ nmr ($CDCl_3$, 162MHz) : δ 47.85 ppm.

Example 3

This Example describes the preparation of the compound of formula

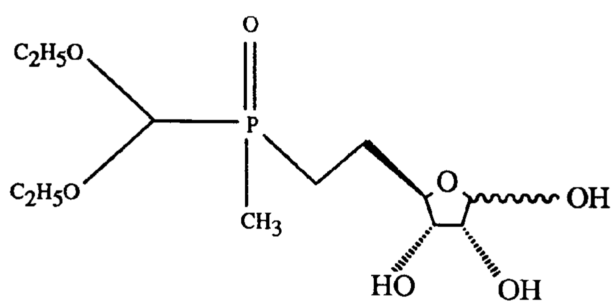

Compound 3

To Compound 2 (2.02g, 0.0057M) in dimethoxyethane (20ml) is added a slurry (30ml) of Dowex 50w x 2 (100) ion exchange resin, $H^+$ form, in water. The mixture is heated to 70°C for 5 hours and then cooled. The Dowex is removed by filtration and evaporation gives a yellow oil which is chromatographed on silica using 14% methanol in dichloromethane as eluant. There is obtained Compound 3 as a viscous oil.

Found: C 46.0; H 7.8; $C_{12}H_{25}O_7P$ requires C 46.15; H 8.07%

$^{31}P$ nmr ($CD_3OD$), 162 MHz) : δ 54.87, 55.13 ppm.

Example 4

This Example describes the preparation of the compound of formula

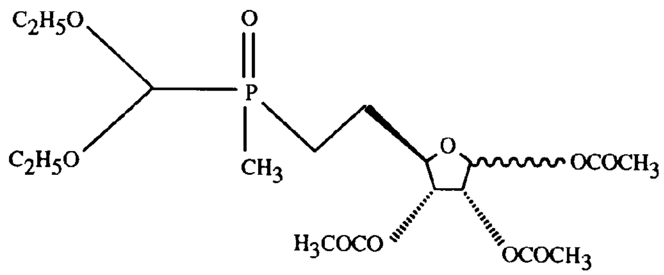

Compound 4

To a solution of Compound 3 (2.16g, 0.0069M) in pyridine (8ml) at room temperature is added dropwise a solution of acetic anhydride (4ml). The reaction mixture is stirred at room temperature for 18 hours. Excess acetic anhydride and pyridine are removed by evaporation. The residual oil is dissolved in dichloromethane, washed with dilute hydrochloric acid and water and dried over $MgSO_4$. Evaporation gives a yellow oil which is chromatographed on silica using 7% methanol in ethyl acetate as eluant to give Compound 4.

$^{31}$P nmr ($CDCl_3$, 162 MHz) : δ 47.33 ppm.

Example 5

This Example describes the preparation of the compound of formula

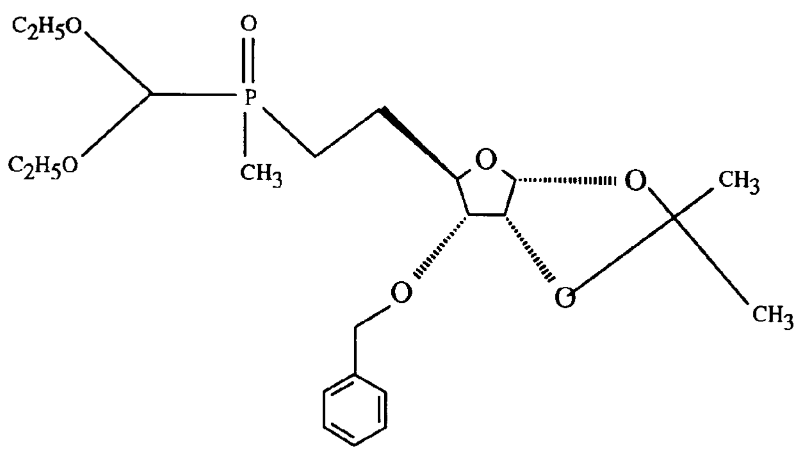

Compound 5

To a suspension of sodium hydride (0.34g, 0.014M) in tetrahydrofuran (40ml) maintained at 5°C under argon is added dropwise a solution of Compound 2 (3g, 0.0085M) in tetrahydrofuran (40ml). The cooling bath is removed and the solution is stirred for 60 minutes at room temperature. The solution is then recooled to 5°C and benzyl bromide (1.6g, 0.0094M) is added. The cooling bath is removed and the solution stirred at room temperature for 3 hours. The inorganic salts are removed by filtration and evaporation gives an oil which is chromatographed on silica using 6% methanol in dichloromethane as eluant to give Compound 5.

$^{31}$P nmr ($CDCl_3$, 162MHz): δ 46.16 ppm.

Example 6

This Example describes the preparation of the compound of formula

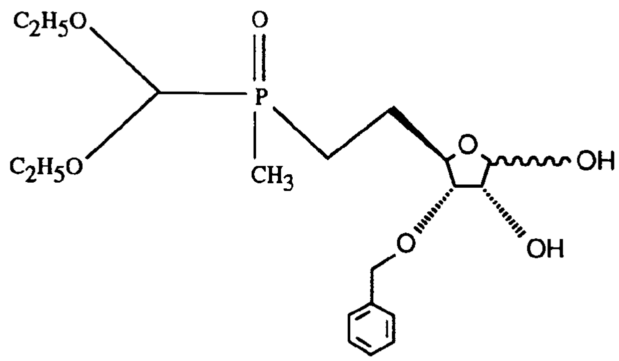

Compound 6

To compound 5 (2.35g, 0.0053M) in dimethoxyethane (25ml) is added a slurry (50ml) of Dowex 50W x 2 (100) ion exchange resin, H+ form, in water. The mixture is heated to 70°C for 3 hours (reaction monitored by TLC). The Dowex is removed by filtration and evaporation gives a yellow oil which is chromatographed on silica using 8% methanol in dichloromethane as eluant to give Compound 6.

$^{31}P$ nmr ($CDCl_3$, 162MHz) $\delta$ = 47.81 and 49.04 ppm.

Example 7

This Example describes the preparation of the compound of formula

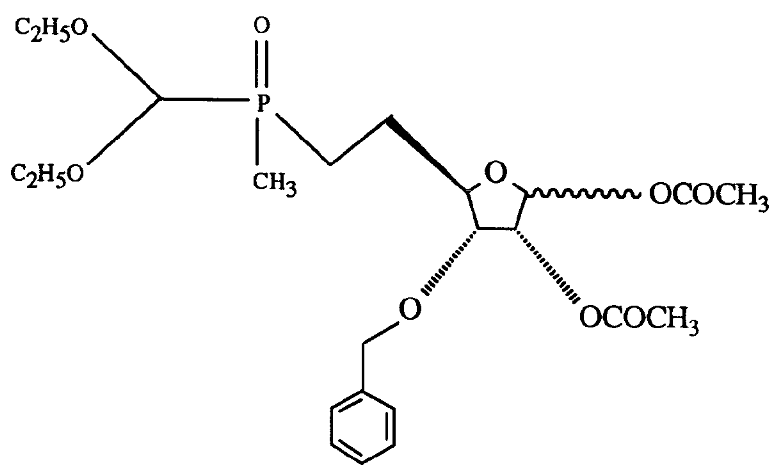

Compound 7

To a solution of Compound 6 (0.99g, 0.0025M) in pyridine (2ml) at room temperature is added acetic anhydride (1ml). This mixture is stirred at room temperature for 18 hours. Dichloromethane (20ml) is added and the solution is washed with dilute hydrochloric acid and water and then dried over $MgSO_4$. Evaporation gives a yellow oil which is chromatographed on silica using 3% methanol in dichloromethane as eluant to give Compound 7.

$^{31}P$ nmr ($CDCl_3$, 162MHz): $\delta$ 46.07, 46.15 ppm.

Example 8

This Example describes the preparation of the compound of formula

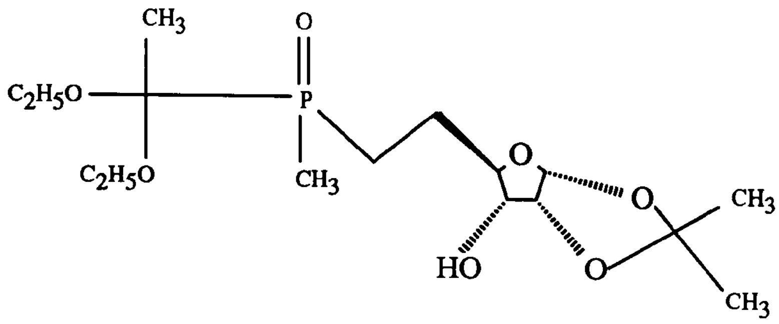

Compound 8

To a solution of Compound X (4g, 0.022M) and t-butylcyclohexylperdicarbonate (0.8g, 0.0022M) at 0°C (ice bath) under argon is added dropwise a solution of 1,1-diethoxyethyl methyl phosphine oxide (5.81g, 0.032M) in toluene (4ml). The cooling bath is removed and the solution is heated to 80°C for 4 hours. When reaction is complete (monitored by $^{31}$ P nmr and TLC) the solvent is evaporated to give a yellow oil. This oil is chromatographed on silica using 6% methanol in dichloromethane as eluant to give Compound 8.

$^{31}$ P nmr ($CDCl_3$, 162MHz) : δ 52.20, 52.48 ppm.

Example 9

This Example describes the preparation of the compound of formula

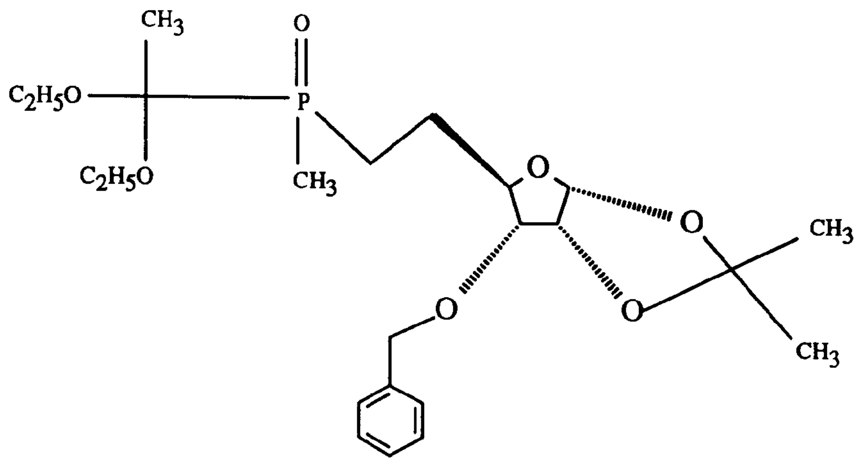

Compound 9

To a suspension of sodium hydride (0.22g, 0.0093M) in tetrahydrofuran (40ml) maintained at 5°C under argon is added dropwise a solution of Compound 8 (3.39g, 0.0093M) in tetrahydrofuran (40ml). The cooling bath is removed and the solution is stirred for 60 minutes at room temperature. The solution is then recooled to 5°C and benzyl bromide (1.74g, 0.01M) is added dropwise. The cooling bath is removed and the solution stirred at room temperature for 3 hours. When the reaction is complete (monitored by TLC), the inorganic salts are removed by filtration. Evaporation of the filtrate gives an oil which is chromatographed on silica using 5% methanol in dichloromethane as eluant to give Compound 9.

$^{31}$P nmr ($CDCl_3$, 162 MHz) : δ 50.49, 50.63 ppm.

M/Z C.I. ($NH_3$) 457 ($MH^+$).

Example 10

This Example describes the preparation of the compound of formula

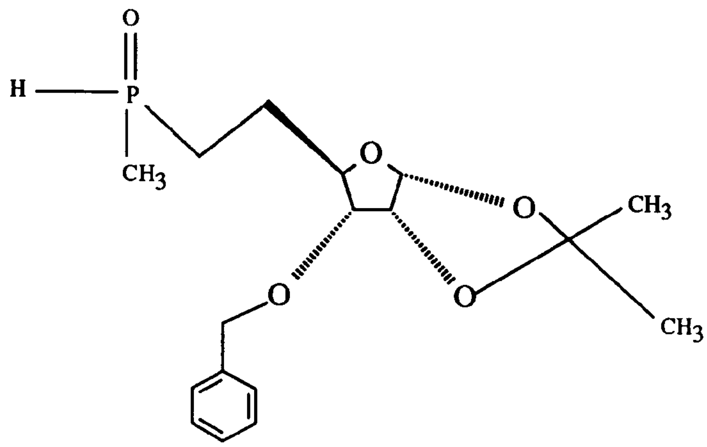

Compound 10

A solution of Compound 9 (1g, 0.0022mol) in 60% aqueous acetic acid (10ml) is heated to 70°C for two hours. When the reaction is complete (monitored by $^{31}P$ nmr), the solution is evaporated to leave an oil, which is chromatographed on silica using 6% methanol in dichloromethane as eluant to give Compound 10.

$^{31}P$ nmr ($CDCl_3$, 162MHz) : δ 27.98, 28.45 ppm.

$J_{PH}$ = 462 Hz

M/Z C.I. ($NH_3$) 341 ($MH^+$)

Example 11

This Example describes the preparation of the compound of formula

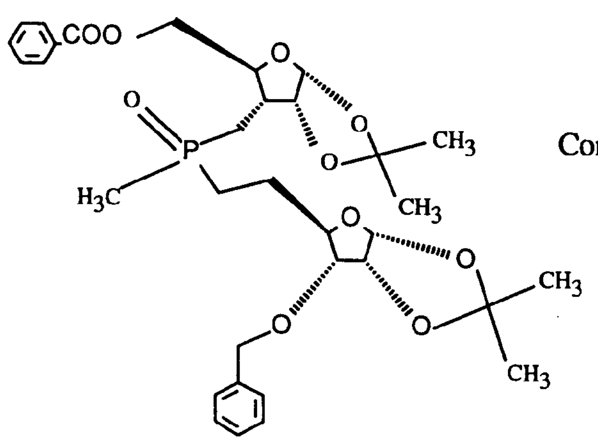

Compound 11

To a mixture of Compound Y (0.28g, 0.00096M) and t-butylcyclohexylperdicarbonate (0.04g, 0.0001M) at 0°C under argon is added Compound 10 (0.5g, 0.0015M) in toluene (1ml). The cooling bath is removed and the solution is heated to 80°C for a total of 7 hours, reaction progress being monitored by $^{31}P$ nmr and TLC. (Every 60 minutes during the 7 hours, a further 0.04g of the t-butylcyclohexylperdicarbonate initiator is added). The resulting solution is evaporated and the oil obtained is chromatographed on silica using 2% methanol in chloroform as eluant. There is obtained Compound 11 as a mixture of diastereoisomers.

$^{31}P$ nmr ($CDCl_3$, 162MHz) : δ 43.99, δ 44.63

M/Z C.I. ($NH_3$) 631 ($MH^+$).

Example 12

This Example describes the preparation from Compound 11 of the compound of formula

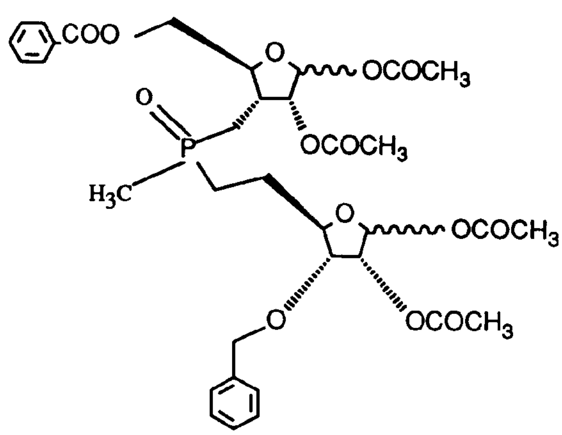

Compound 12

A solution of Compound 11 (0.1g, 0.16mmole) in 1,4-dioxan (0.7ml), trifluoroacetic acid (0.7ml) and water (0.7ml) is stirred at room temperature for 18 hours. The solution is then evaporated and coevaporated three times with toluene. To the residual oil is added pyridine (0.64ml) and acetic anhydride (0.3ml). The mixture is stirred at room temperature for 1 hour and stored at 4°C for 18 hours. The solution is evaporated and the resulting oil is chromatographed on silica using 6% methanol in chloroform as eluant. There is obtained Compound 12 as a mixture of diastereoisomers.

$^{31}P$ nmr ($CDCl_3$, 162MHz) δ 43.24, 43.47, 44.55, 44.89 ppm.

Example 13

This Example describes the preparation of the compound of formula

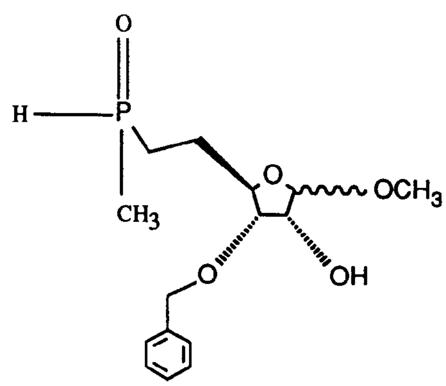

Compound 13

A solution of Compound 9 (4.64g, 0.015M) and p-toluenesulphonic acid (0.9g, 0.0047M) in methanol: water (25ml : 3ml) is heated under reflux for 3 hours. When the reaction is complete (monitored by TLC), the solution is evaporated to leave a gum which is chromatographed on silica using 5% methanol in dichloromethane as eluant to give Compound 13.

$^{31}P$ nmr ($CDCl_3$, 162 MHz) : δ 27.98, 28.50

$J_{PH}$ 461 Hz

M/Z C.I. ($NH_3$) 315 ($MH^+$)

Example 14

This Example describes the preparation of the compound of formula

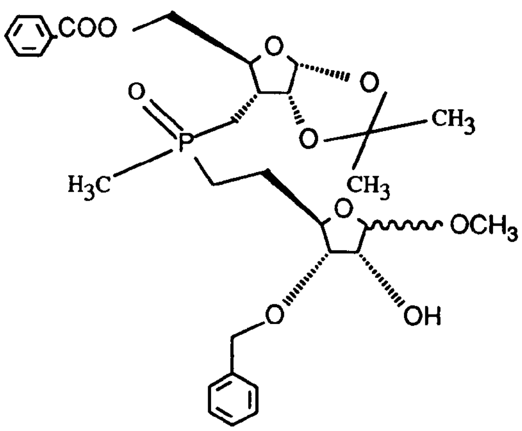

Compound 14

A solution of Compound 13 (1.04g, 0.0033M), Compound Y (1.24g, 0.0043M) and tert-butylcyclohexylperdicarbonate (0.2g, 0.0005M) in toluene (2ml) is heated to 80°C for a total of 16 hours (reaction monitored by $^{31}P$ nmr), a further 50mg of the initiator being added every 60 minutes. The resulting mixture is dissolved in dichloromethane (50ml) and the solution is washed with water (50ml) and dried ($MgSO_4$). Evaporation gives an oil which is chromatographed on silica using 1% methanol in dichloromethane as eluant. There is obtained Compound 14 as a mixture of diastereoisomers.

$^{31}P$ nmr ($CDCl_3$, 162 MHz) : δ 44.46, 45.08.

Example 15

This Example describes the preparation (from Compound 14) of the compound of formula

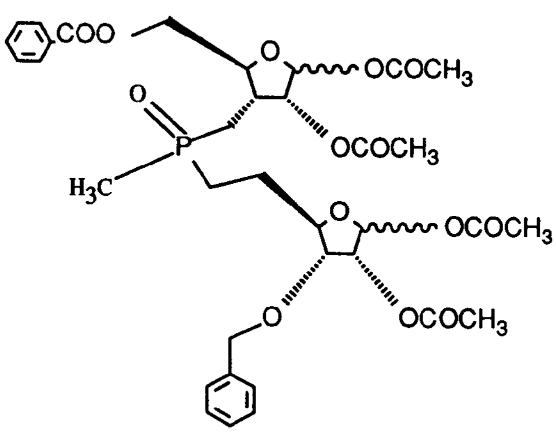

Compound 12

To Compound 14 (85mg, 0.14mmol) in dimethoxyethane (0.5ml) is added a slurry (2ml) of Dowex 50W x 2 (100) ion exchange resin, $H^+$ form, in water and the mixture is heated to 70°C for 3 hours. The resulting solution is cooled, the Dowex is removed by filtration and the filtrate solution is evaporated. The residual oil is taken up in pyridine (0.57ml) and acetic anhydride (0.27ml) is added. This solution is stirred at room temperature for 18 hours, after which it is evaporated and coevaporated three times with toluene. The oil obtained is chromatographed on silica using 5% methanol in ethyl acetate as eluant. There is obtained Compound 12 as a mixture of diastereoisomers.

$^{31}P$ nmr ($CDCl_3$, 162 MHz) : δ 43.24, 43.47, 44.55 and 44.89 ppm.

Example 16

This Examples describes the preparation of the compound of formula

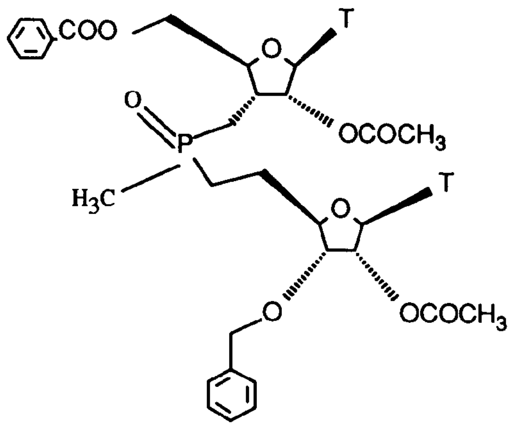

Compound 15

where T is 1-thyminyl.

A mixture of thymine (0.02g, 0.17mmol) and N, O-bis-trimethylsilylacetamide (0.07g, 0.33mmol) in dichloroethane (1 ml) is heated at 80°C under argon until a clear solution is obtained (60mins). The solution is cooled to room temperature and a solution of Compound 12 (0.06g, 0.08mmol) in dichloroethane (1.5ml) is added followed by trimethylsilyltrifluoromethane sulphonate (0.11g, 0.42mmol). The reaction mixture is heated to 50°C and stirred for 4 hours until reaction is complete (TLC). Chloroform (50ml) and water (50ml) are added, followed by saturated aqueous sodium bicarbonate until the aqueous phase is neutral. The mixture is washed with chloroform (3 x 50 ml) and the extracts are washed with water, then brine, and dried ($MgSO_4$). Evaporation gives an off-white solid which is chromatographed on silica using 5% methanol in chloroform as eluant to give Compound 15.

$^{31}P$ nmr ($CDCl_3$, 162 MHz) : δ 45.31, 45.61

M/Z ($FAB^+$) 851 ($MH^+$).

Example 17

This Example describes the preparation of the compound of formula

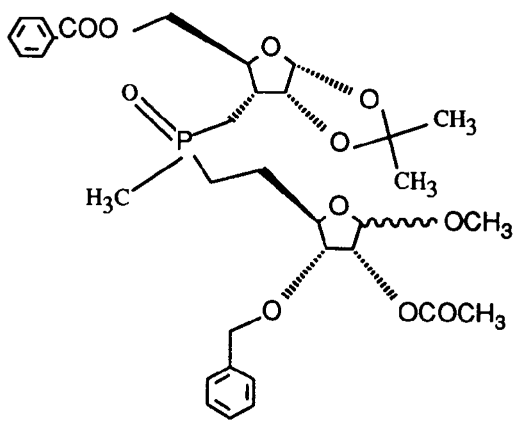

Compound 16

To a solution of Compound 14 (0.1g, 0.16mmol) in pyridine (1ml) is added acetic anhydride (0.31ml) and dimethylaminopyridine (0.004g, 0.033mmol). This mixture is stirred at room temperature for 18 hours. The resulting solution is evaporated and then co-evaporated three times with toluene. The residue is chromatographed on silica using 6% methanol in ethyl acetate as eluant to give Compound 16.

$^{31}P$ nmr ($CDCl_3$, 162MHz) : δ 44.59, 45.25.

Example 18

This Example describes the preparation of the compound of formula

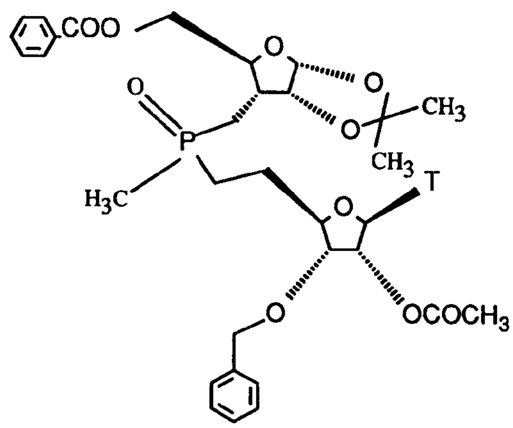

Compound 17

where T is 1-thyminyl.

A mixture of thymine (0.011g, 0.088mmol) and N, O-bis trimethylsilylamide (0.036g, 0.176 mmol) in dichloroethane (1ml) is heated to 80°C under argon until a clear solution is obtained. The solution is cooled to room temperature and a solution of Compound 16 (0.057g, 0.088 mmol) in dichloroethane (1.5ml) is added, followed by trimethylsilyltrifluoromethane sulphonate (0.058g, 0.22mmol). The reaction mixture is heated at 50°C for 4 hours until reaction is complete (TLC). Chloroform (50ml) is added and this solution is added to a well stirred solution of saturated sodium bicarbonate. The mixture is washed with chloroform (3 x 50ml) and the extracts are washed with water, then brine, and dried ($MgSO_4$). Evaporation gives a residue which is chromatographed on silica using 3% methanol in dichloromethane as eluant to give Compound 17.

$^{31}P$ nmr ($CDCl_3$, 162 MHz) : δ 44.73, 45.21.

Example 19

This Example describes the preparation of the compound of formula

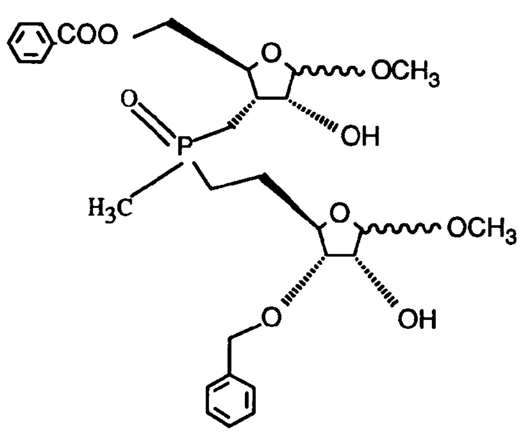

Compound 18

A solution of Compound 14 (0.1g, 0.16mmol) in methanol (1ml) and 2M hydrochloric acid (0.5ml) is stirred at 80°C for 2 hours and then at room temperature for 18 hours. The resulting solution is evaporated and coevaporated three times with toluene. The residue is chromatographed on silica using 5% methanol in dichloromethane to give Compound 18.

$^{31}P$ nmr ($CDCl_3$, 162MHz) : δ 47.57, 47.93.

Example 20

This Example describes the preparation of the compound of formula

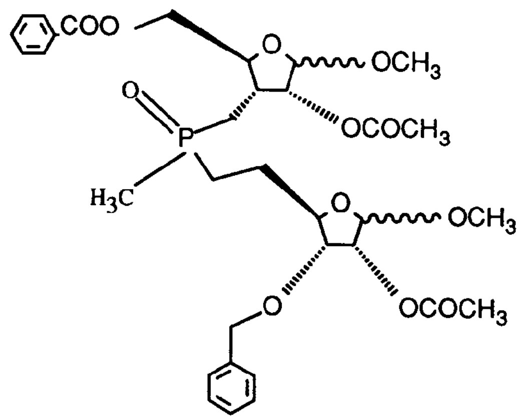

Compound 19

A solution of Compound 18 (0.06g, 0.1mmol) and acetic anhydride (0.2ml) in pyridine (1ml) is stirred at room temperature for 18 hours. The resulting solution is evaporated and then coevaporated three times with toluene. The residue is chromatographed on silica using 5% methanol in dichloromethane to give Compound 19.

$^{31}$P nmr ($CDCl_3$, 162 MHz) : δ 43.39, 44.45.

Example 21

This Example describes the preparation of the compound of formula

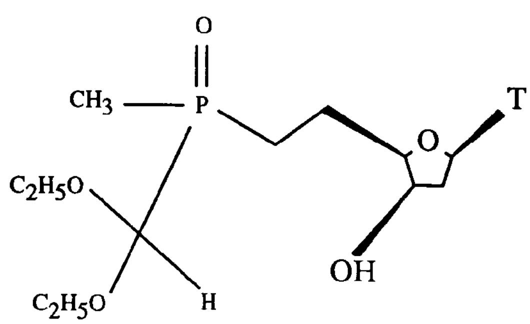

Compound 20

where T is 1-thyminyl.

To a solution of dimethyl(diethoxymethyl) phosphine oxide, prepared as described in EP 0501702 (0.40g, 2.2mmole) in dry THF (5ml) at -78°C under an atmosphere of argon is added n-butyllithium (1.37ml, 2.2mmole, 1.6 molar solution in hexanes) dropwise over 2 minutes. The resulting solution is stirred at -78°C for 1 hour. Boron trifluoride etherate (0.27ml, 2.2mmole) is then added, immediately followed by the dropwise addition of a saturated solution of 1-(3,5 anhydro-β-D-threo-pentafuranosyl)thymine (100mg, 4.5 mmole) in THF (3ml) over 1 hour. The resulting solution is stirred for 2 hours at -78°C before quenching with a suspension of sodium bicarbonate in water (1.5g, 5ml). After slow warming to room temperature, the mixture is concentrated, dichloromethane is added (100ml) and the resulting solution is dried over magnesium sulphate. Concentration and purification by flash silica column chromatography (chloroform-ethanol 20:1) and further purification by flash silica column chromatography (ethylacetate-ethanol 4:1), gives Compound 20.

$^{31}$P nmr $^{1}$H decoupled ($CDCl_3$, 36MHz) δ 48.8, 48.4 ppm.

Example 22

This Example describes the preparation of

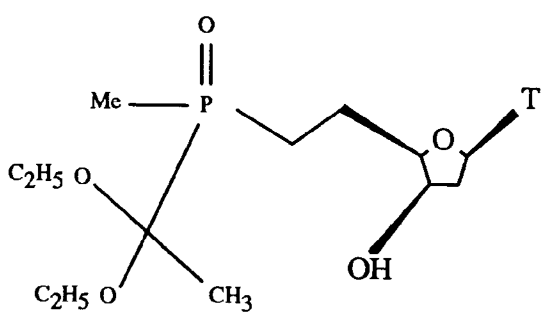

Compound 21

where T is 1-thyminyl.

To a solution of dimethyl (1,1-diethoxyethyl) phosphine oxide (11g, 56.6mmole) in dry THF (100ml) at -78°C under an atmosphere of argon is added n-butyllithium (35ml, 1.6 molar solution in hexanes) dropwise over 10 minutes. The resulting solution is stirred at -78°C for 2 hours. Boron trifluoride etherate (7ml, 57 mmole) is then added over 5 minutes, immediately followed by the dropwise addition of a solution of 1-(3, 5 anhydro-β-D-threo-pentafuranosyl)thymine (2.51g, 11.2mmole) in dry THF (50-100ml) over 45 minutes. The resulting mixture is stirred for 2 hours at -78°C before quenching with a suspension of $NaHCO_3$ in water (5g, 10ml). The resulting mixture is slowly warmed to room temperature and then concentrated. Dichloromethane (200ml) is added with magnesium sulphate. After a few minutes, the solution is filtered off and the solids washed with dichloromethane (4 x 100ml). Concentration of the filtrate and purification by flash silica column chromatography (gradient elution: ethyl acetate-ethanol 20:1 - 8:1) gives Compound 21 as a white foam.

Found: C 49.4; H 7.8; N 6.3; P 6.8%.$C_{18}H_{31}N_2O_7P.H_2O$ requires C 49.5; H 7.6; N 6.4; P 7.1%.

$^{31}$P nmr $^1$H decoupled ($CDCl_3$, 162MHz) δ 54.6, 53.2 ppm.

Example 23

This Example describes the preparation of the compound of formula

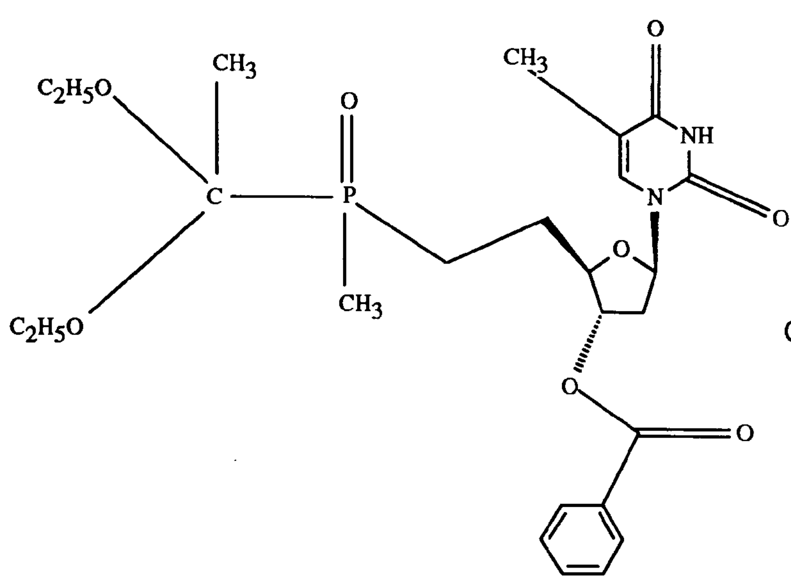

Compound 22

To a solution of Compound 21 (0.50g, 1.2mmol), triphenylphosphine (0.34g, 1.3mmol) and benzoic acid (0.22g, 1.8mmol) in THF (5ml) under argon, toluene (3ml) is added slowly. To the resulting solution diethylazodicarboxylate (200 μl, 1.3mmol) is added dropwise. After 20 hours at room temperature, the reaction mixture is concentrated and purified by flash chromatography on silica gel, eluting with an ethyl acetate/ethanol gradient. Compound 22 is obtained as a mixture of two diastereomers at phosphorus.

$^{31}$P nmr $^1$H decoupled ($CDCl_3$, 161.9MHz) δ 51,3, 51.1 ppm.

M/Z C.I. ($NH_3$) 523 ($MH^+$).

Example 24

This Example describes the preparation of the compound of formula

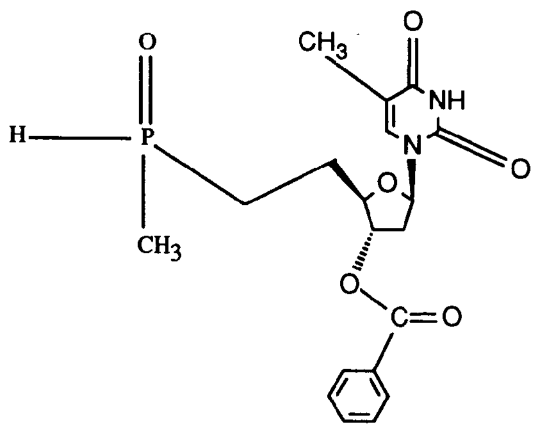

Compound 23

To a solution of Compound 22 (0.90g, 1.7 mmol) in chloroform (10ml) and ethanol (1ml) under argon, chlorotrimethylsilane (1.1ml, 8.6mmol) is added dropwise over two minutes, at room temperature. The reaction mixture is kept at 10°C for 16 hours, and then concentrated. The resulting mixture is dissolved in dichloromethane and washed with a dilute aqueous solution of sodium bicarbonate. The organic layer is dried with brine and magnesium sulphate, and evaporated to give a gum. Chromatographic purification on silica gel, eluting with a chloroform/methanol gradient gives Compound 23 as a mixture of two diastereomers at phosphorus, m.p. 90-95°C.

$^{31}P$ nmr $^{1}H$ decoupled ($CDCl_3$, 161.9 MHz) δ 26.9, 26.9 ppm.
M/Z C.I. ($NH_3$) 424 ($MNH_4^+$), 407 ($MH^+$)

Example 25

This Example describes the preparation of the compound of formula

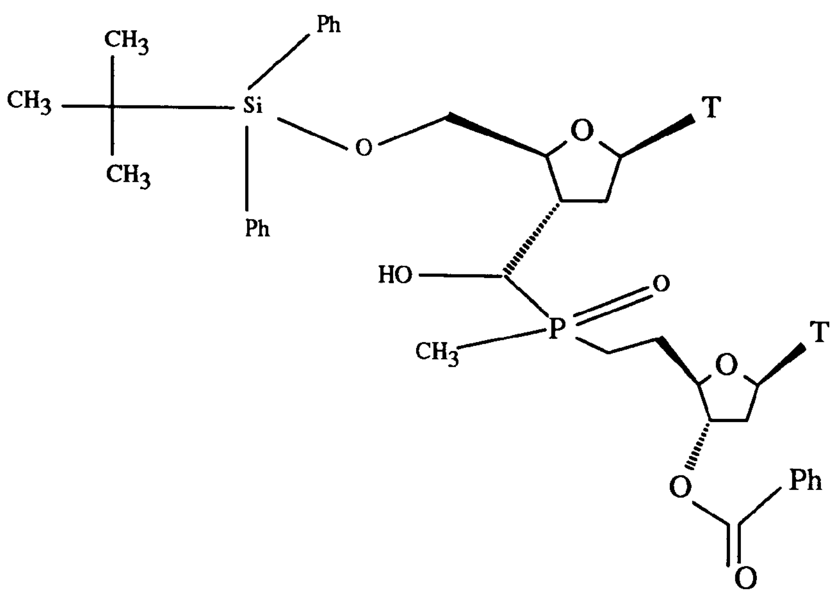

Compound 24

where Ph is phenyl and T is 1-thyminyl.

1,8-Diazobicyclo [5.4.0]undec-7-ene (DBU) (0.15ml, 1.01mmol) is added dropwise to a solution of Compound 23 (0.41g, 1.01mmol) and an aldehyde of formula IX where $R^{10}$ is tert-butyl diphenylsilyl, $R^{13}_a$ is hydrogen and $R^{14}$ is 1-thyminyl (0.50g, 0.99mmol) in THF (7ml) under argon at room temperature. After 4 hours, a further quantity of the aldehyde (0.05g, 0.09mmol) is added. After a further 4½ hours, the reaction mixture is

concentrated, and purified by flash chromatography on silica gel, eluting with a chloroform/methanol gradient. Compound 24 is obtained as a mixture of four diastereomers.

$^{31}P$ nmr $^{1}H$ decoupled ($CDCl_3$, 161.9MHz) δ 53.5, 53.0, 51.0, 49.8 ppm

M/Z ($FAB^+$) 899 ($MH^+$), 921 ($MNa^+$).

Example 26

This Example describes the preparation of the compound of formula

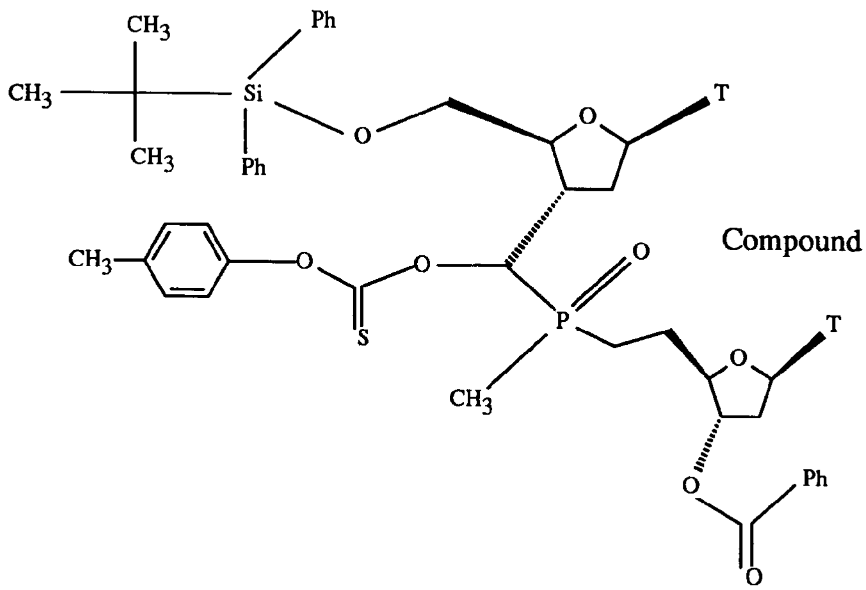

Compound 25

where Ph is phenyl and T is 1-thyminyl.

A solution of Compound 24 (0.63g, 0.70mmol), 4-(dimethylamino)pyridine (86mg, 0.70mmol) and triethylamine (98μl, 0.70mmol) in dry dichloromethane (15ml) under argon is cooled to 0-5°C, and a solution of p-tolylchlorothionoformate (0.12ml, 0.77mmol) in dichloromethane (lml) is added over 30 minutes. The solution is allowed to warm to room temperature, and kept at this temperature for 18 hours. The resulting mixture is diluted with dichloromethane, washed with a dilute solution of sodium hydrogen carbonate, and dried with magnesium sulphate. After evaporation, the crude product is purified by chromatography on silica gel, eluting with a chloroform/methanol gradient.

Compound 25 is obtained as a mixture of four diastereomers.

$^{31}P$ nmr $^{1}H$ decoupled ($CDCl_3$, 161.9 MHz) δ 48.3, 47.3, 46.7, 46.1 ppm.

Example 27

This Example describes the preparation of the compounds of formula

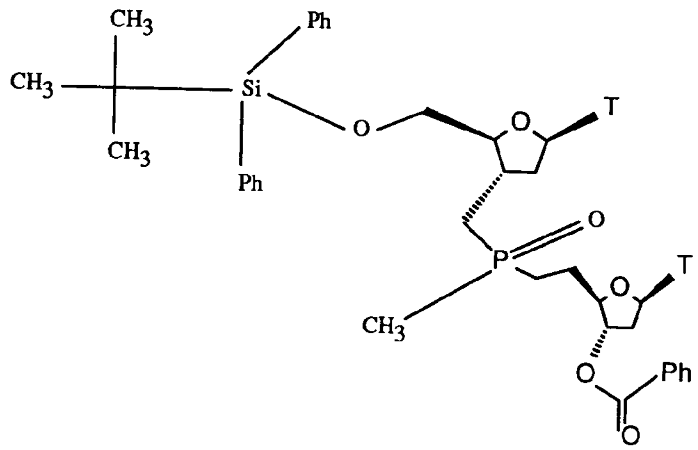

Compounds 26, 26A and 26B

where Ph is phenyl and T is 1-thyminyl.

A solution of Compound 25 (0.40g, 0.38mmol) and tri-n-butylstannane (0.12ml, 0.46mmol) in dry toluene (5ml) under an argon atmosphere is heated to 100°C, and azobis(isobutyrylnitrile) (6mg, 0.04mmol) is added. Over 3 hours, additional azobis(isobutyrylnitrile) (9mg, 0.06mmol) is added. After 4.25 hours, the reaction mixture is concentrated, dissolved in acetonitrile (15ml) and washed with hexane. The acetonitrile phase is concentrated and purified by column chromatography on silica gel, eluting with an ethyl acetate/methanol gradient. Compound 26 is obtained as a mixture of two diastereomers.

Found: C 61.8; H 6.2; N 6.2; P 3.2%; $C_{46}H_{55}N_4O_{10}PSi.H_2O$ requires C 61.3; H 6.4; N 6.2; P 3.4%.

$^{31}$P nmr $^1$H decoupled ($CDCl_3$, 161.9MHz) δ 44.6ppm

M/Z ($FAB^+$) 883 ($MH^+$)

Separation of Diastereoisomers

One of the isomers, Compound 26A, is separated by recrystallisation from methanol/ethyl acetate.

$^{31}$P nmr$^1$H decoupled ($CDCl_3$,161.9 MHz) δ 44.8 ppm.

The other isomer, Compound 26B, is obtained by chromatography of the mother liquor on silica gel. Elution with a gradient of methanol in ethyl acetate gives first Compound 26B

$^{31}$P nmr $^1$H decoupled ($CDCl_3$,161.9 MHz) δ 44.6 ppm.

Example 28

This Example describes the preparation of the compounds of formula

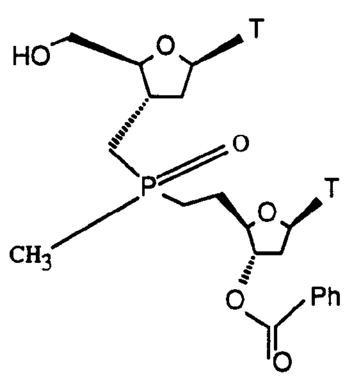

Compounds 27 and 27A

where Ph is phenyl and T is 1-thyminyl.

To a solution of Compound 26 (143mg, 0.16mmol) in dry THF (1.5ml), tetrabutylammonium fluoride trihydrate (51mg, 0.18mmol) and acetic acid (9μl, 0.16mmol) are added. After maintaining the reaction mixture at -18°C for 20 hours, Dowex 50W x 2 - $NH_4^+$ ion exchange resin is added. The resin is separated by filtration and washed with methanol. The methanol solution is evaporated to give Compound 27.

The procedure is repeated using the single isomer Compound 26A in place of Compound 26. The product

is a single isomer - Compound 27A.

Example 29

This Example describes the preparation of the compounds of formula

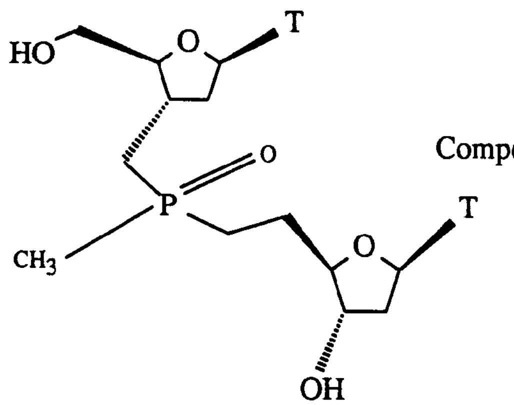

Compounds 28 and 28A

where T is 1-thyminyl.

Compound 27 as obtained in Example 28 is dissolved in dry methanol (2ml) under an argon atmosphere, and sodium methoxide (25% weight solution in methanol, 40μl) is added dropwise. The reaction mixture is maintained at 10°C for 16 hours and 20°C for 5 hours, and then a further portion of the sodium methoxide solution (40μl) is added. After a further 1.5 hours, Dowex 50W x 2-$H^+$ ion exchange resin is added and the mixture is stirred for 12 minutes. The ion exchange resin is separated by filtration and washed with methanol. The methanol solution is concentrated and purified by column chromatography on silica gel, eluting with an ethyl acetate/ethanol gradient. Compound 28 is isolated as a mixture of two diastereomers.

Found: C 48.5; H 6.2; N 9.5; P 5.1%; $C_{23}H_{33}N_4O_9P.2H_2O$ requires C 47.9; H 6.5; N 9.7; P 5.4%.

$^{31}$ P nmr $^1$H decoupled ($CD_3OD$, 161.9MHz) δ 53.0, 52.9 ppm.

M/Z ($FAB^+$) 541 ($MH^+$), 563 ($MNa^+$).

The procedure is repeated, replacing Compound 27 by Compound 27A. The product is a single isomer - Compound 28A.

$^{31}$P nmr $^1$H decoupled ($CD_3OD$,161.9 $MH_z$) δ 53.06 ppm

Example 30

This Example describes the preparation of the compound of formula

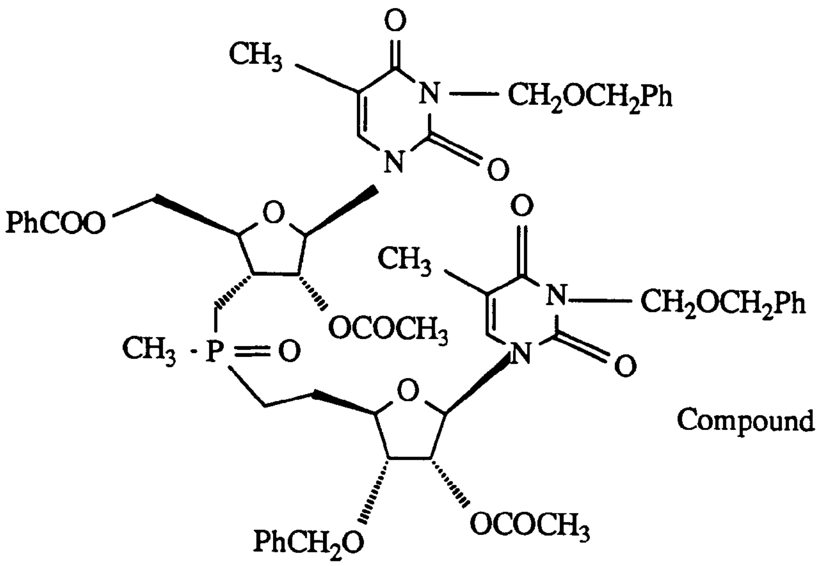

Compound 29

where Ph is phenyl.

To a solution of Compound 15 (O.35g, 0.41 mmol) in acetonitrile (5ml) at 0°C under argon is added 1.8-diaza-bicyclo [5.4.0] undec-7-ene (0.15g, 0.98mmol) followed by benzylchloromethyl ether (0.15g, 0.96mmol). The mixture is stirred for 5 hours at 0°C and then at room temperature until reaction is complete (monitored by TLC). The mixture is dissolved in dichloromethane and washed with aqueous 10% potassium hydrogen sulphate solution and then with water. The organic layer is dried with magnesium sulphate and evaporated to a foam. This is chromatographed on silica using 4% methanol in ethyl acetate as eluant to give Compound 29.

Found: C62.5, H6.0, N5.3, P2.6%; $C_{57}H_{63}N_4O_{16}P$ requires C62.7, H5.8, N5.1, P2.8%.

$^{31}P$ nmr ($CDCl_3$, 162 MHz) : $\delta$ 43.7 ppm

M/Z (FAB+) 1091 (MH+)

Example 31

This Example describes the preparation of the compound of formula

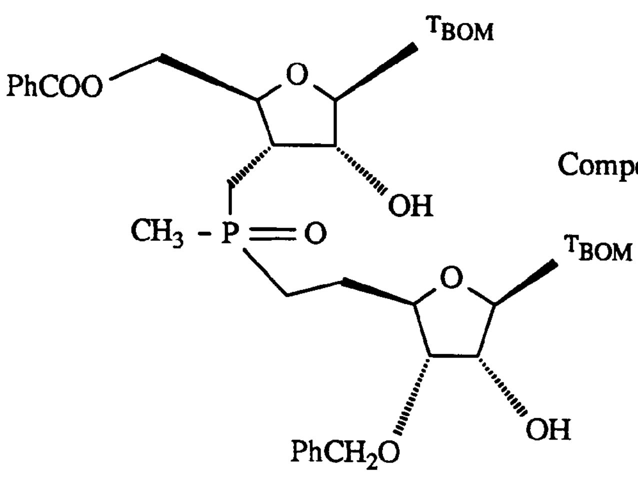

Compound 30

where Ph is phenyl and $T_{BOM}$ is 1-(N-benzyloxymethyl)thyminyl.

To a solution of Compound 29 (0.21g, 0.19mmol) in dichloromethane (lml) at room temperature is added a 50% solution of ammonia in methanol (2ml). This solution is stirred for 60 minutes at room temperature. The reaction mixture is then dissolved in dichloromethane and washed with water. The organic layer is dried with magnesium sulphate and evaporated. The residue is chromatographed on silica using 2% methanol in dichloromethane as eluant to give Compound 30.

$^{31}P$ nmr ($CDCl_3$, 162 MHz) = δ 48.02, 48.24 ppm

Example 32

This Example describes the preparation of the compound of formula

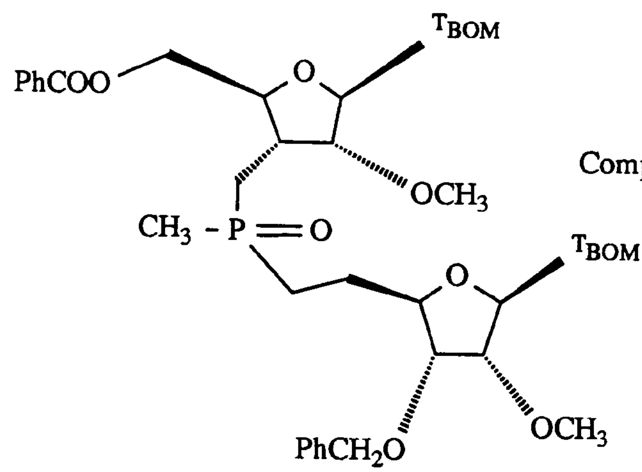

Compound 31

where Ph is phenyl and $T_{BOM}$ is 1-(N-benzyloxymethyl)thyminyl.

To a solution of Compound 30 (0.12g, 0.12mmol) in acetonitrile (2ml) at 0°C is added 2-tert-butylimino-2-diethylamino- 1,3-dimethylperhydro- 1,3,2-diaza-phosphorine (BDDDP) (0.099g, 0.36 mmol) followed by methyl iodide (0.051g, 0.36 mmol). The cooling bath is removed and the solution stirred at room temperature until reaction is complete (monitored by TLC). The mixture is evaporated and the residue chromatographed on silica using 4% methanol in ethyl acetate as eluant to give Compound 31.

$^{31}P$ nmr ($CDCl_3$, 162 MHz) : δ 43.88, 44.24 ppm

Example 33

This Example describes the preparation of the compound of formula

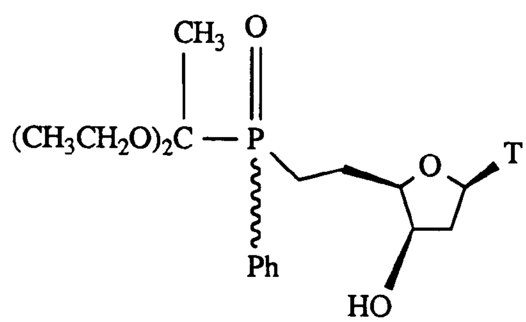

Compound 32

where Ph is phenyl and T is 1-thyminyl.

To a solution of Compound Z (7.2g, 28mmol) in THF (15ml) at -75°C, n-butyl lithium (1.6M in hexanes, 18ml, 29 mmol) is added dropwise over 25 minutes. After a further 45 minutes, boron trifluoride etherate (3.5ml, 28mmol) is added dropwise over four minutes, followed immediately by a solution of 1-(3,5 anhydro-β-D-threo-pentafuranosyl)thymine (1.25g, 5.63mmol) added over ten minutes. After 4 hours, a saturated aqueous solution of sodium bicarbonate (30ml) is added. The resulting mixture is evaporated to dryness and the resulting solid washed with chloroform. The organic solution is evaporated and the residue is purified by chromatography on silica gel to give Compound 32 as a mixture of two isomers.

$^{31}P$ nmr ($CDCl_3$, 36 MHz) δ 44.2, 42.2 ppm

M/Z($FAB^+$) 481($MH^+$)

Example 34

This Example describes the preparation of the compound of formula

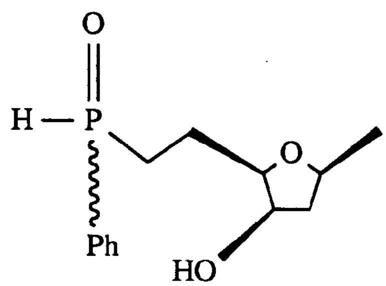

Compound 33

where Ph is phenyl and T is 1-thyminyl.

To a solution of Compound 32 (0.70g, 1.5mmol) in chloroform (7ml) and ethanol (0.14ml), chlorotrimethylsilane (1.9ml, 15mmol) is added dropwise. After 18 hours, the solution is evaporated and the residue is purified by chromatography on silica gel, eluting with a gradient of ethanol in chloroform to give Compound 33 as a mixture of two isomers.

$^{31}P$ nmr ($CDCl_3$, 162 MHz) δ 30.1, 28.4 ppm

$J_{PH}$ 474 Hz

M/Z ($FAB^+$) 365 ($MH^+$)

Example 35

This Example describes the preparation of the compound of formula

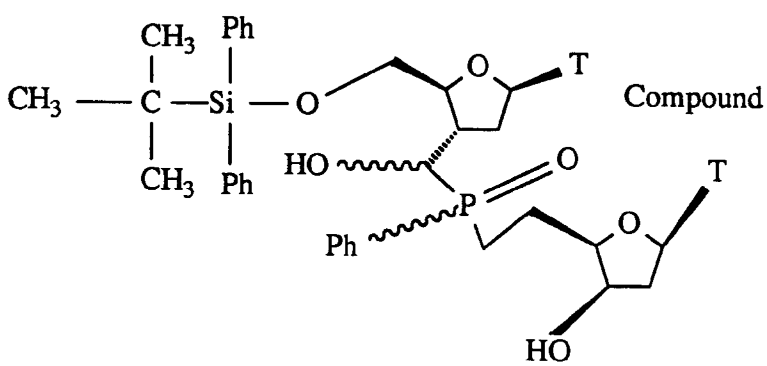

Compound 34

where Ph is phenyl and T is 1-thyminyl.

To a stirred solution of Compound 33 (150mg, 0.41mmol) and an aldehyde of Formula 1X where $R^{10}$ is tert-butyldiphenylsilyl, $R^{13}$a is hydrogen and $R^{14}$ is 1-thyminyl (203mg, 0.41mmol) in THF (4.5ml), DBU (61μl, 0.41mmol) is added dropwise. After 4 hours, the reaction mixture is evaporated and the residue is purified by chromatography on silica gel, to give Compound 34 as a mixture of four isomers.

$^{31}P$ nmr ($CDCl_3$-$CD_3OD$, 162 MHz) δ 47.1, 45.1, 44.0 ppm

M/Z )$FAB^+$) 879 ($M+Na^+$), 857($MH^+$)

Example 36

This Example describes the preparation of compounds of formula

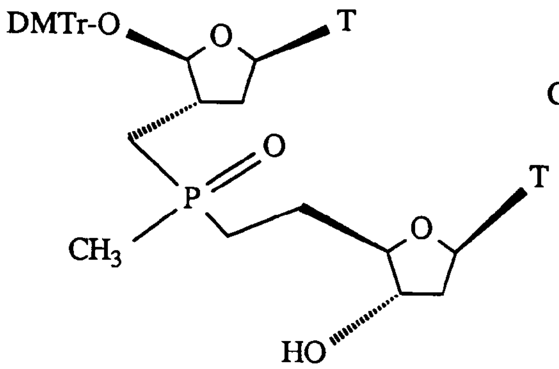

Compounds 35 and 35A

where DMTr is dimethoxytriphenylmethyl and T is 1-thyminyl.

Compound 35

To a solution of Compound 28 (400mg, 0.74mmol) in dry pyridine (16ml) at room temperature under argon atmosphere are added triethylamine (148mg, 1.47mmol), dimethylaminopyridine (6.5mg, 0.05mmol) and 4,41-dimethoxytriphenylmethyl chloride (427mg, 1.26mmol). After 1.5 hours at room temperature, a second portion of triethylamine (23mg, 0.22mmol), dimethylaminopyridine (1mg, 0.008mmol) and dimethoxytriphenylmethyl chloride (66mg, 0.19mmol) are added. After stirring at room temperature overnight, a third portion of triethylamine (23mg, 0.22mmol), dimethylaminopyridine (1mg, 0.008mmol) and dimethoxytriphenylmethyl chloride (66mg, 0.19mmol) are added. After 3 hours, the reaction mixture is poured into water (20ml). The aqueous layer is extracted with ethyl acetate (3x20ml), the combined organic fractions are dried ($Na_2SO_4$) and the solvent is removed under vacuum. Flash-chromatography on silica gel (eluant: $CH_2C1_2/C_2H_5OH$:6/1 and 1% triethylamine) gives Compound 35.

$^{31}P$ nmr ($CDC1_3$, 101 MHz) δ 46.2 ppm.

FAB-MS $(M+H)^+$ = 843

Compound 35A

The procedure used for the preparation of Compound 35 is repeated, but using Compound 28A (350mg, 0.65mmol) in place of Compound 28 and using 91mg (0.91mmol) of triethylamine, 4mg (0.032mmol) of dimethylaminopyridine, 263mg (0.78mmol) of dimethoxytriphenylmethyl chloride and 14ml of pyridine. The product is Compound 35A.

$^{31}P$ nmr ($CDCl_3$, 101 MHz) δ 45.8 ppm.

Example 37

This describes the preparation of compounds of formula

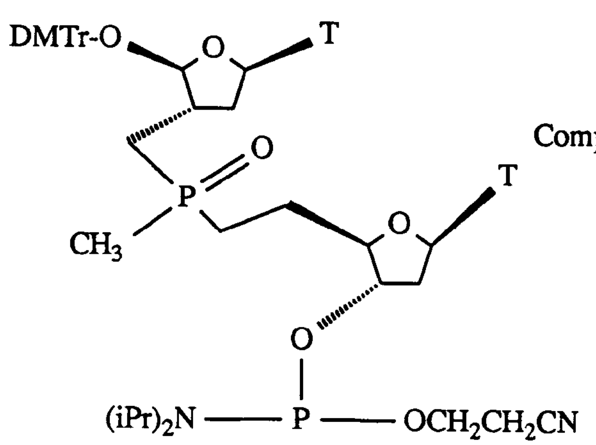

Compounds 36 and 36A

where DMTr is dimethoxytriphenylmethyl, iPr is isopropyl and T is 1-thyminyl.

Compound 36

A solution of Compound 35 (389mg, 0.46mmol) in dry dichloromethane (18ml) is added dropwise at room temperature to a mixture of diisopropylammonium tetrazolide (120mg, 0.70mmol) and 2-cyanoethyl-N,N,$N^1$,$N^1$-tetraisopropylphosphoro diamidite (191mg, 0.63mmol) in dry dichloromethane (25ml) under an argon atmosphere. After 2.5 hours at room temperature, the reaction mixture is stirred at 40°C for 4 hours. Further diisopropylammonium tetrazolide (34mg, 0.2mmol) and 2-cyanoethyl-N,N,$N^1$,$N^1$-tetraisopropylphosphorodiamidite (54mg, 0.18mmol) are added and, after stirring at 40°C overnight, the mixture is poured into a saturated aqueous $NaHCO_3$ solution (30ml). The aqueous layer is extracted with dichloromethane (3x20ml). The combined organic layers are dried ($Na_2SO_4$) and evaporated. Flash chromatography on silica gel (eluant: $CH_2C1_2$/ethanol: 9/1 and 1% triethylamine) gives an oil. Pure Compound 36 is obtained by dissolving the residue in dry chloroform and precipitating with pentane. After co-evaporation with benzene, Compound 36 is isolated.

$^{31}P$ nmr ($CDC1_3$, 101 MHz): δ 43.4, 43.9 and 148.7 ppm.

Compound 36A

The procedure for the preparation of Compound 36 is repeated, but using Compound 35A (543mg, 0.64mmol) in place of Compound 35 and using 133mg (0.77mmol) of diisopropylammonium tetrazolide and 213mg (0.71mmol) of 2-cyanoethyl-N,N,$N^1$,$N^1$-tetra-isopropylphosphoro diamidite in 17ml of dry dichloromethane. The product is Compound 36A.

$^{31}P$ nmr ($CDC1_3$, 101 MHz) δ 43.9, 44.0, 148.5 and 148.7 ppm.

Examples 38 - 41

Oligonucleotides are prepared from Compounds 36 and 36A and similarly DMTr-protected and phosphoramidite-activated natural nucleosides -$2^1$-deoxycytidine (dC), -$2^1$-deoxyguanosine (dG) and thymidine (dT)- on an ABI 390 automatic DNA synthesiser (available from Applied Biosystems Inc) using standard phosphoramidite chemistry according to Oligonucleotide Synthesis: A Practical Approach, M.J. Gait, IRL Press, Oxford 1984, but with prolonged coupling times (10 minutes). DMTr oligonucleotides are purified by reverse phase HPLC (eluant A = 50mM triethylammonium acetate (TEAA), pH 7.0; eluant B:50mM TEAA, pH7.0 in 70% acetonitrile; elution with gradient from 15% to 45% B in 45 minutes). After purification by HPLC, the DMTr protective group is removed by treatment with 80% aqueous acetic acid and the oligonucleotides are precipitated with ethanol and isolated by centrifugation. The purity of the oligodeoxynucleotides is checked by capillary gel electrophoresis (polyacrylamide; buffer: 100mM $H_3PO_4$, 100mM Tries, 2mM ethylenediaminetetracetic acid, 7M urea, pH 8.8).

The prepared oligonucleotide sequences are as follows:

Examples 38-39

GCG T*TT *TT*T T*TT *TGC G

T*T indicating the unit derived from the modifying dinucleotide.

| Example | Modifying Dinucleotide |
|---|---|
| 38 | Compound 36 |
| 39 | Compound 36A |

Examples 40-41

TTT T*TC TCT CTC TCT

T*T indicating the unit derived from the modifying dinucleotide.

| Example | Modifying Dinucleotide |
|---|---|
| 40 | Compound 36 |
| 41 | Compound 36A |

The structure of the oligodeoxynucleotides is checked by matrix assisted laser-desorption time-of-flight (MALDI-TOF) mass spectroscopy; the oligonucleotides being desorbed using 2,4,6-trihydroxyacetophenone as a matrix with diammonium hydrogen citrate as additive (25mM final concentration) (U.Pieles et al, Nucl. Acids Res. 1993, 21, 3191).

The ability of the oligonucleotides to hybridise to their base-complementary natural DNA and RNA sequences is assessed by recording UV melting curves and determining the melting temperature (Tm) values therefrom. The method is described, for example, by S.M. Freier et al, Biopolymers, 1982, 22, 1107. The thermal denaturation of the DNA/RNA hybrids is performed at 260nm using a Gilford Response II Spectrophotometer (available from Ciba-Corning Diagnostics).

Absorption vs temperature profiles are measured at 4μM of each strand in 10mM phosphate, 10OmM $Na^+$, 0.1mM EDTA, pH 7.0. The Tm values are determined from the melting curves obtained. The change in Tm per number of modifying units in the oligonucleotide compared with the corresponding unmodified natural oligonucleotides (ΔTm/mod) is recorded for each oligonucleotide. The results are given below:

| Oligonucleotide | Complementary Sequence | Tm(°C) | ΔTm/mod(°C) |
|---|---|---|---|
| Ex. 38 | RNA | 38.2 | - 2.1 |
| Ex. 38 | DNA | 33.8 | - 3.9 |
| Ex. 39 | RNA | 29.2 | - 3.9 |
| Ex. 39 | DNA | 22.3 | - 6.2 |
| Ex. 40 | RNA | | - 2.4 |
| Ex. 41 | RNA | | - 3.5 |

Example 42

Compound 34 is tested for antiviral activity against herpes simplex virus type 1(HSV-1) (strain 17i) and herpes simplex virus type 2(HSV-2) (strain HG52) in vitro. Aqueous solutions of the compounds are prepared at concentrations between 10mM and 50MM. The solutions are stored at -70°C after preparation and thawed prior to use in the antiviral assays. After thawing, the solutions are diluted to the appropriate concentration in the cell-culture medium without prior filtration.

In a procedure similar to that described by Tyms et al, J. Antimicrob. Chemother, 8, 65-72 (1981), cell monolayers are infected with 20-200 plaque forming units and after virus adsorption the inoculum is replaced by maintenance medium containing different concentrations of the compound under investigation. Virus spread is prevented by the incorporation of 0.5% low gelling temperature agarose. At the end of a set period (2 or 3 days) monolayers are fixed, stained with methylene blue and plaque numbers determined. The results are as follows:

| Virus | Cells | $IC_{50}$ |
|---|---|---|
| HSV-1 | Vero | 2.6 μm |
| HSV-2 | Vero | 3.8 μm |

## Claims

1. A compound of formula

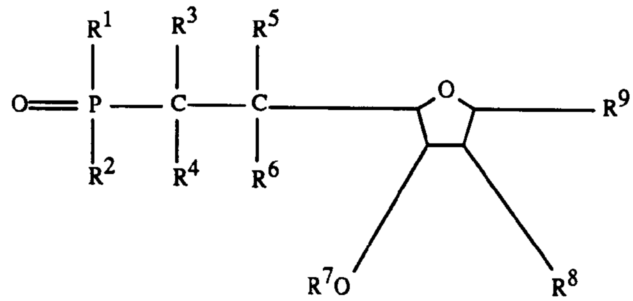

I

where $R^1$ is hydrogen, $R^1_a$ or a group of formula

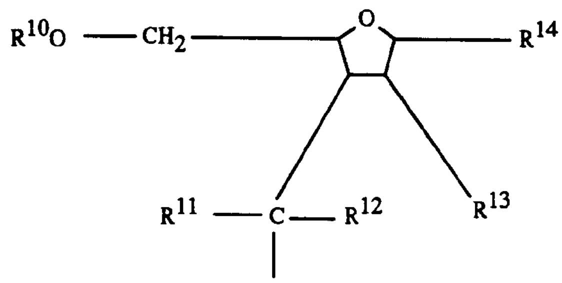

II

$R^1_a$ is $R^1_b$ or a protecting group Q,
$R^1_b$ is $C_1$-$C_{20}$ alkyl, $C_2$-$C_{20}$ alkenyl, $C_3$-$C_{10}$ cycloalkyl, $C_6$-$C_{15}$ aryl, $C_7$-$C_{16}$ aralkyl or a 5- or 6- membered heterocyclic group attached by a carbon atom in the heterocyclic group to the indicated phosphorus atom,
$R^2$ is an unsubstituted or substituted $C_1$-$C_{20}$ alkyl or $C_2$-$C_{20}$ alkenyl group, an unsubstituted or substituted $C_3$-$C_{10}$ cycloalkyl group, an unsubstituted or substituted $C_6$-$C_{15}$ aryl group, an unsubstituted or substituted $C_7$-$C_{16}$ aralkyl group, or a 5- or 6- membered heterocyclic group attached by a carbon atom in the heterocyclic group to the indicated phosphorus atom,
$R^3$ is hydrogen, halogen, hydroxy, $R^{15}$, $-OR^{15}$ or $-OSO_2R^{15}$
$R^4$ is $R^4_a$ or together with $R^6$ denotes a valence bond,
$R^4_a$ is hydrogen, halogen or $R^{16}$,
$R^5$ is hydrogen, halogen, hydroxy, $R^{17}$, $-OR^{17}$, $-OCOR^{17}$ or $-OSO_2R^{17}$,
$R^6$ is hydrogen, halogen or $R^{18}$, or together with $R^4$ denotes a valence bond,
$R^7$ is hydrogen, alkyl-N, N-dialkylphosphoramidyl or $R^7_a$, or $R^7O$- together with $R^8_a$ denotes a $C_1$-$C_{15}$ hydrocarbylidenedioxy group,
$R^7_a$ is $R^{19}$, $-COR^{19}$, $-SO_2R^{19}$ or tri($C_1$-$C_{15}$ hydrocarbyl)silyl,
$R^8$ is $R^8_a$ or Z,
$R^8_a$ is hydrogen, halogen, hydroxy, $R^{20}$, $-OR^{20}$, $-OCOR^{20}$, $-OSO_2R^{20}$, or tri($C_1$-$C_{15}$ hydrocarbyl)silyloxy, or together with $R^9$ denotes a $C_1$-$C_{15}$ hydrocarbylidenedioxy group, or together with $R^7O$- denotes a $C_1$-$C_{15}$ hydrocarbylidenedioxy group,
$R^9$ is halogen, hydroxy, $-OR^{21}$, $-OCOR^{21}$, $-OSO_2R^{21}$, or $B^2$, or together with $R^8_a$ denotes a $C_1$-$C_{15}$ hydrocarbylidenedioxy group,
$R^{10}$ is hydrogen or $R^{10}_a$,
$R^{10}_a$ is $R^{22}$, $-COR^{22}$, $-SO_2R^{22}$ or tri($C_1$-$C_{15}$ hydrocarbyl)silyl,
$R^{11}$ is hydrogen, halogen, hydroxy, $R^{23}$, $-OR^{23}$, $-OCOR^{23}$, $-OSO_2R^{23}$ or Z,
$R^{12}$ is hydrogen, halogen or $R^{24}$,
$R^{13}$ is $R^{13}_a$ or Z,
$R^{13}_a$ is hydrogen, halogen, hydroxy, $R^{25}$, $-OR^{25}$, $-OCOR^{25}$, $-OSO_2R^{25}$, or tri($C_1$-$C_{15}$ hydrocarbyl)silyloxy, or together with $R^{14}$ denotes a $C_1$-$C_{15}$ hydrocarbylidenedioxy group,
$R^{14}$ is halogen, hydroxy, $-OR^{26}$, $-OCOR^{26}$, $-OSO_2R^{26}$, or $B^1$, or together with $R^{13}_a$ denotes a $C_1$-$C_{15}$ hydrocarbylidenedioxy group,
$R^{15}$, $R^{16}$, $R^{17}$, $R^{18}$, $R^{19}$, $R^{20}$, $R^{21}$, $R^{22}$, $R^{23}$, $R^{24}$, $R^{25}$ and $R^{26}$ are independently a $C_1$ to $C_{10}$ aliphatic group, a $C_3$ to $C_{10}$ cycloaliphatic group, a $C_6$ to $C_{15}$ aromatic group or a $C_7$-$C_{30}$ araliphatic group,
$B^1$ and $B^2$ are independently each a monovalent nucleoside base radical, and
Z is substituted or unsubstituted $C_6$-$C_{10}$ aryloxythiocarbonyloxy;
provided that compounds where simultaneously $R^1$, $R^3$, $R^4$ and $R^6$ are hydrogen, $R^2$ is isopropyl, $R^5$ is me-

thoxy, $R^7$ is methyl, $R^8$ and $R^9$ are each hydroxy or each acetoxy or $R^8$ and $R^9$ together denote isopropylidenedioxy, compounds where simultaneously $R^1$, $R^3$, $R^4$ and $R^6$ are hydrogen, $R^2$ is butyl, $R^5$ is methoxy, $R^7$ is benzyl, $R^8$ and $R^9$ are each hydroxy or each acetoxy or $R^8$ and $R^9$ together denote isopropylidenedioxy, and compounds where simultaneously $R^1$, $R^3$, $R^4$, $R^5$, $R^6$ and $R^7$ are hydrogen, $R^2$ is phenyl and $R^8$ and $R^9$ together denote isopropylidenedioxy are excluded.

**2.** A compound according to claim 1, in which the aliphatic groups are independently substituted or unsubstituted alkyl or alkenyl groups, the cycloaliphatic groups are substituted or unsubstituted cycloalkyl groups, the aromatic groups are substituted or unsubstituted aryl groups and the araliphatic groups are substituted or unsubstituted aralkyl groups.

**3.** A compound according to claim 2, in which the alkyl groups are $C_1$-$C_4$ alkyl groups, the alkenyl groups are $C_2$-$C_4$ alkenyl groups, the cycloalkyl groups are $C_5$-$C_8$ cycloalkyl groups, the aryl groups are $C_6$-$C_{10}$ aryl groups, the $C_7$-$C_{16}$ aralkyl group is a $C_7$-$C_9$ aralkyl group and the $C_7$-$C_{30}$ aralkyl groups are $C_7$-$C_{20}$ aralkyl groups, any of which are substituted or unsubstituted.

**4.** A compound according to claim 1, 2 or 3 in which the protecting group Q is a $C_1$ to $C_{20}$ hydrocarbyl group substituted on the carbon atom thereof attached to the indicated phosphorus atom by at least one hydroxy or $C_1$-$C_{10}$ alkoxy group.

**5.** A compound according to claim 4, in which the protecting group Q is of formula

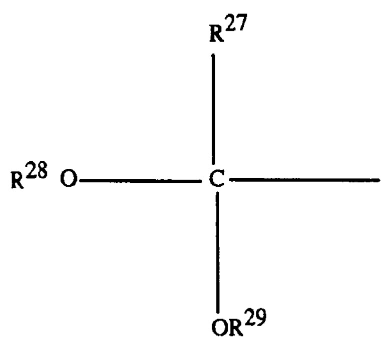

III

where $R^{27}$ is hydrogen, $C_1$ to $C_{10}$ alkyl, $C_3$ to $C_8$ cycloalkyl, $C_6$ to $C_{10}$ aryl or $C_7$ to $C_{11}$ aralkyl and $R^{28}$ and $R^{29}$ are independently each $C_1$ to $C_{10}$ alkyl.

**6.** A compound according to any of the preceding claims, in which $R^1$ is hydrogen, a protecting group Q or a group of Formula II as defined in claim 1; $R^2$ is $C_1$ to $C_4$ alkyl, $C_2$ to $C_4$ alkenyl, $C_5$ to $C_8$ cycloalkyl, $C_6$-$C_{10}$ aryl or $C_7$ to $C_9$ aralkyl; $R^3$ is hydrogen, halogen, hydroxy, $C_1$ to $C_4$ alkyl, $C_1$ to $C_4$ alkoxy, $C_7$ to $C_9$ aralkyloxy, or $-OCOR^{15}$ or $-OSO_2R^{15}$ where $R^{15}$ is $C_1$ to $C_4$ alkyl or $C_6$ to $C_{10}$ aryl; $R^4$ is hydrogen, halogen or $C_1$ to $C_4$ alkyl; $R^5$ is hydrogen, halogen, hydroxy, $C_1$ to $C_4$ alkyl, $C_1$ to $C_4$ alkoxy, $C_7$ to $C_9$ aralkyloxy or $-OCOR^{17}$ or $0SO_2R^{17}$ where $R^{17}$ is $C_1$ to $C_4$ alkyl or $C_6$ to $C_{10}$ aryl; $R^6$ is hydrogen, halogen or $C_1$ to $C_4$ alkyl; $R^7$ is hydrogen, cyano- $C_1$ to $C_4$ alkyl-N, N-di($C_1$-$C_4$ alkyl)phosphoramidyl, $C_1$ to $C_4$ alkyl, $C_7$ to $C_9$ aralkyl or $-COR^{19}$ or $-SO_2R^{19}$ where $R^{19}$ is substituted or unsubstituted $C_1$ to $C_4$ alkyl or $C_6$ to $C_{10}$ aryl; $R^8$ is hydrogen, hydroxy or $-OR^{20}$, $-OCOR^{20}$ or $-OSO_2R^{20}$ where $R^{20}$ is substituted or unsubstituted $C_1$ to $C_4$ alkyl or $C_6$ to $C_{10}$ aryl, or together with $R^9$ denotes a $C_1$-$C_5$ alkylidenedioxy group; $R^9$ is a monovalent nucleoside base radical $B^2$, hydroxy, $-OR^{21}$, $-OCOR^{21}$ or $-OSO_2R^{21}$ where $R^{21}$ is substituted or unsubstituted $C_1$ to $C_4$ alkyl or $C_6$-$C_{10}$ aryl, or together with $R^8$ denotes a $C_1$-$C_5$ alkylidenedioxy group; $R^{10}$ is hydrogen, substituted or unsubstituted $C_1$ to $C_4$ alkyl, substituted or unsubstituted $C_7$ to $C_{20}$ aralkyl, $-COR^{22}$ or $-SO_2R^{22}$ where $R^{22}$ is substituted or unsubstituted $C_1$ to $C_4$ alkyl or $C_6$ to $C_{10}$ aryl, or $C_1$ to $C_6$ alkyl di($C_6$-$C_8$ aryl)silyl; $R^{11}$ is hydrogen, halogen, hydroxy, $-OCOR^{23}$ or $-OSO_2R^{23}$ where $R^{23}$ is substituted or unsubstituted $C_1$-$C_4$ alkyl or $C_6$-$C_{10}$ aryl, or $C_1$-$C_4$ alkyl- or halogen- substituted phenyloxythiocarbonyloxy; $R^{12}$ is hydrogen or halogen; $R^{13}$ is hydrogen, hydroxy, $-OR^{25}$, $-OCOR^{25}$ or $-OSO_2R^{25}$ where $R^{25}$ is substituted or unsubstituted $C_1$ to $C_4$ alkyl or $C_6$ to $C_{10}$ aryl, or together with $R^{14}$ denotes a $C_1$ to $C_5$ alkylidenedioxy group; and $R^{14}$ is a monovalent base radical $B^1$, hydroxy, $-OR^{26}$ or $-OCOR^{26}$ where $R^{26}$ is substituted or unsubstituted $C_1$ to $C_4$ alkyl or $C_6$ to $C_{10}$ aryl, or together with $R^{13}$ denotes a $C_1$ to $C_5$ alkylidenedioxy group.

7. A compound according to claim 6, in which $R^1$ is hydrogen, a protecting group of Formula III as defined in claim 5 or a group of Formula II as defined in claim 1; $R^2$ is methyl, cyclohexyl or phenyl; $R^3$, $R^4$, $R^5$ and $R^6$ are each hydrogen; $R^7$ is hydrogen, 2-cyanoethyl-N, N-diisopropylphosphoramidyl, acetyl, benzyl or benzoyl; $R^8$ is hydrogen, hydroxy, methoxy or acetoxy, or together with $R^9$ denotes an ispropylidene-dioxy group; $R^9$ is $B^1$ which is thyminyl or N-benzyloxymethylthyminyl, or $R^9$ is hydroxy, methoxy or acetoxy, or together with $R^8$ denotes an isopropylidenedioxy group; $R^{10}$ is hydrogen, benzoyl, tert-butyldiphenylsilyl, or dimethoxytriphenylmethyl; $R^{11}$ is hydrogen, hydroxy or p-tolyoxythiocarbonyloxy; $R^{12}$ is hydrogen; $R^{13}$ is hydrogen, hydroxy, methoxy or acetoxy, or together with $R^{14}$ denotes an isopropylodenedioxy group; and $R^{14}$ is $B^2$ which is thyminyl or N-benzyloxymethylthyminyl, or $R^{14}$ is hydroxy, methoxy or acetoxy, or together with $R^{13}$ denotes an isopropylidenedioxy group.

8. A compound according to any of the preceding claims, which is of formula

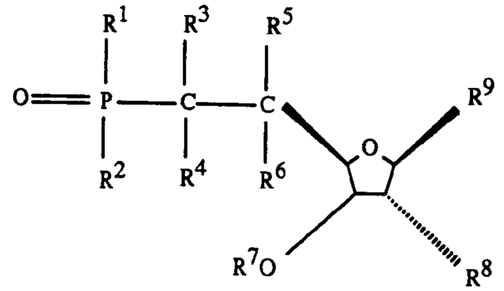

V

where $R^2$ to $R^7$ are as defined in any of claims 1 to 3, 6 and 7, $R^1$ is hydrogen, $R^1{}_a$ as defined in claim 1, 4 or 5 or a group of formula

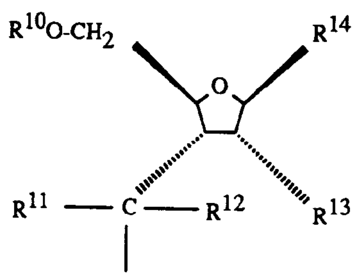

VI

and $R^{10}$ to $R^{12}$ are as defined in any of claims 1 to 3, 6 and 7, and $R^8$ is hydrogen, halogen, hydroxy, $R^{20}$, $-OR^{20}$, $-OCOR^{20}$, $-OSO_2R^{20}$, tri($C_1$-$C_{15}$ hydrocarbyl)silyloxy or Z, $R^9$ is $B^2$, halogen, hydroxy, $-OR^{21}$, $-OCOR^{21}$ or $-OSO_2R^{21}$, $R^{13}$ is hydrogen, halogen, hydroxy, $R^{25}$, $-OR^{25}$, $-OCOR^{25}$, $-OSO_2R^{25}$, tri($C_1$-$C_{15}$ hydrocarbyl)silyloxy or Z, $R^{14}$ is $B^1$, halogen, hydroxy, $-OR^{26}$, $-OCOR^{26}$ or $-OSO_2R^{26}$ and Z is as defined in claim 1, or a compound of formula

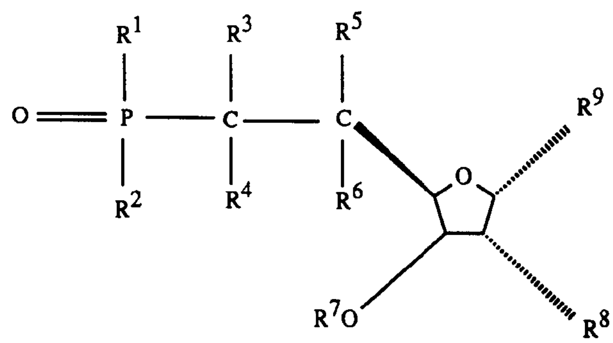

VA

where $R^2$ to $R^8$ are as defined in any of claims 1 to 3, 6 and 7, $R^1$ is hydrogen, $R^1{}_a$ as defined in claim 1, 4 or 5, or a group of formula

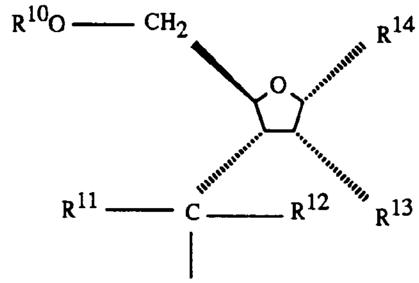

VIA

where $R^{10}$ to $R^{13}$ are as defined in any of claims 1 to 3, 6 and 7, $R^9$ is halogen, hydroxy, $-OR^{21}$, $-OCOR^{21}$ or $-OSO_2R^{21}$ or together with $R^8$ denotes a $C_1$-$C_{15}$ alkylidenedioxy group and $R^{14}$ is halogen, hydroxy, $-OR^{26}$, $-OCOR^{26}$ or $-OSO_2R^{26}$, or together with $R^{13}$ denotes a $C_1$-$C_{15}$ alkylidenedioxy group.

**9.** A compound according to claim 1, which is a dinucleotide analogue of formula

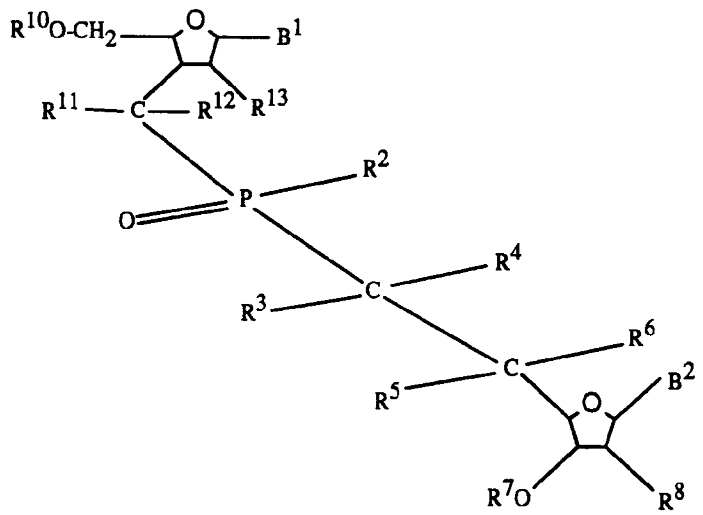

VII

where $B^1$ and $B^2$ are as defined in claim 1 or 7, $R^2$ to $R^8$ and $R^{10}$ to $R^{13}$ are as defined in any of claims 1, 3, 6 and 7.

**10.** A compound according to claim 9, which is of formula

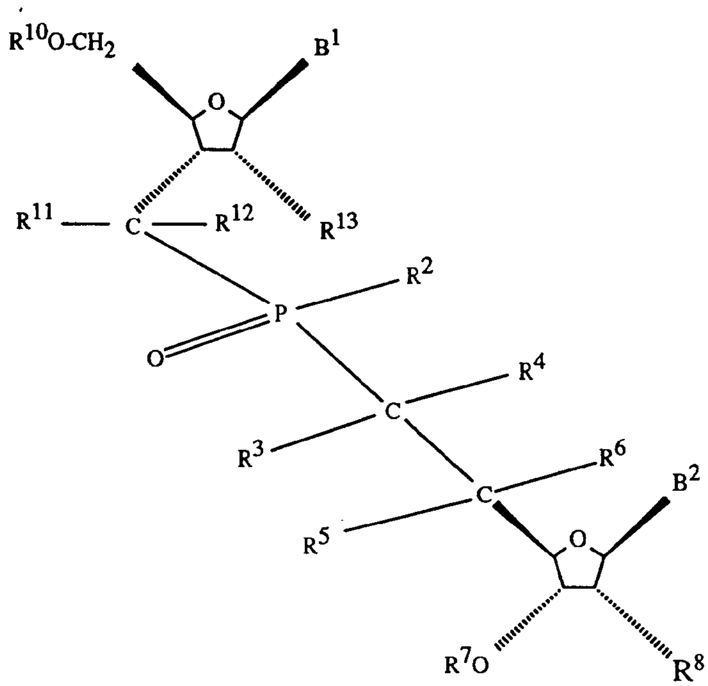

VIII

where $B^1$, $B^2$ and $R^2$ to $R^8$ and $R^{10}$ to $R^{13}$ are as defined in claim 9.

**11.** A method of preparing a compound according to claim 1 where $R^1$ is a group of formula II in which $R^{11}$ is hydroxy and $R^{12}$ is hydrogen, which comprises

A) reacting a compound of formula I where $R^1$ is hydrogen with an aldehyde of formula

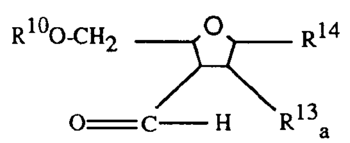

IX

where $R^{10}$, $R^{13}_a$ and $R^{14}$ are as defined in claim 1, in the presence of a base;

or

B) reacting compound of formula I where $R^1$ is hydrogen with a silylating agent to give a P(III) silyl compound and reacting the P(III) silyl compound with an aldehyde of formula IX.

**12.** A method of preparing a compound according to claim 1 where $R^1$ is a group of formula II, which comprises reacting a compound of formula I where $R^1$ is hydrogen with a compound of formula

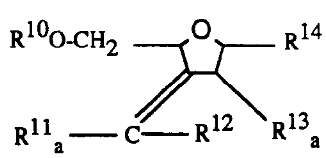

X

where $R^{10}$, $R^{12}$, $R^{13}_a$ and $R^{14}$ are as defined in claim 1 and $R^{11}_a$ is hydrogen, halogen or $R^{23}$ as defined in claim 1, in the presence of a free radical initiator.

**13.** A method of preparing a compound according to claim 1 which is a dinucleotide analogue according to claim 9 in which $B^1$ and $B^2$ are the same, which comprises glycosylation of a compound of formula

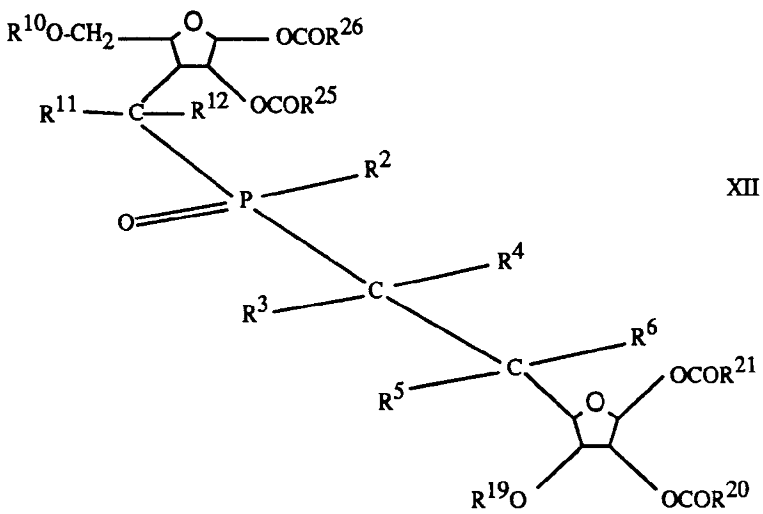

XII

where $R^2$ to $R^6$, $R^{10}$, $R^{11}$, $R^{12}$, $R^{19}$, $R^{20}$, $R^{21}$, $R^{25}$ and $R^{26}$ are as defined in claim 1, with a base of formula $B^1H$ where $B^1$ is as defined in claim 1.

**14.** A method of preparing a compound according to claim 1 which is a dinucleotide analogue according to claim 9 in which $B^1$ and $B^2$ are the same or different, which comprises glycosylation of a compound of formula

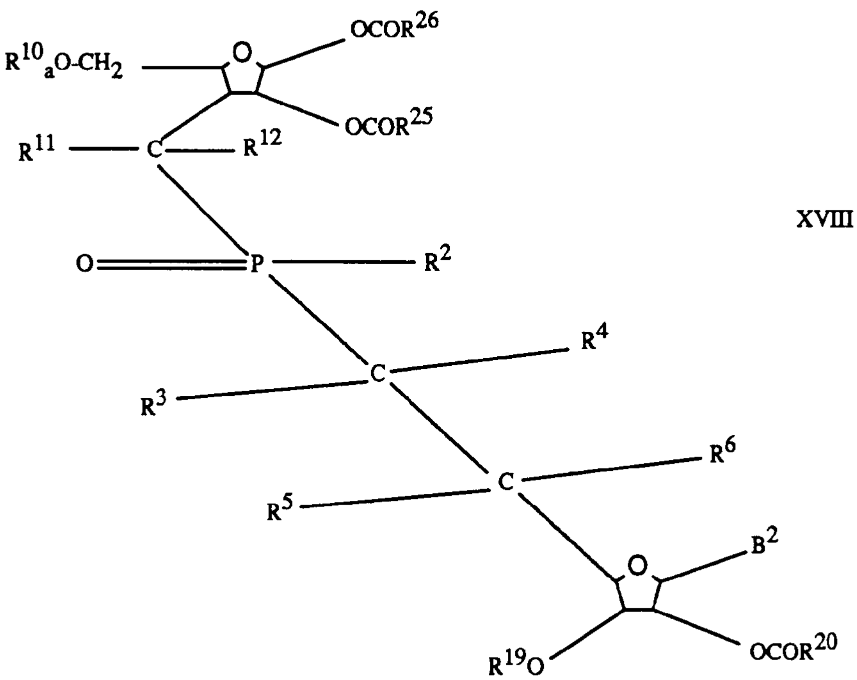

XVIII

where $B^2$, $R^2$ to $R^6$, $R^{10}{}_a$, $R^{11}$, $R^{12}$, $R^{19}$, $R^{20}$, $R^{25}$ and $R^{26}$ are as defined in claim 1, with a base of formula $B^1H$, where $B^1$ is as defined in claim 1.

**15.** A method of preparing a compound according to claim 1, which is a dinucleotide according to claim 9 where $B^1$ and $B^2$ are the same, which comprises glycosylation of a compound of formula

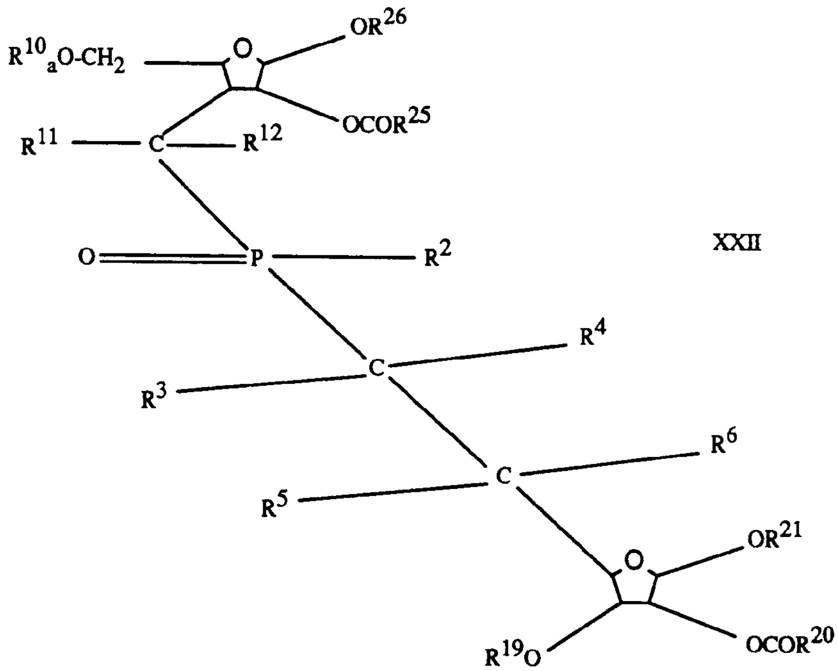

XXII

where $R^2$ to $R^6$, $R^{10}_a$, $R^{11}$, $R^{12}$, $R^{19}$, $R^{20}$, $R^{21}$, $R^{25}$ and $R^{26}$ are as defined in claim 1, with a base of formula $B^1H$, where $B^1$ is as defined in claim 1.

**16.** A method of preparing a compound according to claim 1 where $R^1$ is a group of formula II in which $R^{11}$ and $R^{12}$ are each hydrogen, which comprises deoxygenation of a compound according to claim 1 where $R^1$ is a group of formula II in which $R^{11}$ is hydroxy and $R^{12}$ is hydrogen.

**17.** A method of preparing a compound according to claim 1 which is a compound of formula

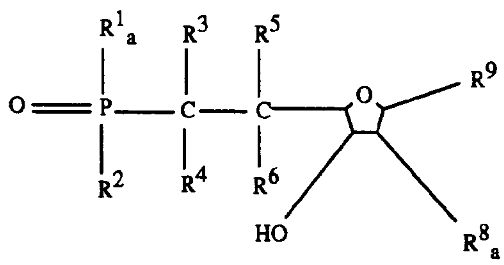

XXVI

where $R^1a$, $R^2$ to $R^6$, $R^8a$ and $R^9$ are as defined in claim 1, which comprises reacting an oxetane of formula

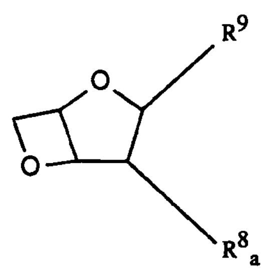

XXIX

where $R^8_a$ and $R^9$ are as defined in claim 1, with an organometallic compound of formula

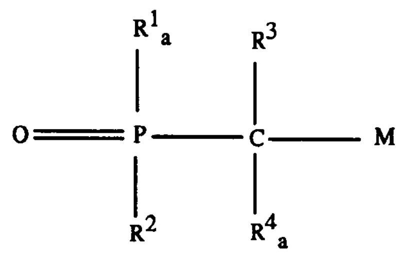

XXX

where $R^1{}_a$, $R^2$, $R^3$ and $R^4{}_a$ are as defined in claim 1 and M is lithium or magnesium, in the presence of a Lewis acid.

**18.** A method of preparing a compound according to claim 1 where $R^1$ is $R^1{}_a$, $R^3$, $R^4$, $R^5$ and $R^6$ are each hydrogen and $R^8$ and $R^9$ together denote a $C_1$ to $C_{15}$ hydrocarbylidenedioxy group, which comprises reacting an olefine of formula

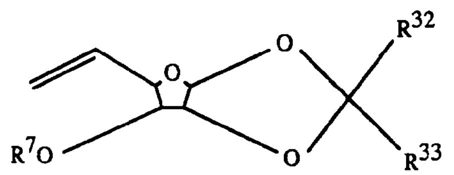

XXXVI

where $R^7$ is hydrogen or $R^7{}_a$ as defined in claim 1, and $R^{32}$ and $R^{33}$ are independently hydrogen, $C_1$ to $C_{10}$ alkyl, $C_3$ to $C_8$ cycloalkyl or $C_6$ to $C_{10}$ aryl, with a phosphine oxide of formula

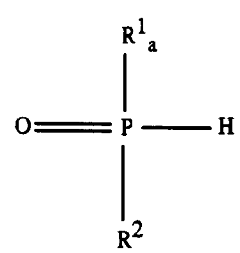

XXXIII

where Q and $R^2$ are as defined in claim 1, in the presence of a free radical initiator.

**19.** A method of preparing a compound of formula I where $R^1$ is $R^1{}_a$, $R^7$ is $R^7{}_a$ and $R^9$ is $B^1$, which comprises glycosylating a compound according to claim 1 where $R^1$ is $R^1{}_a$, $R^7$ is $R^7{}_a$, $R^8$ is $-OCOR^{20}$ and $R^9$ is $-OR^{21}$ or $-OCOR^{21}$ with a base of formula $B^1H$.

**20.** An oligonucleotide containing at least one unit derived from a dinucleotide analogue according to claim 9 or 10.

**21.** An oligonucleotide of formula

$$5^1\text{-U-(O-L-O-V)}_n\text{O-L-O-W-}3^1 \qquad \text{XXXXV}$$

where U, V and W are the same or different residues of natural or synthetic nucleosides and at least one of the residues U, V and W is derived from a dinucleotide analogue according to claim 9 or 10 and has the formula

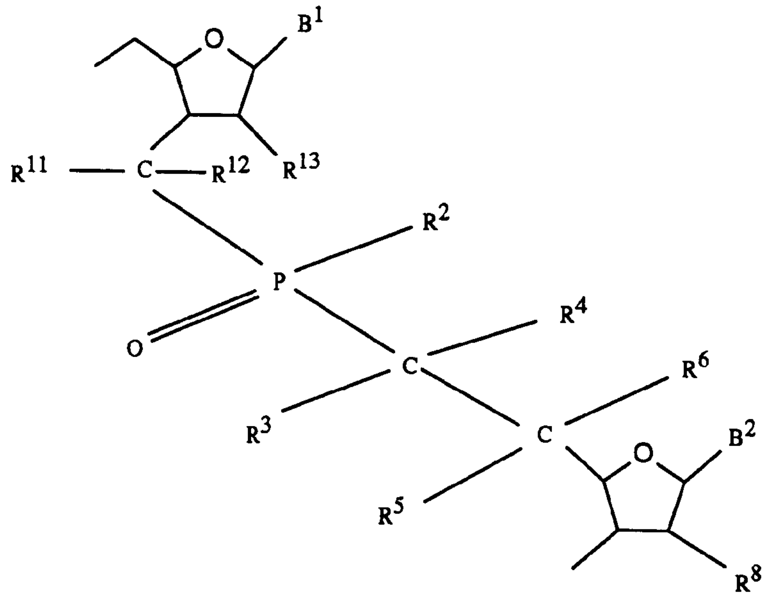

XXXXVI

where $R^2$ to $R^6$, $R^8$, $R^{11}$, $R^{12}$ and $R^{13}$, $B^1$ and $B^2$ are as defined in claim 9, L is a nucleoside bridging group and n is a number from 0 to 200.

22. An oligonucleotide according to claim 21, in which n is a number from 0 to 30 and L is a group -P(O)$O^{\ominus}$.

23. An oligonucleotide according to claim 21 or 22, in which n is 1 to 6.

24. An oligonucleotide according to any of claims 21 to 23, in which U, V and W are the same or different residues of a natural nucleoside and at least one of the residues U, V and W is of Formula XXXXVI where $B^1$ and $B^2$ are natural nucleoside base radicals.

25. A pharmaceutical composition comprising as active ingredient a compound according to any of claims 1 to 10 or an oligonucleotide according to any of claims 20 to 24.